# EUROPEAN PATENT SPECIFICATION

(11) **EP 3 980 776 B1**
(45) Date of publication and mention of the grant of the patent: **28.08.2024**
(21) Application number: 20731438.6
(22) Date of filing: 04.06.2020
(51) Int. Cl.: A61K 35/76, G01N 33/554, G01N 15/10, C12N 5/0783, G01N 15/14, A61K 35/17

(54) **SORTING WITH COUNTER SELECTION USING SEQUENCE SIMILAR PEPTIDES**
SORTIERUNG MIT ZÄHLERAUSWAHL UNTER VERWENDUNG VON SEQUENZÄHNLICHEN PEPTIDEN
TRI AVEC SÉLECTION DE COMPTEUR À L'AIDE DE PEPTIDES SIMILAIRES À UNE SÉQUENCE

(30) Priority: 06.06.2019 US 201962858167 P; 30.10.2019 DE 102019129341
(43) Date of publication of application: 13.04.2022
(73) Proprietor: Immatics Biotechnologies GmbH, 72076 Tübingen (DE); immatics US, Inc., Houston TX 77030 (US)
(72) Inventor: BUNK, Sebastian, 72074 Tübingen (DE); MAURER, Dominik, 72116 Mössingen (DE); SCHIMMACK, Gisela, 72074 Tübingen (DE); SCHUSTER, Heiko, 72074 Tübingen (DE); WAGNER, Claudia, 72074 Tübingen (DE); YOUSEF, Sara, 72074 Tübingen (DE); ALPERT, Amir, Houston, TX 77030 (US)
(74) Representative: Zwicker, Jörk
(86) International application number: PCT/EP2020/065567
(87) International publication number: WO 2020/245326

(56) References cited:
- WO-A1-2014/096803
- WO-A1-2016/090177
- WO-A2-97/04311
- Y. UEMURA ET AL: "Systematic Analysis of the Combinatorial Nature of Epitopes Recognized by TCR Leads to Identification of Mimicry Epitopes for Glutamic Acid Decarboxylase 65-Specific TCRs", THE JOURNAL OF IMMUNOLOGY, vol. 170, no. 2, 15 January 2003 (2003-01-15), US, pages 947 - 960, XP055578553, ISSN: 0022-1767, DOI: 10.4049/jimmunol.170.2.947
- ARMEN R. KARAPETYAN ET AL: "TCR Fingerprinting and Off-Target Peptide Identification", FRONTIERS IN IMMUNOLOGY, vol. 10, 22 October 2019 (2019-10-22), CH, XP055716082, ISSN: 1664-3224, DOI: 10.3389/fimmu.2019.02501

## Description

The present invention relates to a method for selecting a eukaryotic cell expressing on its surface an antigen-binding protein specifically binding to a protein antigen of interest (PAI) while counter selection using a similar protein antigen (SPA) is applied. Further, the invention provides a method for determining the sequence of a nucleic acid encoding an antigen-binding protein or an antigen-binding part thereof and a method for producing a cell expressing a nucleic acid encoding an antigen-binding protein or an antigen-binding part thereof.

### Background of the Invention

The field of adoptive cell transfer (ACT) has become one of the most promising and innovative approaches to treat cancer, viral infections and other immune-modulated disease. To support the broader clinical application of T-cell receptor (TCR)-modified T-cells, it is important that risks can be appropriately identified and mitigated, preferably at the pre-clinical level. The toxicity observed to date with the administration of TCR-modified T-cells is similar to that observed during standard ACT and can be grossly divided into three main groups: toxicity due to the lymph depleting preparation regimen, cytokine-related toxicity and immune-related toxicity. Immune-related toxicity can be classified into two subcategories: so-called "off-tumor/on-target" effects and "off-tumor/off-target" effects. The optimal gene-engineered T-cell therapy target antigen is one that is only present on the tumor cell and absent in healthy cells; however, in most cases the selected tumor target antigens are over-expressed or aberrantly expressed proteins that may be present to varying extent in normal cells (Johnson LA et al., Gene therapy with human and mouse T-cell receptors mediate cancer regression and targets normal tissues expressing cognate antigen, Blood 2009; 114:535-46).

### "Off-tumor/on-target" toxicity

Gene-engineered T-cell therapies may, therefore, trigger a potent cellular immune response against normal cells, even those that express the target antigens at low levels. This type of toxicity is known as "off-tumor/on-target" and is due to, for example, the engineered T-cells being unable to distinguish between normal cells and cancer cells that express the targeted antigen. Targeting of Melan A (MLA; also referred to as "melanoma antigen recognized by T-cells 1" (MART-1)) has been associated with significant "off-tumor/on-target" side effects (Johnson LA et al., Gene therapy with and mouse T-cell receptors mediates cancer regression and targets normal tissue expressing cognate antigens, Blood 2009, 114:535-46; van den Berg JH et al., Case report of a Fatal Serious Adverse event upon Administration of T-cells transduced with a MART-1 specific T-cell Receptor, Mol. Ther. 2015; 23:1541-50). Specifically, a case report has been published describing a fatal serious adverse event 3 days after transduced T-cell administration with a MART-1 specific TCR to a patient with metastatic melanoma. Infused T-cells were recovered from blood, broncho-alveolar lavage, ascites, tumor sites and heart tissue, and although no cross-reactivity of the modified T-cells toward a 3-D beating cardiomyocyte culture was observed, the authors were not able to exclude the possibility of cross-reactivity with an allogeneic MHC-peptide complex. Additionally, multiple-organ failure was found to be due to on-target cytokine release. Off-tumor/on target toxicity can be avoided by selecting target antigens that show a sufficiently low expression off-tumor to lead to an acceptable toxicity upon application of doses that are therapeutically effective on the tumor.

### "Off-tumor/off-target" toxicity

Because most tumor antigens are derived from self-proteins (tumor-associated antigens), the isolation of high-affinity tumor-specific T-cells is effectively precluded by thymic selection. TCR affinity can, nevertheless, be considerably enhanced through mutation of specific regions within the complementarity-determining regions (CDRs). Although useful to promote modified T-cell efficacy, due to TCR degeneracy, this approach carries the risk that a TCR might recognize other related peptide antigens present on normal tissue through cross-reactivity. Previously published results have shown lethal toxicities in two patients, who were infused with T-cells engineered to express a TCR targeting melanoma-associated antigen A3 (MAGE-A3) cross-reacting with a peptide from the muscle protein Titin, even though no cross-reactivities had been predicted in the pre-clinical studies (Linette, GP et al., Cardiovascular toxicity and titin cross-reactivity of affinity enhanced T-cells in myeloma and melanoma, Blood 2013; 122:863-71; Cameron, BJ et al., Identification of a Titin-derived HLA-A1-presented peptide as a cross-reactive target for engineered MAGE-A3 directed T-cells, Sci. Transl. Med. 2013; 5:197-103). These patients demonstrated that TCR-engineered T-cells can have serious and not readily predictable off-target and organ-specific toxicities and highlight the need for improved methods to define the specificity of engineered TCRs. Strategies such as peptide scanning and the use of more complex cell structures are therefore recommended in pre-clinical studies to mitigate the risk of off-target toxicities in future clinical investigations. Therefore, there is still an unmet medical need to develop and provide TCRs with low off-tumor/off-target toxicity. The present invention provides methods to rapidly identify antigen binding molecules, in particular TCRs that specifically and selectively bind to their target antigens and, thus provide enhanced safety profiles and reduced cross-reactivity to sequence similar target antigens, in particular sequence similar peptides on healthy tissues. The rapid, preferably one step, selection method of the present invention is particularly useful in the identification of patient-derived T-cells expressing TCRs with desired anti-tumor activity.

WO 97/04311 A2 discloses a method of sorting cells.

Y. Uemura et al., THE JOURNAL OF IMMUNOLOGY, vol. 170, no. 2, 15 January 2003, pages 947-960, disclose that systematic analysis of the combinatorial nature of epitopes recognized by TCR lead to identification of mimicry epitopes for glutamic acid decarboxylase 65-specific TCRs.

WO 2016/090177 A1 discloses a method for identifying T-cell epitopes.

WO 2014/096803 A1 discloses a method for predicting the off-target binding of a peptide, which binds to a target peptide presented by a Major Histocompatibility Complex (MHC).

Armen R. Karapetyan et al., FRONTIERS IN IMMUNOLOGY, vol. 10, 22 October 2019 disclose methods for TCR fingerprinting and off-target peptide identification.

### Summary of the Invention

The present invention relates to the subject matter of claim 1 to 14. A first aspect of the invention relates to a method for selecting a eukaryotic cell expressing on its surface an antigen-binding protein specifically and/or selectively binding to a protein antigen of interest (PAI) comprising the following steps:
(i) providing a eukaryotic cell population;
(ii) contacting the eukaryotic cell population of step (i) with a first antigen complex (1^{st} AC) comprising the PAI and a detectable label A or with the PAI comprising a detectable label A;
(iii) contacting the eukaryotic cell population of step (i) with at least a second antigen complex (2^{nd} AC) comprising a similar protein antigen (SPA), wherein the amino acid sequence of the SPA differs by at least 1-20 amino acids from the amino acid sequence of the PAI, preferably by 2-10 amino acids from the amino acid sequence of the PAI, and wherein the 2^{nd} AC comprises a detectable label B; or with the SPA and a detectable label B; and
(iv) selecting at least one eukaryotic cell that specifically and/or selectively binds to the 1^{st} AC, wherein the detectable label A and the detectable label B are detectably different from each other, and wherein the selected eukaryotic cell is a T-cell, a B-cell, or a mammalian cell expressing a heterologous antigen binding protein selected from the group consisting of a T-cell receptor (TCR) or antigen binding fragments thereof, a B-cell receptor (BCR) or antigen binding fragments thereof, and a chimeric antigen receptor (CAR) or antigen binding fragments thereof.

A second aspect of the invention further relates to a method for determining the sequence of a nucleic acid encoding an antigen-binding protein or an antigen-binding part thereof comprising the steps of:
(i) isolating the nucleic acid encoding the antigen-binding protein or the antigen-binding part thereof from the eukaryotic cell selected in the method of the first aspect of the invention; and
(ii) determining the sequence of the nucleic acid.

A third aspect of the invention relates to a method for producing a cell expressing a nucleic acid encoding an antigen-binding protein or an antigen-binding part thereof comprising the steps of
(i) providing the nucleic acid sequence encoding the antigen-binding protein or an antigen-binding part thereof from the eukaryotic cell selected in the method of the first aspect of the invention;
(ii) producing a nucleic acid vector comprising the nucleic acid sequence provided in step (i) optionally under the control of an expression control element; and
(iii) introducing the nucleic acid vector of step (ii) into a host cell.

### List of Figures

In the following, the content of the Figures comprised in this specification is described. In this context please also refer to the detailed description of the invention above and/or below.

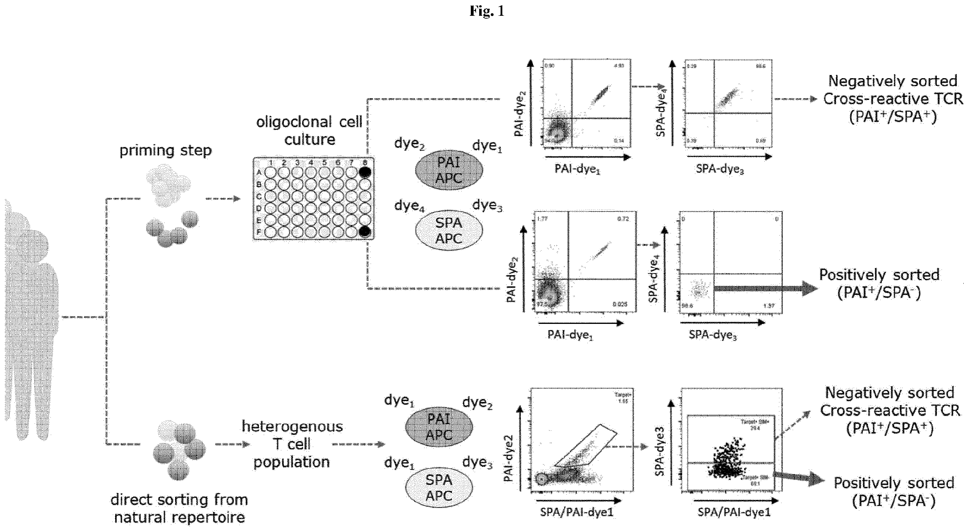

**Figure 1** shows a schematic presentation of two exemplifying applications of the invention. The upper pathway of the figure represents the use of the gating strategy when applied to primed T-cells that underwent an individual T cell culturing step, the lower part of the figure shows the use of the gating strategy in a direct sorting approach, wherein a heterogenous T cell population obtained from a natural repertoire is enriched with target specific T cells using the gating strategy. In both examples the positively sorted cell fraction represents immune cells expressing on their surface an antigen-binding protein specifically and/or selectively binding to a protein antigen of interest. Abbreviations used in the figure: SPA: similar protein antigen, PAI: protein antigen of interest, APC: antigen-presenting cell.

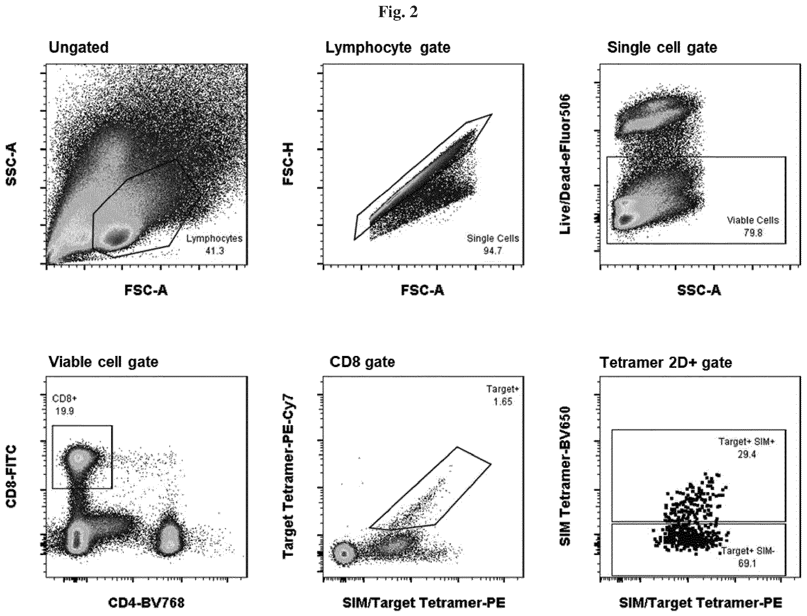

**Figure 2** shows an exemplary gating strategy of non-amplified target-specific T cells. To enhance the frequency of low-frequency target-specific T cells in the test sample, the cells have been enriched by fluorochrome-tetramer specific magnetic bead isolation. Subsequently, cells were stained for surface markers and assessed by flow cytometry. Individual 2D-color tetramer combinations were used to stain target-specific and similar peptide-specific T cells. In this example 1.65% of CD8 T cells bind to target-peptide tetramer and of those target-specific CD8 T cells 29.4% also bind to similar peptide-tetramer (Target⁺/SIM⁺), which is comprised of 3 different similar peptide-HLAs. By including similar peptide tetramers in the sorting procedure, a high proportion of cross-reactive T cells (Target⁺/SIM⁺) can be excluded.

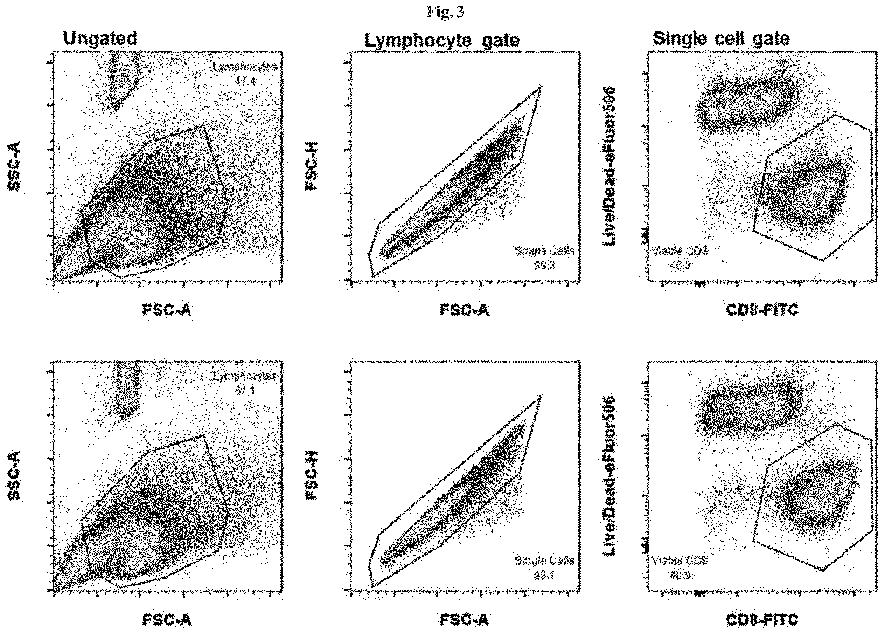

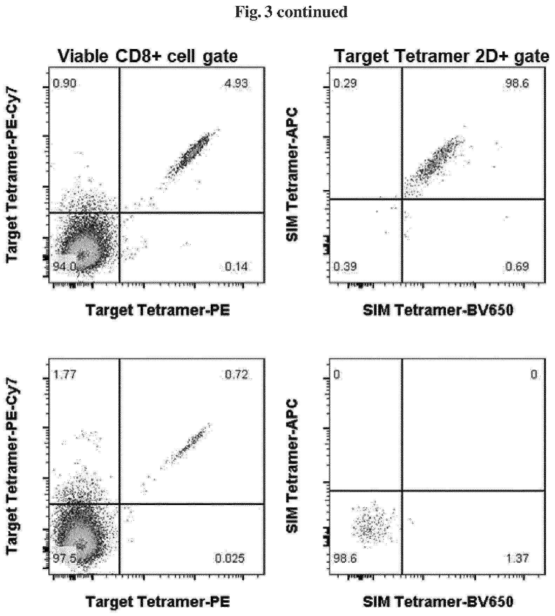

**Figure 3** shows an exemplary gating strategy of primed T cell populations. Individual T cell cultures were repeatedly stimulated with target peptide HLA-coated artificial presenting cells to enhance low-frequency target-specific CD8 T cells. After 4 weeks in culture those primed T cell cultures were stained for surface markers and individual 2D-color tetramer combinations for target-HLA and 3 similar peptide-HLAs. The upper panel shows a monoclonally enriched T cell population binding to both target- and similar-peptide tetramers (Target⁺/SIM⁺). The lower panel shows a monoclonally enriched T cell population binding only to the target- but not similar-peptide tetramer. By including similar peptide tetramers in the staining procedure cross-reactive T cells (Target⁺/SIM⁺) can be excluded from sorting.

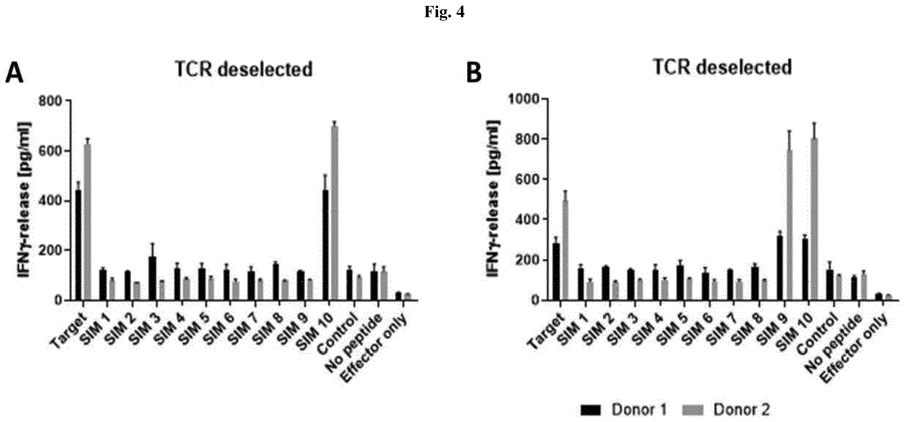

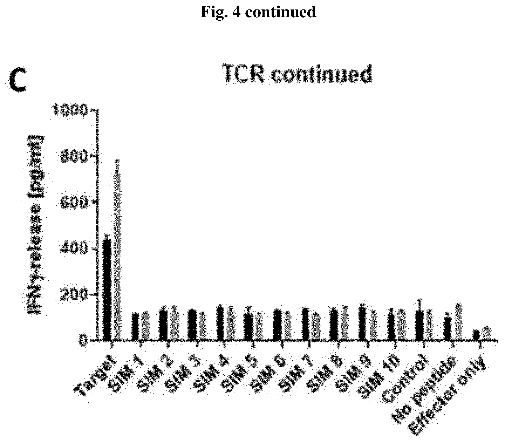

**Figure 4** shows that TCRs from T cells sorted using target-peptide tetramers only, can be cross-reactive to target-similar peptides. TCRs identified using target-peptide tetramers were assessed for cross-reactivity against 10 target similar peptides after mRNA electroporation into healthy donor T cells. As measure for reactivity, IFNγ secretion upon co-culture with peptide-loaded T2 cells was assessed. All TCRs in this example react against the target peptide (positive control) and not against controls, which are unrelated/irrelevant peptide loaded T2 cells, unloaded T2 cells or effector only cells. However, the TCRs in Fig. 4A and Fig. 4B also show reactivity to similar peptides, namely similar peptide 1 and 10 for TCR in Fig. 4A and similar peptide 9 and 10 for the TCR in Fig. 4B. Only the TCR in Fig. 4C shows no cross-reactivity and is therefore selected for further characterization.

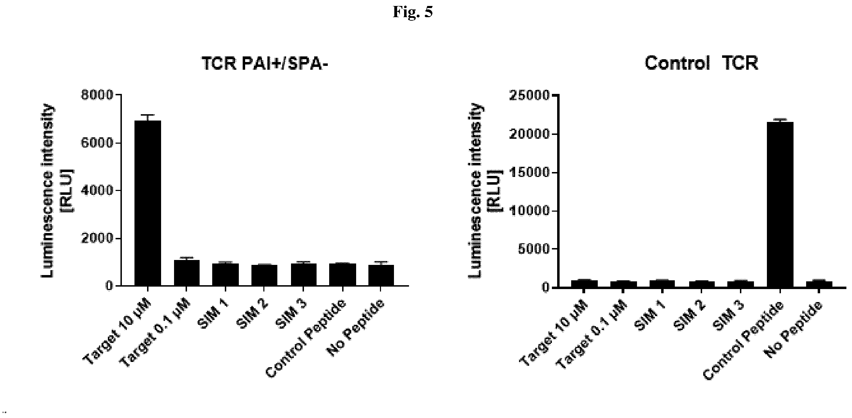

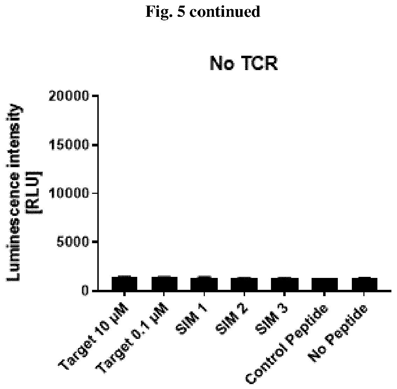

**Figure 5** shows the functional assessment of a TCR isolated from T cells binding to target-peptide tetramers only (TCR PAI+/SPA-), as well as a control TCR specific for a control peptide ("control peptide"), and a no TCR control ("no peptide"). For this end, TCR-mRNA was electroporated into NFAT-luciferase Jurkat reporter cells and their activation assessed after co-culture with peptide/target similar peptides ("SIM 1, SIM 2, and SIM 3") loaded T2 antigen-presenting cells. The TCR derived from PAI+/SPA- sorted T cells triggers activation only when co-cultured with target peptide-loaded T2 cells. The control TCRs shows reactivity in the presence of control-peptide and Jurkat cells without TCR mRNA electroporation do not respond to peptide-loaded T2 cells. This example shows that TCRs binding to target-peptide tetramers also show reactivity toward those peptides on a functional level.

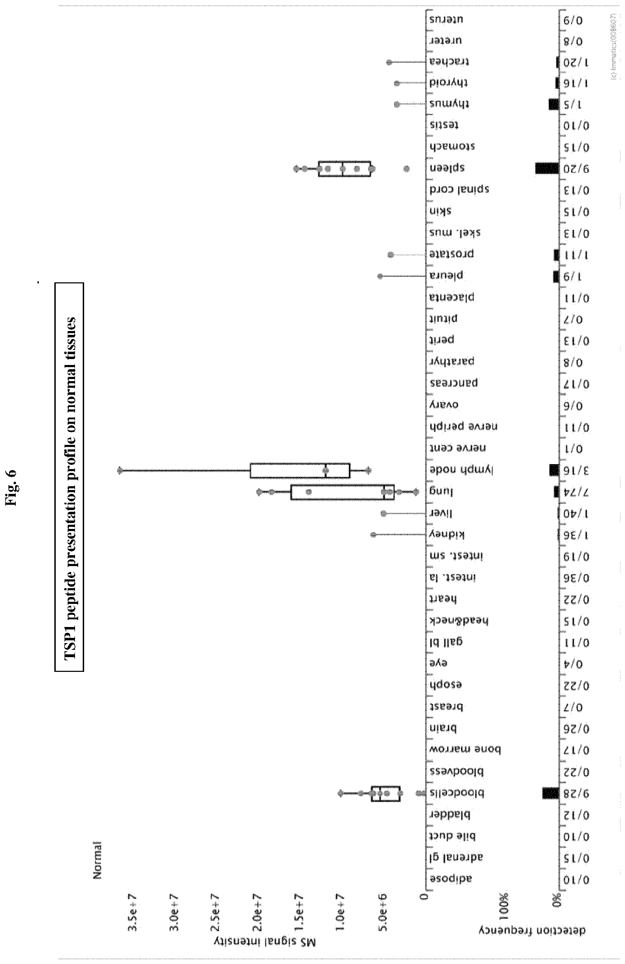

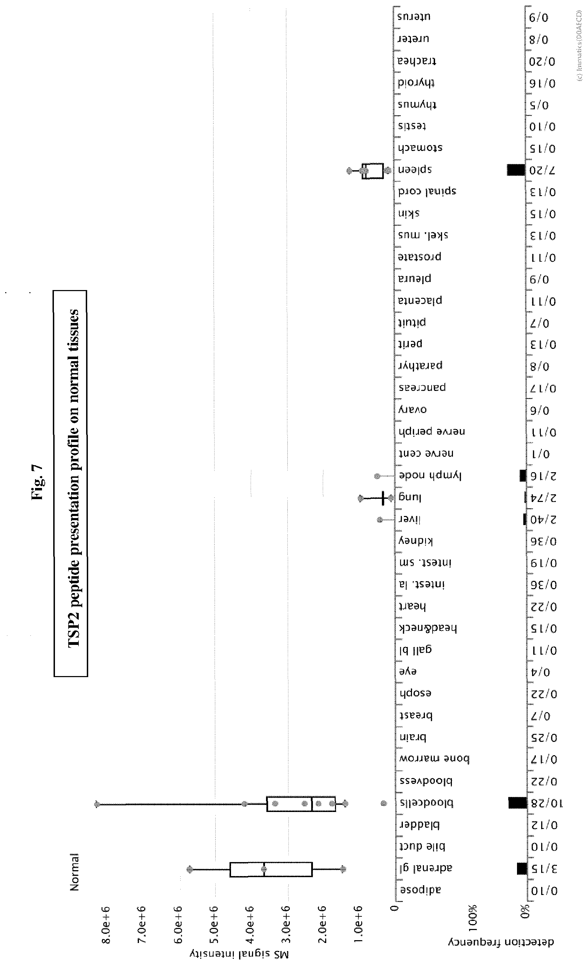

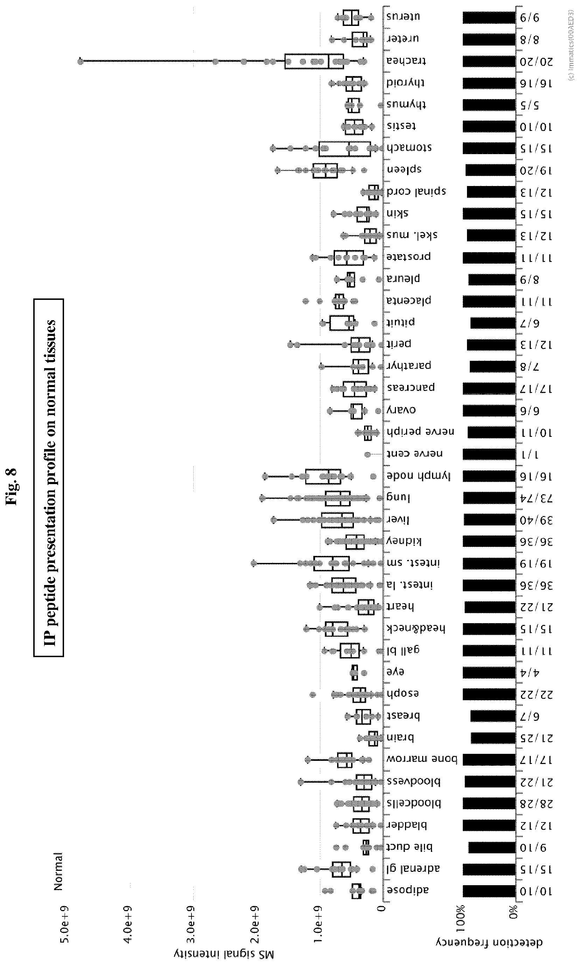

**Figures 6****,** **7** **and** **8** show peptide presentation profiles of a target similar peptide 1 (TSP1) **(****Figure 6****),** TSP2 **(****Figure 7**) and an irrelevant peptide (IP; **Figure 8**) from Example 4 based on XPRESIDENT mass spectrometry data. Upper part: Median relative MS signal intensities from technical replicate measurements are plotted as colored dots for single HLA-A*02 normal samples on which the peptide was detected. Normal samples are grouped according to organ of origin. Boxand-whisker plots represent normalized signal intensities over multiple samples and have been defined in the log space. Boxes display median, 25^{th} and 75^{th} percentile. Whiskers extend to the lowest data point still within 1.5 interquartile range (IQR) of the lower quartile, and the highest data point still within 1.5 IQR of the upper quartile. Lower part: The peptide detection frequency in every organ is shown as a bar plot. Numbers below the panel indicate number of samples on which the peptide was detected out of the total number of samples analyzed for each organ (N ≥ 628 for normal samples across all organs). If the peptide has been detected on a sample but could not be quantified for technical reasons, the sample is included in this representation of detection frequency, but no dot is shown in the upper part of the figure, adipose: adipose tissue; adrenal gl: adrenal gland; bladder: urinary bladder; bloodvess: blood vessel; esoph: esophagus; gall bkgallbladder; intest. la: large intestine; intest. sm: small intestine; nerve cent: central nerve; nerve periph: peripheral nerve; parathyr: parathyroid gland; perit: peritoneum; pituit: pituitary; skel. mus: skeletal muscle.

### List of selected Sequences

| | |
|---|---|
| SEQ ID NO: 1 | X₁-X₂-X₃-X₄-X₅-X₆-X₇-X₈, wherein X₁-X₈ are amino acids positions in a target peptide of a length of 8 amino acids and X in each case is any amino acid; |
| SEQ ID NO: 2 | X₁-X₂-X₃-X₄-X₅-X₆-X₇-X₈-X₉, wherein X₁-X₉ are amino acids positions in a target peptide of a length of 9 amino acids and X in each case is any amino acid; |
| SEQ ID NO: 3 | X₁-X₂-X₃-X₄-X₅-X₆-X₇-X₈-X₉-X₁₀, wherein X₁-X₁₀ are amino acids positions in a target peptide of a length of 10 amino acids and X in each case is any amino acid; |
| SEQ ID NO: 4 | X₁-X₂-X₃-X₄-X₅-X₆-X₇-X₈-X₉-X₁₀-X₁₁, wherein X₁-X₁₁ are amino acids positions in a target peptide of a length of 11 amino acids and X in each case is any amino acid; |
| SEQ ID NO: 5 | X₁-X₂-X₃-X₄-X₅-X₆-X₇-X₈-X₉-X₁₀-X₁₁-X₁₂ , wherein X₁-X₁₂ are amino acids positions in a target peptide of a length of 12 amino acids and X in each case is any amino acid. |

### Detailed Description of the Invention

Before the present invention is described in detail below, it is to be understood that this invention is not limited to the particular methodology, protocols and reagents described herein as these may vary. It is also to be understood that the terminology used herein is for the purpose of describing particular embodiments only and is not intended to limit the scope of the present invention which will be limited only by the appended claims. Unless defined otherwise, all technical and scientific terms used herein have the same meanings as commonly understood by one of ordinary skill in the art.

Several documents are cited throughout the text of this specification. Nothing herein is to be construed as an admission that the invention is not entitled to antedate such disclosure by virtue of prior invention. In the event of a conflict between the definitions or teachings of such references and definitions or teachings recited in the present specification, the text of the present specification takes precedence.

In the following, the elements of the present invention will be described. These elements are listed with specific embodiments however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

### Definitions

To practice the present invention, unless otherwise indicated, conventional methods of chemistry, biochemistry, and recombinant DNA techniques are employed which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

In the following, some definitions of terms frequently used in this specification to characterize the invention are provided. These terms will, in each instance of its use, in the remainder of the specification have the respectively defined meaning and preferred meanings.

As used in this specification and the appended claims, the singular forms "a", "an", and "the" include plural referents, unless the content clearly dictates otherwise.

The term "amino acid" refers in the context of this invention to any monomer unit that comprises a substituted or unsubstituted amino group, a substituted or unsubstituted carboxy group, and one or more side chains or groups, or analog of any of these groups. Exemplary side chains include, e.g., thiol, seleno, sulfonyl, alkyl, aryl, acyl, keto, azido, hydroxyl, hydrazine, cyano, halo, hydrazide, alkenyl, alkynl, ether, borate, boronate, phospho, phosphono, phosphine, heterocyclic, enone, imine, aldehyde, ester, thioacid, hydroxylamine, or any combination of these groups. Other representative amino acids include, but are not limited to, amino acids comprising photoactivatable cross-linkers, metal binding amino acids, spin-labelled amino acids, fluorescent amino acids, metal-containing amino acids, amino acids with novel functional groups, amino acids that covalently or noncovalently interact with other molecules, photocaged and/or photoisomerizable amino acids, radioactive amino acids, amino acids comprising biotin or a biotin analog, glycosylated amino acids, other carbohydrate modified amino acids, amino acids comprising polyethylene glycol or polyether, heavy atom substituted amino acids, chemically cleavable and/or photocleavable amino acids, carbon-linked sugar-containing amino acids, redoxactive amino acids, amino thioacid containing amino acids, and amino acids comprising one or more toxic moieties. As used herein, the term "amino acid" includes the following twenty natural or genetically encoded alpha-amino acids: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or 1), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V). In cases where "X" residues are undefined, these are to be interpreted as "any amino acid." The structures of these twenty natural amino acids are shown in, e.g., Stryer et al., Biochemistry, 5th ed., Freeman and Company (2002). Additional amino acids, such as selenocysteine and pyrrolysine, can also be genetically coded for (Stadtman (1996) "Selenocysteine," Annu Rev Biochem. 65:83-100 and Ibba et al. (2002) "Genetic code: introducing pyrrolysine," Curr Biol. 12(13):R464-R466). The term "amino acid" also includes unnatural amino acids, modified amino acids (e.g., having modified side chains and/or backbones), and amino acid analogs. See, e.g., Zhang et al. (2004) "Selective incorporation of 5-hydroxytryptophan into proteins in mammalian cells," Proc. Natl. Acad. Sci. U.S.A. 101(24):8882-8887, Anderson et al. (2004) "An expanded genetic code with a functional quadruplet codon" Proc. Natl. Acad. Sci. U.S.A. 101(20):7566-7571, Ikeda et al. (2003) "Synthesis of a novel histidine analogue and its efficient incorporation into a protein in vivo," Protein Eng. Des. Sel. 16(9):699-706, Chin et al. (2003) "An Expanded Eukaryotic Genetic Code," Science 301(5635):964-967, James et al. (2001) "Kinetic characterization of ribonuclease S mutants containing photoisomerizable phenylazophenylalanine residues," Protein Eng. Des. Sel. 14(12):983-991, Kohrer et al. (2001) "Import of amber and ochre suppressor tRNAs into mammalian cells: A general approach to site-specific insertion of amino acid analogues into proteins," Proc. Natl. Acad. Sci. U.S.A. 98(25): 14310-14315, Bacher et al. (2001) "Selection and Characterization of Escherichia coli Variants Capable of Growth on an Otherwise Toxic Tryptophan Analogue," J. Bacteriol. 183(18):5414-5425, Hamano-Takaku et al. (2000) "A Mutant Escherichia coli Tyrosyl-tRNA Synthetase Utilizes the Unnatural Amino Acid Azatyrosine More Efficiently than Tyrosine," J. Biol. Chem. 275(51):40324-40328, and Budisa et al. (2001) "Proteins with {beta}-(thienopyrrolyl) alanines as alternative chromophores and pharmaceutically active amino acids," Protein Sci. 10(7): 1281-1292. Amino acids can be merged into peptides, polypeptides, or proteins. As used in this specification the term "peptide" refers to a short polymer of amino acids linked by peptide bonds. It has the same chemical (peptide) bonds as proteins but is commonly shorter in length. The shortest peptide is a dipeptide, consisting of two amino acids joined by a single peptide bond. There can also be a tripeptide, tetrapeptide, pentapeptide, etc. Typically, a peptide has a length of up to 8, 10, 12, 15, 18 or 20 amino acids. A peptide has an amino end and a carboxyl end, unless it is a cyclic peptide.

The term "virus" refers in the context of the present invention to small obligate intracellular parasites, which by definition contain either a RNA or DNA genome surrounded by a protective protein coat, i.e. a capsid. The genome of a virus may consist of DNA or RNA, which may be single stranded (ss) or double stranded (ds), linear or circular. The entire genome may occupy either one nucleic acid molecule (monopartite genome) or several nucleic acid segments (multipartite genome). The virus can be a double-stranded DNA virus, preferably Myoviridae, Siphoviridae, Podoviridae, Herpesviridae, Adenoviridae, Baculoviridae, Papillomaviridae, Polydnaviridae, Polyomaviridae, Poxviridae; a single-stranded DNA virus, preferably Anelloviridae, Inoviridae, Parvoviridae; double-stranded RNA virus, preferably Reoviridae; a single-stranded RNA virus, preferably Coronaviridae, Picornaviridae, Caliciviridae, Togaviridae, Flaviviridae, Astroviridae, Arteriviridae, Hepeviridae; negative-sense single-stranded RNA virus, preferably Arenaviridae, Filoviridae, Paramyxoviridae, Rhabdoviridae, Bunyaviridae, Orthomyxoviridae, Bornaviridae; a single-stranded RNA reverse transcribing virus, preferably Retroviridae; or a double-stranded RNA reverse transcribing virus, preferably Caulimoviridae, Hepadnaviridae.

The term "bacteriophage" (or "phage") refers in the context of the present invention to a virus that infects and replicates in bacteria and archaea. Bacteriophages are dependent on a host organism, typically bacteria, to replicate in and inject their genome, which is either comprised of proteins that encapsulate desoxyribonucleic acid (DNA) or ribonucleic acid (RNA), into the host organims's cytoplasm. Prominent examples of bacteriophages used in biotechnology are bacteriophage T4 lambda (T4λ) phage, T7 phage, fd filamentous phage, in particular filamentous M13 phage of which all have certain benefits and drawbacks.

The term "virus population" refers in the context of the present invention to a high number of viruses which differ in the genetic information encoding the antigen binding protein expressed on their surface. The viral population can thus, express a library of heterologous antigen binding proteins.

The term "phage display" or "phage library" refers in the context of the present invention to a system that is used for high-throughput screening of protein interactions. Briefly, a gene encoding a protein of interest is inserted into a bacteriophage coat protein gene which causes the bacteriophage to "display", i.e. to show, the protein on its surface while keeping the gene encoding the protein of interest in its DNA or RNA. This results in a connection of genotype and phenotype. The proteins which are displayed on the bacteriophage's surface can subsequently be screened against other proteins, peptides or DNA sequences to study their interaction between the displayed molecule and the molecules to be screened. Such a molecule can be an antibody or a fragment thereof, a TCR or a fragment thereof, a BCR or a fragment thereof or a CAR or a fragment thereof. Fragments of TCRs may comprise the alpha variable domain and the beta variable domain. Fragments in the context of the present inventions are also defined in detail below.

The term "cell" refers in the context of the present invention to eukaryotic cells which contain a nucleus and cell organelles and can be found in protozoa, fungi, plants and animals. Animals can comprise mammalian cells. Mammalian cells comprise *inter alia* human cells, rhodent cells, such as mouse, or rat cells, monkey cells, pig cells or dog cells. Fungi cells *inter alia* comprise yeast cells. Typical yeast cells used in biotechnology, for example in a yeast surface display, are *Saccharomyces cerevisiae* cells.

The term "yeast surface display" or "yeast display" or "yeast library" refers in the context of the present invention to a protein engineering technique using yeast cells that express recombinant proteins of interest and incorporate these proteins into their cell wall. This allows for isolation and engineering of proteins, in particular antibodies or fragments thereof, TCRs or fragments thereof, BCRs or fragments thereof or CARs or fragments thereof. In detail, in the yeast surface display, the unit of selection is a yeast cell that is decorated with tens of thousands of copies of the protein of interest and that carries the plasmid encoding that protein. The plasmid can be shuttled between *Saccharomyces cerevisiae,* for display and sorting, and *E. coli,* for DNA preparation and molecular biology. In the form of yeast display pioneered by the Wittrup group (Chao *et al.*, 2006), each ¹⁰Fn3 variant is expressed as a genetic fusion with a native yeast protein found in the cell wall, Aga2p. Aga2p is a domain of the native yeast, an agglutinin mating factor; typically, it is cloned upstream of the sequence encoding the ¹⁰Fn3 variant. In addition, an epitope tag, such as c-myc and V5, is engineered immediately downstream from the sequence encoding the ¹⁰Fn3 variant. Upon induction, the mating-factor secretory signal peptide directs the fusion protein to be exported from the cell; it is captured on the surface of the yeast cell wall by its binding partner, Aga1p, to which it forms two disulfide bonds. The result is a culture where each yeast cell displays between 10,000 and 100,000 copies of a single ¹⁰Fn3 variant. On average, the more thermostable the variant, the larger the number of its molecules on the yeast surface (Hackel *et al.*, 2010).

The term "immune cell" refers in the context of this invention to a cell of the immune system. The immune system comprises different cell types such as precursor cells comprising lymphoid stem cells, which ultimately differentiate into B and T lymphocytes and natural killer (NK) cells, and myeloblasts, which ultimately differentiate into granulocytes and monocytes as well as fully differentiated leukocytes. Differentiated leukocytes are thymus-, spleen-, bone marrow or lymph node-derived cells and can be categorized into the main groups of granulocytes, B-lymphocytes, T-lymphocytes and monocytes, macrophages, and mast cells and dendritic cells. Granulocytes are further divided into neutrophil, eosinophil and basophil granulocytes, which phagocytose bacteria, virus or fungi in the blood circulation. B-lymphocytes are precursors of plasma cells and B-memory cells. The group of T-cells comprises regulatory T-cells, memory T-cells, T helper cells and cytotoxic T-cells. While T helper cells activate plasma cells and natural killer cells, regulatory T-cells inhibit the function of B and other T-cells and thus, slow down the immune response. T memory cells are long-living and possess a memory for specific antigens, and cytotoxic T-cells recognize and kill tumor cells or cells attacked by viruses by interacting with tumor antigens or antigens of the attacked cells. Examples of T-cells and their surface phenotype described by the specific surface markers of the respective T-cells are given in below Table 1 (according to Dong and Martinez, Nature Reviews Immunology, 2010):

**Table 1: Common T-cell surface markers (non-exhaustive enumeration).**

| **Cell:** | **Surface marker:** |
|---|---|
| Cytotoxic T-cells: | αβ TCR, CD3, CD8 |
| Regulatory T-cells: | αβ TCR, CD3, CD4 |
| Regulatory T-cells | αβ TCR, CD3, CD4, CD25, CTLA4, GITR |

| (natural and inducible): | |
|---|---|
| Natural Killer cells: | NK1.1, SLAMF1, SLAMF6, TGFβ, Vα24, Jα18 |
| T helper cells: | |
| TH1 cells | αβ TCR, CD3, CD4, IL-12R, IFNγR, CXCR3; |
| TH2 cells | αβ TCR, CD3, CD4, IL-4R,IL33R, CCR4, IL-17RB, CRTH2; |
| TH9 cells | αβ TCR, CD3, CD4; |
| TH17 cells | αβ TCR, CD3, CD4, IL-23R, CCR6, IL-1R, CD161; |
| TH22 cells | αβ TCR, CD3, CD4, CCR10; |
| TILs | Tumor-infiltrating lymphocytes |
| T memory cells | CCR7 hi, CD44, CD62Lhi, TCR, CD3, IL-7R (CD127), IL-15R |

The term "tumor-infiltrating lymphocytes" (TILs) refers in the context of the present invention to T-cells and B-cells that have migrated towards a tumor and can often be found in the tumor stroma or the tumor itself. TILs typically comprise a cell population of white blood cells that may be used in ACT or autologous cell therapy. Such therapies have already shown promising results, for example in patients with metastatic melanoma in a variety of clinical trials (Guo et al.; "Recent updates on cancer immunotherapy"; Precision Clinical Medicine, 1(2), 2018-65-74). In the context of ACT, TILs are expanded *ex vivo* from surgically resected tumors or single cell suspensions isolated from tumor fragments. TILs are expanded with a high doses of cytokines, for example IL-2. Selected TIL lines that presented best tumor reactivity are then further expanded in a "rapid expansion protocol" (REP), which uses anti-CD3 activation for a typical period of two weeks. The final post-REP TIL is infused back into the patient. The process can also involve a preliminary chemotherapy regimen to deplete endogenous lymphocytes in order to provide the adoptively transferred TILs with enough access to surround the tumor sites.

The term "immune cell enriched fraction" refers in the context of this invention to a cell population, which is derived from a naturally occurring cell population, e.g. blood, in which the relative abundance of the immune cells has been increased in comparison to their abundance in the naturally occurring cell mixture. One ml of blood of a healthy human subject comprises, e.g. 4.7 to 6.1 million (male), 4.2 to 5.4 million (female) erythrocytes, 4,000-11,000 leukocytes and 200,000-500,000 thrombocytes. Thus, in blood immune cells only constitute 0.06% to 0.25% of the total number of blood cells. An immune cell enriched fraction of blood thus may comprise more than 0,25%, more preferably more than 10%, even more preferably more than 50%, even more preferably more than 80% and most preferably more than 90% immune cells. The immune cell enriched fraction may be enriched for one or more subtypes of immune cells. For example, the immune cell enriched fraction may be enriched for lymphoid stem cells, T-cells, B-cells, plasma cells or combinations. Usually, immune cells in immune cell enriched fractions are selected by using one or more fluorescently labelled antibodies that specifically bind to a surface marker of the immune cells of interest. Suitable surface markers to select T-cells or sub-fractions within the group of T-cells are indicated in table 1 above. Cytotoxic T-cells can be selected, e.g. by using an antibody that specifically binds to CD8 or by using antibodies that specifically bind to CD8 and CD3 .

The term "cell population" refers in the context of this invention to a plurality of cells which may be homogenous or heterogenous, i.e. a mixture of cells of different characteristic. Blood is an example of a cell population which is a mixture of different cells. Homogenous cell populations can be obtained by selection of a particular subtype or by clonal expansion.

The term "antigen binding protein" refers in the context of this invention to one polypeptide or a complex of two or more polypeptides that comprise a paratope (alternatively referred to as "antigen binding site") that specifically binds to an antigen. Examples of antigen binding proteins are single chain antibodies, single chain TCRs, chimeric antigen receptor (CAR) and examples of antigen binding complexes are antibodies, B cell receptors (BCRs) or TCRs.

The term "chimeric antigen receptor" (CAR; also known as chimeric immunoreceptor, chimeric T cell receptor, artificial T cell receptor) in the context of the present invention refers to engineered receptors, which graft an arbitrary specificity onto an immune effector cell, preferably a T cell. Cells are genetically equipped with a CAR, which is a composite membrane receptor molecule and provides both targeting specificity and T cell activation. The most common form of CARs are fusions of single chain variable fragment (scFv) derived from monoclonal antibodies, fused to CD3 transmembrane- and endodomain. The CAR targets the T cell to a desired cellular target through an antibody-derived binding domain in the extracellular moiety, and T cell activation occurs via the intracellular moiety signalling domains when the target is encountered. The transfer of the coding sequence of these receptors into suitable cells, in particular T cells, is commonly facilitated by retro- or lentiviral vectors. The receptors are called chimeric because they are composed of parts from different sources.

The term "epitope" refers in the context of this invention to the functional epitope of an antigen. The functional epitope comprises those residues, typically amino acids or polysaccharides that contribute to the non-covalent interaction between the paratope of the antigen binding protein and the antigen. The non-covalent interaction comprises electrostatic forces, van der Walls forces, hydrogen bonds, and hydrophobic interaction. The functional epitope is a subgroup of the residues that constitute the structural epitope of an antigen binding protein. The structural epitope comprises all residues that are covered by an antigen binding protein, i.e. the footprint of an antigen binding protein. Typically, the functional epitope of an antigen bound by an antibody comprises 4 to 10 amino acids. Similarly, the functional epitope of a peptide that is MHC presented typically comprises 4 to 8 amino acids.

The term "expression" refers in the context of this invention to the presence of a protein or peptide, in particular a PAI or a SPA in human tissue. The term expression of a protein or peptide means that it is translated from its nucleic acid sequence into its amino acid sequence during the process of protein biosynthesis in the ribosomal machinery of the cell. The expressed protein can be located intracellularly or extracellularly, e.g. on the surface of cell. The human tissue wherein the protein is expressed may be healthy or diseased tissue.

The term "protein antigen of interest" (PAI) refers in the context of this invention to a protein or a portion of a protein or a protein complex that comprises an epitope that is specifically bound by the paratope of an antigen-binding protein. A PAI is typically a naturally occurring protein and can be of any length. It is preferred that the PAI comprises at least 25 amino acids. If that the PAI is specifically bound by a TCR accordingly, it is preferred that the length of the PAI is 8 to 12 amino acids. The PAI may be a tumor associated target antigen (TAA), a viral protein or a bacterial protein. The PAI is typically a tumor associated antigen (TAA), which is to be specifically targeted in, e.g. a tumor therapy.

The term "humanized mice" refers in the context of the present invention to genetically modified mice which carry human genes, cells, tissues and/or organs that exert their biological function, e.g. are intact regarding their biological function. Typically, immunodeficient mice are used as recipients for human cells or tissues, because they can relatively easily accept heterologous cells or tissues due to lack of host immunity. Examples of humanized mice are the nude mouse, the severe combined immunodeficiency (SCID) mouse, the NCG mouse, the NOG (NOD/Shi-*scid*/IL-2Rγ^{null}) mouse or the NSG (NOD scid gamma) mouse. Mice that accept human version of genes into their respective mouse loci are called "knock-in" mice. B-cells and T-cells can be isolated from humanized mice and be used in the methods of the present invention.

The term "T-cell receptor libraries" refers in the context of the present invention to a library that contains a high number of different T cell receptor (TCR) proteins or fragments thereof, wherein each TCR protein or fragment thereof is different.

A "viral antigenic peptide" in the context of the present invention is shorter fragment of a viral protein that is presented by a major histocompatibility complex (MHC) molecule on the surface of an antigen presenting cell, which is typically a diseased cell. The viral antigenic peptide is of a viral origin, i.e. the cell is typically infected by said virus. The viral antigenic peptide in the context of the present invention may be an antigenic peptide selected from the group consisting of human immune deficiency virus (HIV) antigenic peptides, human cytomegalovirus (HCMV) antigenic peptides, cytomegalovirus (CMV) antigenic peptides, human papillomavirus (HPV) antigenic peptides, hepatitis B virus (HBV) antigenic peptides; hepatitis C virus (HCV) antigenic peptides; Epstein-Barr virus (EBV) antigenic peptides, Influenza antigenic peptides, preferably HIV, HBV, Influenza and HCMV antigenic peptides. Viral antigenic peptides can be used in the method and the embodiments described herein include, for example, viral antigenic peptides as described in table 2 below. In one aspect, viral antigenic peptides that are used in the method and embodiment described herein include at least one viral antigenic peptide comprising or consisting of an amino acid sequence selected from the amino acid sequences of SEQ ID NO: 6 to SEQ ID NO: 8.

**Table 2: List of viral antigenic peptides**

| SEQ ID NO: | Amino acid sequence | Virus | MHC |
|---|---|---|---|
| 6 | SLYNTVATL | HIV | HLA-A*02:01 |
| 7 | GILGFVFTL | Influenza A | HLA-A*02:01 |
| 8 | NLVPMVATV | HCMV | HLA-A*02:01 |

A "bacterial antigenic peptide" in the context of the present invention is shorter fragment of a bacterial protein that is presented by an MHC molecule on the surface of an antigen presenting cell, which is typically a diseased cell. The bacterial antigenic peptide is of a bacterial origin, i.e. the cell is typically infected by a bacterium. Such bacterial antigenic peptides have been discovered in the context of infections from, for example, *Mycobacterium tuberculosis.* Accordingly, the bacterial antigenic peptide in the context of the present invention may be a *Mycobacterium tuberculosis* antigenic peptide.

The term "tumor associated antigen" (TAA) refers in the context of this invention to autologous cellular antigens derived from all protein classes, such as enzymes, receptors, transcription factors, etc. that are preferentially or exclusively expressed by tumor cells. TAA can be broadly categorized into aberrantly expressed self-antigens, mutated self-antigens, and tumor-specific antigens. TAAs that are preferentially expressed by tumor cells, are also found in normal tissues. However, their expression differs from that of normal tissues by their degree of expression in the tumor, by alterations in their protein structure in comparison with their normal counterparts, or by their aberrant subcellular localization within tumor cells. The TAA peptides that can be used in the methods and embodiments described herein include, for example, TAA peptides described in U.S. Publication 20160187351, U.S. Publication 20170165335, U.S. Publication 20170035807, U.S. Publication 20160280759, U.S. Publication 20160287687, U.S. Publication 20160346371, U.S. Publication 20160368965, U.S. Publication 20170022251, U.S. Publication 20170002055, U.S. Publication 20170029486, U.S. Publication 20170037089, U.S. Publication 20170136108, U.S. Publication 20170101473, U.S. Publication 20170096461, U.S. Publication 20170165337, U.S. Publication 20170189505, U.S. Publication 20170173132, U.S. Publication 20170296640, U.S. Publication 20170253633, U.S. Publication 20170260249, U.S. Publication 20180051080, and U.S. Publication No. 20180164315, the contents of each of these publications and sequence listings described therein. Furthermore, the TAA in the context of the present invention is a specific ligand of MHC-class-I-molecules or MHC-class-II-molecules, preferably MHC-class-I-molecules.

In an aspect, the antigen binding protein selected by the method of the present invention selectively recognize cells which present a TAA peptide described in one of more of the patents and publications listed above. In another aspect, TAA peptides that may be used in the methods and embodiments described herein include at least one TAA consisting of an amino acid sequence selected from the amino acid sequences of SEQ ID NO: 9 to 164. In an aspect, the antigen binding protein selected by the method of the present invention selectively binds cells which present a TAA peptide/MHC complex, wherein the TAA peptide comprises or consists of an amino acid sequence of SEQ ID NO: 1 to 164.Further examples of TAAs are listed in table 3.

**Table 3: List of TAAs**

| SEQ ID NO: | Amino Acid Sequence | SEQ ID NO: | Amino Acid Sequence | SEQ ID NO: | Amino Acid Sequence |
|---|---|---|---|---|---|
| 9 | YLYDSETKNA | 61 | VLDGKVAVV | 113 | KMSELQTYV |
| 10 | HLMDQPLSV | 62 | GLLGKVTSV | 114 | ALLEQTGDMSL |
| 11 | GLLKKINSV | 63 | KMISAIPTL | 115 | VIIKGLEEITV |
| 12 | FLVDGSSAL | 64 | GLLETTGLLAT | 116 | KQFEGTVEI |
| 13 | FLFDGSANLV | 65 | TLNTLDINL | 117 | KLQEEIPVL |
| 14 | FLYKIIDEL | 66 | VIIKGLEEI | 118 | GLAEFQENV |
| 15 | FILDSAETTTL | 67 | YLEDGFAYV | 119 | NVAEIVIHI |
| 16 | SVDVSPPKV | 68 | KIWEELSVLEV | 120 | ALAGIVTNV |
| 17 | VADKIHSV | 69 | LLIPFTIFM | 121 | NLLIDDKGTIKL |
| 18 | IVDDLTINL | 70 | ISLDEVAVSL | 122 | VLMQDSRLYL |
| 19 | GLLEELVTV | 71 | KISDFGLATV | 123 | KVLEHVVRV |
| 20 | TLDGAAVNQV | 72 | KLIGNIHGNEV | 124 | LLWGNLPEI |
| 21 | SVLEKEIYSI | 73 | ILLSVLHQL | 125 | SLMEKNQSL |
| 22 | LLDPKTIFL | 74 | LDSEALLTL | 126 | KLLAVIHEL |
| 23 | YLMDDFSSL | 75 | VLQENSSDYQSNL | 127 | ALGDKFLLRV |
| 24 | KVWSDVTPL | 76 | HLLGEGAFAQV | 128 | FLMKNSDLYGA |
| 25 | LLWGHPRVALA | 77 | SLVENIHVL | 129 | KLIDHQGLYL |
| 26 | KIWEELSVLEV | 78 | SLSEKSPEV | 130 | GPGIFPPPPPQP |
| 27 | LLIPFTIFM | 79 | AMFPDTIPRV | 131 | ALNESLVEC |
| 28 | FLIENLLAA | 80 | FLIENLLAA | 132 | GLAALAVHL |
| 29 | LLWGHPRVALA | 81 | FTAEFLEKV | 133 | LLLEAVWHL |
| 30 | FLLEREQLL | 82 | ALYGNVQQV | 134 | SIIEYLPTL |
| 31 | SLAETIFIV | 83 | LFQSRIAGV | 135 | TLHDQVHLL |
| 32 | TLLEGISRA | 84 | ILAEEPIYIRV | 136 | SLLMWITQC |
| 33 | ILQDGQFLV | 85 | FLLEREQLL | 137 | FLLDKPQDLSI |
| 34 | VIFEGEPMYL | 86 | LLLPLELSLA | 138 | YLLDMPLWYL |
| 35 | SLFESLEYL | 87 | SLAETIFIV | 139 | GLLDCPIFL |
| 36 | SLLNQPKAV | 88 | AILNVDEKNQV | 140 | VLIEYNFSI |
| 37 | GLAEFQENV | 89 | RLFEEVLGV | 141 | TLYNPERTITV |
| 38 | KLLAVIHEL | 90 | YLDEVAFML | 142 | AVPPPPSSV |
| 39 | TLHDQVHLL | 91 | KLIDEDEPLFL | 143 | KLQEELNKV |
| 40 | TLYNPERTITV | 92 | KLFEKSTGL | 144 | KLMDPGSLPPL |
| 41 | KLQEKIQEL | 93 | SLLEVNEASSV | 145 | ALIVSLPYL |
| 42 | SVLEKEIYSI | 94 | GVYDGREHTV | 146 | FLLDGSANV |
| 43 | RVIDDSLVVGV | 95 | GLYPVTLVGV | 147 | ALDPSGNQLI |
| 44 | VLFGELPAL | 96 | ALLSSVAEA | 148 | ILIKHLVKV |
| 45 | GLVDIMVHL | 97 | TLLEGISRA | 149 | VLLDTILQL |
| 46 | FLNAIETAL | 98 | SLIEESEEL | 150 | HLIAEIHTA |
| 47 | ALLQALMEL | 99 | ALYVQAPTV | 151 | SMNGGVFAV |
| 48 | ALSSSQAEV | 100 | KLIYKDLVSV | 152 | MLAEKLLQA |
| 49 | SLITGQDLLSV | 101 | ILQDGQFLV | 153 | YMLDIFHEV |
| 50 | QLIEKNWLL | 102 | SLLDYEVSI | 154 | ALWLPTDSATV |
| 51 | LLDPKTIFL | 103 | LLGDSSFFL | 155 | GLASRILDA |
| 52 | RLHDENILL | 104 | VIFEGEPMYL | 156 | ALSVLRLAL |
| 53 | GLPSATTTV | 105 | ALSYILPYL | 157 | SYVKVLHHL |
| 54 | GLLPSAESIKL | 106 | FLFVDPELV | 158 | VYLPKIPSW |
| 55 | KTASINQNV | 107 | SEWGSPHAAVP | 159 | NYEDHFPLL |
| 56 | SLLQHLIGL | 108 | ALSELERVL | 160 | VYIAELEKI |
| 57 | YLMDDFSSL | 109 | SLFESLEYL | 161 | VHFEDTGKTLLF |
| 58 | LMYPYIYHV | 110 | KVLEYVIKV | 162 | VLSPFILTL |
| 59 | KVWSDVTPL | 111 | VLLNEILEQV | 163 | HLLEGSVGV |
| 60 | LLWGHPRVALA | 112 | SLLNQPKAV | | |

Furthermore, the TAA antigenic peptide in the context of the present invention is a specific ligand of MHC-class-I-molecules or MHC-class-II-molecules, preferably MHC-class-I-molecules.

The term "tumor-specific antigen" refers in the context of this invention to antigens that are exclusively expressed on tumor cells. They include neo-antigens that arise due to mutations, e.g. point mutations or frame-shift mutations, in the tumor cell. Examples for tumor specific antigens are p53 or BCR-ABL.

The term "MHC" refers in the context of this invention to the abbreviation for the phrase "major histocompatibility complex". MHC's are a set of cell surface receptors that have an essential role in establishing acquired immunity against altered natural or foreign proteins in vertebrates, which in turn determines histocompatibility within a tissue. The main function of MHC molecules is to bind to antigens derived from altered proteins or pathogens and display them on the cell surface for recognition by appropriate T-cells. The human MHC is also called HLA (human leukocyte antigen) complex or HLA. The MHC gene family is divided into three subgroups: class I, class II, and class III. Complexes of peptide and MHC class I are recognized by CD8-positive T-cells bearing the appropriate TCR, whereas complexes of peptide and MHC class II molecules are recognized by CD4- positive-helper-T-cells bearing the appropriate TCR. Since both types of response, CD8 and CD4 dependent, contribute jointly and synergistically to the anti-tumor effect, the identification and characterization of tumor-associated antigens and corresponding TCRs is important in the development of cancer immunotherapies such as vaccines and cell therapies.

The term "MHC-I" refers in the context of the present invention to MHC class I molecules or MHC-I. The MHC I molecule consists of an alpha chain, also referred to as MHC I heavy chain and a beta chain, which constitutes a beta 2 microglobulin molecule. The alpha chain, comprises three alpha domains, i.e. alpha1 domain, alpha2 domain and alpha3 domain. Alpha1 and alpha2 domain mainly contribute to forming the peptide pocket to produce a peptide ligand MHC (pMHC) complex. MHC-I typically bind peptides that are derived from cytosolic antigenic proteins and which are degraded by the proteasome after ubiquitylation and subsequently transported through a specific transporter associated with antigen processing (TAP) from the cytosol to the endoplasmatic reticulum (ER). MCH I typically binds peptides of 8-12 amino acids in length.

The term "MHC-II" refers in the context of the present invention to MHC class II molecules or MHC-II. The MHC-II molecule consists of an alpha and a beta chain, wherein the alpha chain comprises two alpha domains, alpha1 domain, alpha2 domain and the beta chain comprises two beta domains, beta domain1 and beta domain2. MHC II typically fold in the ER in complex with a protein called invariant chain and are then transported to late endosomal compartments where the invariant chain is cleaved by cathepsin proteases and a short fragment remains bound to the peptide-binding groove of MHC II, termed class II-associated invariant chain peptide (CLIP). This placeholder peptide is then normally exchanged against higher affinity peptides, which are derived from proteolytically degraded proteins available in endocytic compartments. MHC-II typically binds peptides of 10-30 amino acids in length or peptides of 13-25 amino acids in length.

The term "HLA" refers in the context of the present invention to molecules which differ between different human beings in amino acid sequence. However, HLAs can be identified by an internationally agreed nomenclature, the IMGT nomenclature, of HLA. The HLA-A gene is located on the short arm of chromosome 6 and encodes the larger, α-chain, constituent of HLA-A. Variation of HLA-A α-chain is key to HLA function. This variation promotes genetic diversity in the population. Since each HLA has a different affinity for peptides of certain structures, greater variety of HLAs means greater variety of antigens to be 'presented' on the cell surface. Each individual can express up to two types of HLA-A, one from each of their parents. Some individuals will inherit the same HLA-A from both parents, decreasing their individual HLA diversity. However, the majority of individuals receive two different copies of HLA-A. The same pattern follows for all HLA groups. In other words, every single person can only express either one or two of the 2432 known HLA-A alleles coding for currently 1740 active proteins. HLA-A*02 signifies a specific HLA allele, wherein the letter A signifies to which HLA gene the allele belongs to and the prefix "*02 prefix" indicates the A2 serotype. In MHC class I dependent immune reactions, peptides not only have to be able to bind to certain MHC class I molecules expressed by tumor cells, they subsequently also have to be recognized by T-cells bearing specific TCRs.

The term "target peptide" (TP) refers in the context of this invention to a shorter peptide, part or fragment of the protein antigen of interest (PAI). The amino acid sequence of a target peptide comprises typically 8-12 amino acids in length, 8-11 amino acids in length or 8-10 amino acids in length. Preferably, the amino acid sequence of a target peptide comprises typically 8-11 amino acids in length. The target peptide may be bound to an MHC-I molecule or an MHC-II molecule. Whether a target peptide binds to an MHC-I or MHC-II molecule depends on the target peptide's natural origin, i.e. whether it is synthesized in the cytoplasm and processed in the proteasome or absorbed by endocytosis and subsequently processed. Moreover, it depends on the length of the target peptide whether it will bind to the binding groove of an MHC-I or an MHC-II molecule. In one example a target peptide of a length of 8-12, 8-11 or 8-10 amino acids is typically bound to a MHC-I. In another example, the amino acid sequence of a target peptide may comprise 13-23 amino acids in length, preferably 13-18 amino acids in length. A target peptide of a length of 13-25 or 13-18 amino acids is typically bound to an MHC-II.

The term "antigen complex" (AC) refers in the context of this invention to a complex comprising an antigen that is directly or indirectly, e.g. through an MHC or peptide binding part thereof, attached to the surface of a carrier or a soluble multimerized MHC or peptide binding part thereof. Such a carrier can be a cell or synthetic material. If the antigen is attached to a cell, the cell may be an antigen-presenting cells (APCs), preferably a human APC. Synthetic materials for carriers can be beads or particles, preferably microbeads, microparticles or nanoparticles. Such beads can be magnetic or paramagnetic beads. Beads or microparticles are usually made of polymers and can be covalently or non-covalently coated with a first member of a pair of coupling residues. The second member of the pair of coupling residues is covalently or non-covalently coupled to the MHC or peptide binding part thereof. A preferred pair of first and second coupling residues comprises streptavidin and biotin member. The skilled person is aware of other pairs of coupling residues. Accordingly, in a preferred embodiment the carrier may be coated with streptavidin which will allow the immobilization of MHC molecules or peptide binding parts thereof that comprise a biotin moiety. Conversely, a carrier coated with biotin allows the immobilization of MHC molecules or peptide binding parts thereof that comprise a streptavidin moiety. A soluble multimerized MHC or peptide binding part thereof may comprise two or more MHCs, wherein each is covalently or non-covalently, preferably covalently coupled to a third member of a pair of coupling residues and a fourth member of a pair or coupling residues, wherein the fourth member has at least two binding sites for the third member, preferably 3, 4, 5, 6, 7, or 8 binding sites and particularly preferred 4 binding sites. Biotin is a preferred third member of a pair of coupling residues and streptavidin is a preferred fourth member of a pair of coupling residues. Streptavidin has four binding sites for biotin. Thus, if MHC peptide complexes comprising biotin are contacted with streptavidin a soluble tetramer will form in which four peptide loaded MHCs (or peptide binding fragments thereof) are non-covalently bound to streptavidin. Thus, in a preferred embodiment the soluble multimerized MHC or peptide binding fragment thereof is a complex comprising four MHC peptide complexes, wherein each of the MHC peptide complex is attached covalently to one biotin, which are in turn bound non-covalently to streptavidin.

The term "pair of coupling residues" refers to two entities that specifically and noncovalently bind to each other with high affinity. Preferably, the K_{d} is less than 10⁻¹⁰ mol/L, more preferably less than 10⁻¹¹ mol/L, more preferably less than 10-12 mol/L and even more preferably less than 10⁻¹³ mol/L. Preferably, at least one of the members of a binding pair has a molecular weight below 500 g/mol/. Such a molecule can be attached covalently to one chain of the MHC or peptide binding fragment thereof without interfering with the ability of the MHC to interact with a TCR. Preferred pairs of coupling residues are biotin-streptavidin, and biotin-avidin. Alternatively, one member of a pair of coupling residues can be a protein that is fused to one chain of an MHC. Examples include chitin binding protein (CBP), maltose binding protein (MBP), Strep-tag glutathione-S-transferase (GST), poly(His) tag, V5-tag, Myc-tag, HA-tag, Spot-tag, T7-tag and NE-tag. The other member of the pair is determined by the respective protein tag, i.e. chitin, maltose, biotin, glutathione, metal matrix, e.g. Ni-matrix, or an antibody that specifically binds to the V5-, Myc-, HA-, Spot-, T7- or NE-tag.

The term "similar protein antigen" (SPA) refers in the context of this invention to a protein or a portion of a protein or a protein complex that comprises an epitope bound by the paratope of an antigen binding protein. The amino acid sequence of the SPA is determined by the PAI. The amino acid sequence of the SPA differs in at least one amino acid from the amino acid sequence of the given PAI, i.e. the PAI of interest. It serves the purpose of identifying antigen binding proteins that bind the PAI and at the same time the SPA, i.e. that do not exhibit the desired specificity and/or selectivity to the PAI. Such antigen binding proteins may elicit off-tumor/off target toxicity. For a given PAI and antigen binding protein combination, the SPA falls into one of three categories:

### (1) Similar amino acid sequence, identical epitope:

If the amino acids of the SPA that differ with respect to the PAI do not contribute to the epitope bound by a given PAI-specific antigen binding protein then the antigen binding protein will bind with the same affinity both to the PAI and the SPA. An antigen binding protein with this property will be counter selected by the methods of the present invention.

### (2) Similar amino acid sequence, similar epitope:

If at least one of the amino acids of the SPA that differ with respect to the PAI contributes to the epitope bound by a given PAI-specific antigen binding protein than the antigen binding protein will bind with a different affinity to the PAI and the SPA. An antigen binding protein that exhibits significantly lower binding to the SPA than to the PAI may be selected by the methods of the present invention. In this respect significantly lower binding means that the difference between the binding to the PAI and the SPA is at least 2-fold, at least 3-fold, at least 4-fold, at least 5-fold, at least 6-fold, at least 7-fold, at least 8-fold, at least 9-fold, at least 10-fold, at least 15-fold, at least 20-fold, at least 30-fold, at least 40-fold, at least 50-fold, at least 70-fold, at least 100-fold, at least 200-fold, preferably at least 50-fold, more preferably at least 100-fold at identical concentration of the PAI and the SPA.

### (3) Similar amino acid sequence, different epitope:

If the diverging amino acids are located at positions that contributes to the epitope bound by a given PAI-specific antigen binding protein then the antigen binding protein may not bind to the SPA at all. An antigen binding protein with this property will be selected by the methods of the present invention.

The amino acid sequences of the SPAs are generally based on the amino acid sequences of naturally occurring proteins, since such proteins may be expressed on healthy tissue of a tumor patient. Preferably, the SPA is a naturally occurring protein or a fragment thereof. In particular the SPA is present in the same species as the PAI. Thus, it is desired that the SPAs included in the method of the invention have amino acid sequences that allow identification and counterselection of antigen binding proteins in category (1) and (2). The SPA is only likely to allow the counterselection of unsuitable antigen binding proteins if its amino acid sequence is closely related to the amino acid sequence of the PAI. It is, thus, preferred that the amino acid sequence of the SPA used in the method of the present invention has a similarity to the amino acid sequence of the PAI of at least 50%, at least 60%, at least 70%, at least 80%, of at least 90% or at least 95%. Thus, in a preferred embodiment the SPA differs by 1-20, more preferably by 2-10 amino acids from the amino acid sequence of the PAI. It is preferred that the SPA, in particular the target similar peptide (TSP) used in the method of the invention is expressed on healthy tissue, preferably with more than 10 copies per cell, preferably more than 20 copies per cell, preferably more than 50 copies per cell and preferably more than 100 copies per cell. The relative strength of expression can be determined by a variety of art known methods including FACS analysis of healthy and diseased cells with fluorescently labeled antigen binding proteins or mass spectrometry. Gene expression analysis can also be performed using RNA sequencing approaches. Another criteria for the selection of a SPA to be used in the method of the invention is its frequency of presentation on primary normal tissues. The frequency describes how often a SPA is presented on normal, i.e. a healthy tissue - in contrast to the copy number which defines the number of SPAs of a given healthy tissue, for example a cell. Together with the copy number the frequency is an important criterion to select a SPA for a given PAI. The higher the similarity to the PAI and the higher the presentation frequency and the copy number per cell (CpC) on normal tissues, the higher the relevance of a SPA.

The term "target similar peptide" (TSP) refers in the context of this invention to a shorter peptide, part or fragment of the SPA. The amino acid sequence of a TSP comprises typically 8 to 16 amino acids in length. The TSP is typically MHC presented. Similarly to the SPA, the TSP has a similarity to the amino acid sequence of the TP of at least 50%, at least 60%, at least 70%, at least 80%, at least 90%. The TSPs typically have a length of 8 to 16 amino acids, such as 8-12, preferably of 8 to 11 amino acids. In particular, TSPs have a length of 8 to 11 or 8-12 amino acids when they bind to MHC-I. In another example, TSPs typically have a length of 13 to 25 amino acids when they bind to MHC-II. TSPs may differ in one or more amino acids from the TP in as long as they meet the similarity scores outlined above and which may be determined as explained below. Thus, if the amino acid sequence of a TSP of 8 amino acids length is aligned to a given TP it may comprise between 1 to 8 amino acids that are similar to the corresponding amino acids of the TP. The other amino acids may be identical or dissimilar to the TP. Accordingly, a TSP of 9 amino acids length may comprise between 1 to 9 amino acids that are similar to the corresponding amino acids of the TP; a TSP of 10 amino acids length may comprise between 1 to 10 amino acids that are similar to the corresponding amino acids of the TP; a TSP of 11 amino acids length may comprise between 1 to 11 amino acids that are similar to the corresponding amino acids of the TP, a TSP of 12 amino acids length may comprise between 1 to 12 amino acids that are similar to the corresponding amino acids of the TP. If the TSP is bound to MHC II it typically has a length of 13 to 25 amino acids and accordingly, a TSP of 13 amino acids length may comprise between 1 to 13 amino acids that are similar to the corresponding amino acids of the TP; a TSP of 14 amino acids length may comprise between 1 to 14 amino acids that are similar to the corresponding amino acids of the TP; a TSP of 15 amino acids length may comprise between 1 to 15 amino acids that are similar to the corresponding amino acids of the TP, a TSP of 16 amino acids length may comprise between 1 to 16 amino acids that are similar to the corresponding amino acids of the TP, a TSP of 17 amino acids length may comprise between 1 to 17 amino acids that are similar to the corresponding amino acids of the TP; a TSP of 18 amino acids length may comprise between 1 to 18 amino acids that are similar to the corresponding amino acids of the TP; a TSP of 19 amino acids length may comprise between 1 to 19 amino acids that are similar to the corresponding amino acids of the TP, a TSP of 20 amino acids length may comprise between 1 to 20 amino acids that are similar to the corresponding amino acids of the TP. Another criteria for the selection of a TSP to be used in the method of the present invention is its frequency of presentation on primary normal tissues. The frequency of presentation describes how often a TSP is presented on normal, i.e. a healthy tissue - in contrast to the copy number which defines the number of TSPs on different samples of a given healthy tissue, e.g. if a certain TSP is detected on 6 out of 12 samples of adipose tissue is has a frequency of presentation of 50%. The frequency of presentation of a given TSP can be determined by art known methods including MS analysis as used in Example 4 (see Fig. 6 to 8, which indicate frequency of presentation for three different TSPs). Thus, it is preferred that a TSP is used in the method of the invention, which has a frequency of presentation of at least 10% in at least one healthy tissue, preferably, at least 20% in at least one healthy tissue, more preferably at least 30% in at least one healthy tissue. Preferably, the selected TSP is presented in at least one, preferably at least two, more preferably at least three healthy tissues. These tissues are preferably selected from those that were analyzed regarding their presentation of TSP1, and TSP2, respectively, in Figs. 6 and 7.

Together with the copy number the frequency is an important criterion to select a TSP for a given TP. The higher the similarity to the TP and the higher the presentation frequency and CpC on normal tissues, the higher the relevance of a TSP.

The term "irrelevant antigen complex" (IAC) refers in the context of this invention to an AC comprising an irrelevant protein antigen (IPA). Such an AC can be, e.g. an APC, or a multimerized MHC-peptide complex that is soluble or MHC-peptide complexes immobilized on a carrier. The irrelevant protein antigen is defined in the following.

The term "irrelevant protein antigen" (IPA) refers in the context of this invention to a protein antigen which is not bound by a selected TCR. TCRs are screened for binding their respective target peptides. Upon binding of a TCR to its target peptide a desired immune reaction or T-cell mediated immune response is triggered. Such a desired immune response will not be triggered by an irrelevant peptide because an irrelevant peptide will not be bound by a TCR in the screening. The IPA may be a protein encoded by a housekeeping gene. Typically, the IPA has a similarity to the amino acid sequence of the TP of at least less than 50%, at least less than 40%, at least less than 30%, at least less than 20%, at least less than 10%.

The term "irrelevant peptide" (IP) refers in the context of this invention to a shorter peptide, part or fragment of the IPA. The amino acid sequence of an IP comprises typically 8-16 amino acids in length. Such an IP is typically bound to MHC-I. In some examples, when an IP is bound to a MHC-II, an IP may comprise 13-25 amino acids in length. An IP may also comprise 13-18 amino acids in length when bound to a MHC-II. The IP may be encoded by a housekeeping gene.

"Housekeeping genes" in the context of this invention are typically constitutive genes that are required for the maintenance of basic cellular function and are expressed in all cells of an organism under normal and pathophysiological conditions. Although some housekeeping genes are expressed at relatively constant rates in most non-pathological situations, the expression of other housekeeping genes may vary depending on experimental conditions. Housekeeping genes account for majority of the active genes in the genome, and their expression is obviously vital to survival. The housekeeping gene expression levels are fine-tuned to meet the metabolic requirements in various tissues. Examples for housekeeping genes are listed (non-exhaustive) as follows: Transcription factor, translation factors, repressor molecules, RNA splicing molecules, RNA binding proteins, ribosomal proteins, mitochondrial ribosomal proteins, RNA polymerases, protein processing genes, heat shock proteins, histone, cell cycle, apoptosis, oncogenes, DNA repair, DNA replication, metabolism involved genes, e.g. genes involved in carbohydrate metabolism, citrate cycle, lipid metabolism, amino acid metabolism, NADH dehydrogenase, cytochrome C oxidase, ATPase, lysosome, proteasome, ribonuclease, thioreductases, receptors, channels, transporters, HLA/immunoglobulin/cell recognition, kinases, cytoskeletal, growth factors, tumor necrosis factor α. Similarly to the IPA, the IP has a similarity to the amino acid sequence of the TP of at least less than 50%, at least less than 40%, at least less than 30%, at least less than 20%, at least less than 10%, preferably at least less than 30%, at least less than 20%, at least less than 10%.

The term "amino acid sequence identity" refers in the context of this invention to the percentage of sequence identity and is determined by comparing two optimally aligned sequences over a comparison window, wherein the portion of the sequence in the comparison window can comprise additions or deletions (i.e. gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The term "identical" refers in the context of two or more polypeptide or nucleic acid sequences, refers to two or more sequences or subsequences that are the same, i.e. comprise the same sequence of amino acids or nucleic acids. Sequences are "substantially identical" to each other if they have a specified percentage of amino acid residues that are the same (e.g., at least 70%, at least 75%, at least 80, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity over a specified region), when compared and aligned for maximum correspondence over a comparison window, or designated region as measured using one of the following sequence comparison algorithms or by manual alignment and visual inspection. These definitions also refer to the complement of a test sequence. Accordingly, the term "at least 80% sequence identity" is used throughout the specification with regard to polypeptide and polynucleotide sequence comparisons. This expression preferably refers to a sequence identity of at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% to the respective reference polypeptide or to the respective reference polynucleotide.

The term "sequence comparison" refers in the context of this invention to the process wherein one sequence acts as a reference sequence, to which test sequences are compared. When using a sequence comparison algorithm, test and reference sequences are inputted into a computer, if necessary subsequence coordinates are designated, and sequence algorithm program parameters are designated. Default program parameters are commonly used, or alternative parameters can be designated. The sequence comparison algorithm then calculates the percent sequence identities or similarities for the test sequences relative to the reference sequence, based on the program parameters. In case where two sequences are compared and the reference sequence is not specified in comparison to which the sequence identity percentage is to be calculated, the sequence identity is to be calculated with reference to the longer of the two sequences to be compared, if not specifically indicated otherwise. If the reference sequence is indicated, the sequence identity is determined on the basis of the full length of the reference sequence indicated by SEQ ID, if not specifically indicated otherwise.

In a sequence alignment, the term "comparison window" refers to those stretches of contiguous positions of a sequence which are compared to a reference stretch of contiguous positions of a sequence having the same number of positions. It is preferred that the entire length of the PAI, preferably the TP is used as comparison window in the alignment with the SPA and TSP, respectively. If the TP is, e.g. a 10 amino acid long MHC 1 presented peptide the similarity is determined in a comparison window of 10 amino acids. In this case a 9 amino acids long SPA with one amino acid mismatch has an identity of 80% to the given TP.

Methods of alignment of sequences for comparison are well known in the art. Optimal alignment of sequences for comparison can be conducted, for example, by the local homology algorithm of Smith and Waterman (Adv. Appl. Math. 2:482, 1970), by the homology alignment algorithm of Needleman and Wunsch (J. Mol. Biol. 48:443, 1970), by the search for similarity method of Pearson and Lipman (Proc. Natl. Acad. Sci. USA 85:2444, 1988), by computerized implementations of these algorithms (e.g., GAP, BESTFIT, FASTA, and TFASTA in the Wisconsin Genetics Software Package, Genetics Computer Group, 575 Science Dr., Madison, Wis.), or by manual alignment and visual inspection (see, e.g., Ausubel et al., Current Protocols in Molecular Biology (1995 supplement)). Algorithms suitable for determining percent sequence identity and sequence similarity are the BLAST and BLAST 2.0 algorithms, which are described in Altschul et al. (Nuc. Acids Res. 25:3389-402, 1977), and Altschul et al. (J. Mol. Biol. 215:403-10, 1990), respectively. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al.*, supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) or 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff, Proc. Natl. Acad. Sci. USA 89:10915, 1989) alignments (B) of 50, expectation (E) of 10, M=5, N=-4, and a comparison of both strands.

The term "similarity" of two amino acid sequences takes into consideration the relatedness of two amino acids at a given position (see, for example below Table 4). The similarity of two amino acid sequences, e.g. in a TP and a TSP, can be determined using the BLAST algorithm, which performs a statistical analysis of the similarity between two sequences (see, e.g., Karlin and Altschul, Proc. Natl. Acad. Sci. USA 90:5873-87, 1993). Preferred settings for such an alignment are: wordlength of 3, and expectation (E) of 10, and the use of the BLOSUM62 or PMBEC scoring matrix (Kim et al. 2009 BMC Bioinformatics), preferably the PMBEC scoring matrix is used in the determination of the similarity. These matrices quantify amino acid similarity based for example on evolutionary or functional similarity between amino acids, which correlate well with the similarity according to physicochemical parameters. For each substitution of an amino acid in a given TP sequence a score (decimal value) can be calculated by using these matrices, which indicates the similarity of the amino acid in the TP sequence with the substituted amino acid in the TSP sequence. Multiple substitutions can be considered by summing up the effect (scores) of individual substitutions in the TP sequence. By definition, the maximum score which can be achieved for a TSP is provided by the unsubstituted TP sequence, whereas any substitution leading to a TSP will be penalized in the scoring matrix and ultimately lead to a lower score of a TSP. This maximum score is however dependent on the length and amino acid sequence of the TP (i.e. different TP sequences will have different maximum scores). Typically, a longer amino acid sequences results in a higher score. However, the score of a TP depends on the score allotted to the amino acids it consists of. In order to be able to calculate and compare the similarity of a TSP in reference to a TP without considering the difference of maximum scores of distinct TP sequences the respective decimal values are converted, which are the result of calculating the similarity of a TSP in reference to a TP, into a percentage score wherein the maximum score of a TP sequence will therefore, always be 100%.

Another measure of similarity provided by the BLAST algorithm is the smallest sum probability (P(N)), which provides an indication of the probability by which a match between amino acid sequences would occur by chance. For example, an amino acid is considered similar to a reference sequence if the smallest sum probability in a comparison of the test amino acid to the reference amino acid is less than about 0.2, typically less than about 0.01, and more typically less than about 0.001. Semi-conservative and especially conservative amino acid substitutions, wherein an amino acid is substituted with a chemically related amino acid are preferred. Typical substitutions are among the aliphatic amino acids, among the amino acids having aliphatic hydroxyl side chain, among the amino acids having acidic residues, among the amide derivatives, among the amino acids with basic residues, or the amino acids having aromatic residues. Typical semi-conservative and conservative substitutions are indicated in below Table 4.

**Table 4: Amino acids and conservative and semi-conservative substitutions, respectively.**

| Amino acid | Conservative substitution | Semi-conservative substitution |
|---|---|---|
| A | G; S; TN; V; C | |
| C | A; V; L | M; I; F; G |
| D | E; N; Q | A; S; T; K; R; H |
| E | D; Q; N | A; S; T; K; R; |
| F | W; Y; L; M; H | I; V; A |
| G | A | S; N; T; D; E; N; Q |
| H | Y; F; K; R | L; M; A |
| I | V; L; M; A | F; Y; W; G |
| K | R; H | D; E; N; Q; S; T; A |
| L | M; I; V; A | F; Y; W; H; C |
| M | L; I; V; A | F; Y; W; C; |
| N | Q | D; E; S; T; A; G; K; R |
| P | V; I | L; A; M; W; Y; S; T; C; F |
| Q | N | D; E; A; S; T; L; M; K; R |
| R | K; H | N; Q; S; T; D; E; A |
| S | A; T; G; N | D; E; R; K |
| T | A; S; G; N; V D; E; R; K; I | |
| V | A; L; IM; T; C; N | |
| W | F; Y; H | L; M; I; V; C |
| Y | F; W; H | L; M; I; V; C |

Changing from A, F, H, I, L, M, P, V, W or Y to C is semi-conservative if the new cysteine remains as a free thiol. Furthermore, the skilled person will appreciate that glycines at sterically demanding positions should not be substituted and that P should not be introduced into parts of the protein which have an alpha-helical or a beta-sheet structure.

The term "detectable label" refers in the context of this invention to a molecule which labels a different molecule or a cell by allowing this different molecule to be selected due to a property or specific characteristics the label exerts. Molecules that are eligible for labelling are proteins, DNA or RNA or synthetic materials such as beads or other suitable materials. Regarding proteins, labeling strategies result in the covalent attachment of different molecules, including biotin, reporter enzymes, fluorophores, magnetic labels and radioactive isotopes, to the target protein or peptide or nucleotide sequence. Single-cell can be labeled using short DNA or RNA barcode 'tags' to identify reads that originate from the same cell in a sequencing experiment. Labels may be a fluorescent label, e.g. xanthens, acridines, oxazines, cynines, styryl dyes, coumarines, porphines, metal-ligand-complexes, fluorescent proteins, nanocrystals, perylenes and phtalocyanines, phycoerythrin (SA-PE), streptavidin-allophycocyanin (SA-APC) or streptavidin-brilliant-violet 421 (SA-BV421); RNA-barcodes or DNA-barcodes or a radioactive label. A radioactive label is typically a molecule wherein one or more atoms are replaced by the radioactive counterparts, i.e. radio isotopes. Proteins, peptides, DNA or RNA may be labeled radioactively. Magnetic labels may comprise magnetic beads or magnetic nanoparticles whjch can be coated with e.g. antibodies against a particular surface antigen. Magnetic labels may be used in magnetic-activated cell sorting (MACS).

The term "detectably different" refers in the context of this invention to a scenario wherein two labels are present but may only be different in the signal they are emitting. For example, two cells may be labelled with a fluorescent label and are thus, not distinguishable by the characteristics of the label as such, i.e. the fluorescence. However, the fluorescence label attached to one cell may signal in red wherein the fluorescence signal attached to the second cell may signal in green. The two labels of the exemplified cells are thus, detectably different.

The term "flow cytometry analysis" refers in the context of this invention to a sorting technique comprising the measurement of chemical and physical properties of a specific cell populations or cell subpopulations in a sample. The sample usually is a suspension and is adjusted to result in a flow of one cell at a time through a detection unit, typically a laser beam that excites a fluorophore and a light detector. The detected signal, e.g. light scattered by the flow through of the cell, is characteristic to the cell, i.e. its components. Multiple cells can be analyzed by this technique in a short period of time. Routine applications of flow cytometry are cell counting, cell sorting, determination of cell characteristic and functions, diagnosis of diseases, e.g. cancer, detection of biomarkers or detection of microorganisms. A popular flow cytometry technique is fluorescence activated cell sorting (FACS). The FACS technique harnesses the ability to label a target cell / cells with fluorescent dye tags or labels which allows for the cell sorting based on the individual labeling profile of a particular cell population.

The term "magnetic-activated cell sorting" (MACS) refers to a sorting technique that harnesses functional micro- or nanoparticles that are conjugated with antibodies corresponding to particular cell surface antigens. Under application of a magnetic field gradient, the magnetically targeted cells can be separated in either a positive or negative fashion with the respect to the antigen employed. A skilled person is well aware of the different kind of sorting analyses.

The term "specifically binding" refers in the context of this invention to the binding of an antigen binding protein or fragments thereof, e.g. an antibody or fragments thereof or a TCR or fragments thereof, to a specific binding site of its target when the target comprises specific and non-specific binding sites. However, sometimes binding of a protein to closely related proteins is unavoidable, then the actual binding to the target may be specific but the protein is deemed to be non-specific in relation to the intended target binding. An antigen binding protein or a fragment thereof of the present invention is considered to bind specifically to a given antigen, if it binds to the antigen with a K_{d} of 10⁻⁵ M or less when measured by surface plasmon resonance (SPR) at RT. The dissociation constant (K_{d}) for the target to which the binding moiety specifically binds is at least 2-fold, at least 5-fold, at least 7-fold, 10-fold, preferably at least 20-fold, more preferably at least 50-fold, even more preferably at least 100-fold, 200-fold, 500-fold or 1000-fold lower than the dissociation constant (K_{d}) for the target to which the binding moiety of the antigen binding protein or fragment thereof does not bind specifically, for example the similar protein antigen (SPA), preferably the target similar peptide (TSP).

Typically, if the antigen binding protein of the present invention that specifically binds to a given TP is a TCR or a fragment thereof it has a K_{d} in the range of 3×10⁻⁵ to 1×10⁻⁷, 2×10⁻⁵ to 5×10⁻⁷ , 1×10⁻⁵ to 1×10⁻⁶or 5×10⁻⁶ to 1×10⁻⁶. In this situation, it is preferred that the antigen binding protein at the same time has a K_{d} for the TP, that is at least 2-fold lower, at least 5-fold, at least 7-fold, 10-fold, preferably at least 20-fold, more preferably at least 50-fold, even more preferably at least 100-fold, 200-fold, 500-fold or 1000-fold lower than the K_{d} for the target to which the binding moiety of the antigen binding protein does not bind specifically, for example the TSP. Thus, for example a selected TCR may bind to the TP with a K_{D} of 1×10⁻⁶ and to the TSP with a K_{D} of 1×10⁻⁵.

Typically, if the antigen binding protein of the present invention that specifically binds to a given TP is an affinity maturated TCR or a fragment thereof or a soluble molecule in a bispecific format or fragment thereof, such as a TCER^{®} molecule or a fragment thereof, the K_{D} in the range of 9×10⁻⁹ to 1×10⁻¹², 8×10⁻⁹ to 5×10⁻¹² , 7×10⁻⁹ to 1×10⁻¹¹, 6×10⁻⁹ to 2×10⁻¹¹, 5×10⁻⁹ to 5×10⁻¹¹, 4×10⁻⁹ to 8×10⁻¹¹, 3×10⁻⁹ to 1×10⁻¹⁰. In this situation, it is preferred that the antigen binding protein at the same time has a K_{d} for the TP, that is at least 2-fold lower, at least 5-fold, at least 7-fold, 10-fold, preferably at least 20-fold, more preferably at least 50-fold, even more preferably at least 100-fold, 200-fold, 500-fold or 1000-fold lower than the K_{d} for the target to which the binding moiety of the antigen binding protein does not bind specifically, for example the TSP. Molecules in the bispecific format referred to herein as "TCER^{®}" molecules or "TCER^{®}" typically comprise a first polypeptide that specifically binds to a surface molecule on a T cell and a second polypeptide that specifically binds to a MHC-peptide complex.

Typically, if the antigen binding protein of the present invention is an antibody or a fragment thereof or a B-cell that specifically binds to a given PAI, the K_{D} is in the range of 9×10⁻⁹ to 1×10⁻¹², 8×10⁻⁹ to 5×10⁻¹² , 7×10⁻⁹ to 1×10⁻¹¹, 6×10⁻⁹ to 2×10⁻¹¹, 5×10⁻⁹ to 5×10⁻¹¹, 4×10⁻⁹ to 8×10⁻¹¹, 3×10⁻⁹ to 1×10⁻¹⁰. In this situation, it is preferred that the antigen binding protein at the same time has a K_{d} for the PAI, that is at least 2-fold lower, at least 5-fold, at least 7-fold, 10-fold, preferably at least 20-fold, more preferably at least 50-fold, even more preferably at least 100-fold, 200-fold, 500-fold or 1000-fold lower than the K_{d} for the target to which the binding moiety of the antigen binding protein does not bind specifically, for example the SPA.

In some instances, in particular in context of TCRs, if the antigen binding protein of the present invention, in particular in context of a TCR, specifically binds to a given TP might be determined by using a functional assay, for example, in a TCR activation assay, such as an IFNγ-release assay. Accordingly, a specific binding may be characterized by a response, such as signal that is detected for a TP is more than 30%, more than 40%, more than 50%, more than 60%, more than 70%, more than 80%, more than 90%, more than 100% of the response, i.e. the signal, obtained for the TSP in such an assay.

The term "selectively binding" refers in the context of this invention to the characteristic of an antigen binding protein, such as a TCR or antibody, to selectively recognize or bind to preferably only one specific epitope and preferably shows no or substantially no binding (no cross-reactivity) to another epitope, peptide or protein. Assessing the threshold of epitope binding by flow cytometry can be assessed by using non-tetramer stained controls. The gates can be set according to the non-tetramer stained control in a way that <0.01% cells appear in this gate. This gate can be applied to a sample from the same donor that has been stained with tetramer of interest. Cells which appear in this gate are considered to bind selectively to the epitope of interest.

The term "T-cell receptor" (TCR) refers in the context of this invention to a heterodimeric cell surface protein of the immunoglobulin super-family, which is associated with invariant proteins of the CD3 complex involved in mediating signal transduction. TCRs exist in αβ and γδ forms, which are structurally similar but have quite distinct anatomical locations and probably functions. The extracellular portion of native heterodimeric αβ TCR and γδ TCR each contain two polypeptides, each of which has a membrane-proximal constant domain, and a membrane-distal variable domain. Each of the constant and variable domains include an intra-chain disulfide bond. The variable domains contain the highly polymorphic loops analogous to the complementarity determining regions (CDRs) of antibodies. The use of TCR gene therapy overcomes a number of current hurdles. It allows equipping the subjects' (patients') own T-cells with desired specificities and generation of sufficient numbers of T-cells in a short period of time, avoiding their exhaustion. The TCR will be transduced into potent T-cells (e.g. central memory T-cells or T-cells with stem cell characteristics), which may ensure better persistence, preservation and function upon transfer. TCR-engineered T-cells will be infused into cancer patients rendered lymphopenic by chemotherapy or irradiation, allowing efficient engraftment but inhibiting immune suppression. Native alpha-beta heterodimeric TCRs have an alpha chain and a beta chain. Each alpha chain comprises variable, joining and constant regions, and the beta chain also usually contains a short diversity region between the variable and joining regions, but this diversity region is often considered as part of the joining region. The constant, or C, regions of TCR alpha and beta chains are referred to as TRAC and TRBC respectively (Lefranc, (2001), Curr Protoc Immunol Appendix 1: Appendix 10). Each variable region, herein referred to as alpha variable domain and beta variable domain, comprises three "complementarity determining regions" (CDRs) embedded in a framework sequence, one being the hypervariable region named CDR3. The alpha variable domain CDRs are referred to as CDRa1, CDRa2, CDRa3, and the beta variable domain CDRs are referred to as CDRb1, CDRb2, CDRb3. There are several types of alpha chain variable (Valpha) regions and several types of beta chain variable (Vbeta) regions distinguished by their framework, CDR1 and CDR2 sequences, and by a partly defined CDR3 sequence. The Valpha types are referred to in IMGT nomenclature by a unique TRAV number, Vbeta types are referred in IMGT nomenclature to by a unique TRBV number (Folch and Lefranc, (2000), Exp Clin Immunogenet 17(1): 42-54; Scaviner and Lefranc, (2000), Exp Clin Immunogenet 17(2): 83-96; LeFranc and LeFranc, (2001), "T-cell Receptor Factsbook", Academic Press). For more information on immunoglobulin antibody and TCR genes see the international ImMunoGeneTics information system^{®}, Lefranc M-P et al (Nucleic Acids Res. 2015 Jan;43 (Database issue) : D413-22; and http://www.imgt.org/). A conventional TCR antigen-binding site usually includes six CDRs, comprising the CDR set from each of an alpha and a beta chain variable region, wherein CDR1 and CDR3 sequences are relevant to the recognition and binding of the peptide antigen that is bound to the MHC protein and the CDR2 sequences are relevant to the recognition and binding of the MHC protein. Analogous to antibodies, TCRs comprise framework regions which are amino acid sequences interposed between CDRs, i.e. to those portions of TCR alpha and beta chain variable regions that are relatively conserved among different TCRs. The alpha and beta chains of a TCR each have four FRs, herein designated FR1-a, FR2-a, FR3-a, FR4-a, and FR1-b, FR2-b, FR3-b, FR4-b, respectively. Accordingly, the alpha chain variable domain may thus be designated as (FR1-a)-(CDRa1)-(FR2-1)-(CDRa2)-(FR3-a)-(CDRa3)-(FR4-a) and the beta chain variable domain may thus be designated as (FR1-b)-(CDRb 1)-(FR2-b)-(CDRb2)-(FR3-b)-(CDRb3)-(FR4-b).

A "disease caused by a virus or bacteria" may also be referred to as a viral or bacterial infection. In the context of the present invention, the virus causing the disease may be selected from the group constituted of for example, human immunodeficiency viruses (HIV), Humane Cytomegalievirus (HCMV), cytomegalovirus (CMV), human papillomavirus (HPV), Hepatitis B virus (HBV), Hepatitis C virus (HCV), human papillomavirus infection (HPV), Epstein-Barr virus (EBV), Influenza virus, preferably human immunodeficiency viruses (HIV). In the context of the present invention, the bacteria causing the disease may be *Mycobacterium tuberculosis.* The disease caused by this bacterium is, thus, tuberculosis. It will be understood by the skilled in the art, that when the antigen binding protein targets a viral antigenic peptide, for instance, a HIV peptide, the antigen binding protein may be for use in the treatment of HIV. Accordingly, an antigen binding protein targeting the viral or bacterial antigenic peptide TA-C is thus, suitable for use in the treatment of virus or bacteria from which said antigenic viral or bacterial antigenic peptide, is derived.

The term "immune disease" refers in the context of this invention to a disease triggered by the immune system. The term "disease" refers to an abnormal condition, especially an abnormal medical condition such as an illness or injury, wherein a tissue, an organ or an individual is not able to efficiently fulfil its function anymore. In contrast, healthy tissue, organs or individuals are referred to herein if no abnormal conditions are present and the tissue, organ or individual is without pathological finding. In healthy tissues random migration of cells is absent, cells adhere to each other in structures characterizing the tissue and assist in its function. No metastasis is present in healthy tissue. Typically, but not necessarily, a disease is associated with specific symptoms or signs indicating the presence of such disease. The presence of such symptoms or signs may thus, be indicative for a tissue, an organ or an individual suffering from a disease. An alteration of these symptoms or signs may be indicative for the progression of such a disease. A progression of a disease is typically characterized by an increase or decrease of such symptoms or signs which may indicate a "worsening" or "bettering" of the disease. The "worsening" of a disease is characterized by a decreasing ability of a tissue, organ or organism to fulfil its function efficiently, whereas the "bettering" of a disease is typically characterized by an increase in the ability of a tissue, an organ or an individual to fulfil its function efficiently. A tissue, an organ or an individual being at "risk of developing" a disease is in a healthy state but shows potential of a disease emerging. Typically, the risk of developing a disease is associated with early or weak signs or symptoms of such disease. In such case, the onset of the disease may still be prevented by treatment. Examples of a disease include but are not limited to infectious diseases, traumatic diseases, inflammatory diseases, cutaneous conditions, endocrine diseases, intestinal diseases, neurological disorders, joint diseases, genetic disorders, autoimmune diseases, and various types of cancer. Healthy tissue as defined herein usually comprises or consists of healthy cells.

The term "neoplastic disease" refers in the context of this invention to diseases characterized by an abnormal growth of cells, also known as a tumor. Neoplastic diseases are conditions that cause tumor growth. Malignant tumors are cancerous and can grow slowly or quickly and carry the risk of metastasis or spreading to multiple tissues and organs. By "tumor" is meant an abnormal group of cells or tissue that grows by a rapid, uncontrolled cellular proliferation and continues to grow after the stimuli that initiated the new growth cease. Tumors show partial or complete lack of structural organization and functional coordination with the normal tissue, and usually form a distinct mass of tissue, which may be either benign or malignant. A neoplastic disease may result in cancer, wherein exemplified cancer type diseases include but are not limited to Basal cell carcinoma, Bladder cancer, Bone cancer, Brain tumor, Breast cancer, Burkitt lymphoma, Cervical cancer, Colon Cancer, Cutaneous T-cell lymphoma, Esophageal cancer, Retinoblastoma, Gastric (Stomach) cancer, Gastrointestinal stromal tumor, Glioma, Hodgkin lymphoma, Kaposi sarcoma, Leukemias, Lymphomas, Melanoma, Oropharyngeal cancer, Ovarian cancer, Pancreatic cancer, Pleuropulmonary blastoma, Prostate cancer, Throat cancer, Thyroid cancer, and Urethral cancer.

The term "treating" or "treatment" refers in the context of the present invention to a therapeutic use, e.g. for a subject in need thereof, i.e. suffering a disease or disorder) and means reversing, alleviating, inhibiting the progress of one or more symptoms of such a disease, disorder or condition. Therefore, treatment does not only refer to a treatment that leads to a complete cure of the disease, but also to treatments that slow down the progression of the disease and/or prolong the survival of the subject.

The term "immune cell specific surface marker" refers in the context of this invention to cell surface antigens, which serve as monograms to help identify and classify immune cells. Examples of such markers that characterize different T-cell subtypes are indicated in Table 1 above. The majority of immune cell specific surface markers are molecules or antigens within cell's plasma membrane. These molecules serve not only as markers but they also have key functional roles.

The term "growth factor" or "differentiation factor" is used in the context of this invention interchangeably and refers to molecules that are capable of stimulation cellular growth, cell proliferation and cellular differentiation and regulate multiple cellular processes. Growth factors are usually proteins or steroid hormones. Examples of prevailing molecules are listed in the following (non-exhaustive enumeration): Growth factors, such as colony stimulating factor( CSF), Macrophage colony-stimulating factor (M-CSF), Granulocyte colony-stimulating factor (G-CSF) and Granulocyte macrophage colony-stimulating factor (GM-CSF); epidermal growth factor (EGF); erythropoietin (EPO); fibroblast growth factor (FGF); foetal bovine somatotropin (FBS); hepatocyte growth factor (HGF); insulin; insulin like growth factor (IGF); interleukins; neuregulins; neutrotrophins; T-cell growth factor (TCGF); transforming growth factor (TGF); tumor necrosis factor alpha (TNFα); vascular endothelial growth factor (VEGF).

The term "nucleic acid" refers in the context of this invention to single or double-stranded oligo- or polymers of deoxyribonucleotide or ribonucleotide bases or both. Nucleotide monomers are composed of a nucleobase, a five-carbon sugar (such as but not limited to ribose or 2'-deoxyribose), and one to three phosphate groups. Typically, a nucleic acid is formed through phosphodiester bonds between the individual nucleotide monomers, In the context of the present invention, the term nucleic acid includes but is not limited to ribonucleic acid (RNA) and deoxyribonucleic acid (DNA) molecules but also includes synthetic forms of nucleic acids comprising other linkages (e.g., peptide nucleic acids as described in Nielsen et al. (Science 254:1497-1500, 1991). Typically, nucleic acids are single- or double-stranded molecules and are composed of naturally occurring nucleotides. The depiction of a single strand of a nucleic acid also defines (at least partially) the sequence of the complementary strand. The nucleic acid may be single or double stranded or may contain portions of both double and single stranded sequences. Exemplified, double-stranded nucleic acid molecules can have 3' or 5' overhangs and as such are not required or assumed to be completely double-stranded over their entire length. The nucleic acid may be obtained by biological, biochemical or chemical synthesis methods or any of the methods known in the art, including but not limited to methods of amplification, and reverse transcription of RNA. The term nucleic acid comprises chromosomes or chromosomal segments, vectors (e.g., expression vectors), expression cassettes, naked DNA or RNA polymer, primers, probes, cDNA, genomic DNA, recombinant DNA, cRNA, mRNA, tRNA, microRNA (miRNA) or small interfering RNA (siRNA). A nucleic acid can be, e.g., single-stranded, double-stranded, or triple-stranded and is not limited to any particular length. Unless otherwise indicated, a particular nucleic acid sequence comprises or encodes complementary sequences, in addition to any sequence explicitly indicated.

The term "vector" refers in the context of this invention to a polynucleotide that encodes a protein of interest or a mixture comprising polypeptide(s) and a polynucleotide that encodes a protein of interest, which is capable of being introduced or of introducing proteins and/or nucleic acids comprised therein into a cell. Examples of vectors include but are not limited to plasmids, cosmids, phages, viruses or artificial chromosomes. A vector is used to introduce a gene product of interest, such as e.g. foreign or heterologous DNA into a host cell. Vectors may contain "replicon" polynucleotide sequences that facilitate the autonomous replication of the vector in a host cell. Foreign DNA is defined as heterologous DNA, which is DNA not naturally found in the host cell, which, for example, replicates the vector molecule, encodes a selectable or screenable marker, or encodes a transgene. Once in the host cell, the vector can replicate independently of or coincidental with the host chromosomal DNA, and several copies of the vector and its inserted DNA can be generated. In addition, the vector can also contain the necessary elements that permit transcription of the inserted DNA into an mRNA molecule or otherwise cause replication of the inserted DNA into multiple copies of RNA. Vectors may further encompass "expression control sequences" that regulate the expression of the gene of interest. Typically, expression control sequences are polypeptides or polynucleotides such as promoters, enhancers, silencers, insulators, or repressors. In a vector comprising more than one polynucleotide encoding for one or more gene products of interest, the expression may be controlled together or separately by one or more expression control sequences. More specifically, each polynucleotide comprised on the vector may be control by a separate expression control sequence or all polynucleotides comprised on the vector may be controlled by a single expression control sequence. Polynucleotides comprised on a single vector controlled by a single expression control sequence may form an open reading frame. Some expression vectors additionally contain sequence elements adjacent to the inserted DNA that increase the half-life of the expressed mRNA and/or allow translation of the mRNA into a protein molecule. Many molecules of mRNA and polypeptide encoded by the inserted DNA can thus be rapidly synthesized. Such vectors may comprise regulatory elements, such as a promoter, enhancer, terminator and the like, to cause or direct expression of said polypeptide upon administration to a subject. Examples of promoters and enhancers used in the expression vector for animal cell include early promoter and enhancer of SV40 (Mizukami T. *et al.* 1987), LTR promoter and enhancer of Moloney mouse leukemia virus (Kuwana Y *et al.* 1987), promoter (Mason JO *et al.* 1985) and enhancer (Gillies SD *et al.* 1983) of immunoglobulin H chain and the like. Any expression vector for animal cell can be used, as long as a gene encoding the human antibody C region can be inserted and expressed. Examples of suitable vectors include pAGE107 (Miyaji H *et al.* 1990), pAGE103 (Mizukami T *et al.* 1987), pHSG274 (Brady G *et al.* 1984), pKCR (O'Hare K *et al.* 1981), pSG1 beta d2-4-(Miyaji H *et al*. 1990) and the like. Other examples of plasmids include replicating plasmids comprising an origin of replication, or integrative plasmids, e.g. pUC, pcDNA, pBR.

The term "antigen binding part" or "or antigen binding fragment" in the context of the present invention refers to molecules, in particular amino acid chains, that are shorter in length but which retain the binding specificity and/or selectivity of the parent protein because it still comprises the essential amino acid sequence or sequences which are responsible for the binding specificity and/or selectivity of the parent protein. An "antigen binding part" or "or antigen binding fragment" is considered to have retained the binding specificity, if it's K_{d} to the target of the parent protein measured as outlined below is at least 10-fold higher or less, 5-fold higher or less, 3-fold higher or less 2-fold higher or less or identical to the K_{d} of the parent protein. Antigen binding fragments of TCRs are, e.g. the variable domains of the alpha and beta chain, and antigen binding parts of antibodies are the variable light and heavy chain. Antigen binding parts of a TCR, BCR or an antibody are the CDRs that are positioned in the respective variable regions of the alpha and beta or light and heavy chain. Thus, if the Assessment of binding and/or specificity of an antigen binding protein, e.g., an antibody, TCR, BCR or immunologically functional part or fragment thereof, can be conducted by binding affinity measurements of e.g. a TCR to its target peptide or an antibody to its antigen. The term "fragment" used herein refers to naturally occurring fragments (e.g. splice variants or peptide fragments) as well as artificially constructed fragments, in particular to those obtained by gene-technological means.

The term "K_{d}" (measured in "mol/L", sometimes abbreviated as "M") in the context of the present invention refers to the dissociation equilibrium constant of the particular interaction between a binding moiety (e.g. an antibody or fragment thereof) and a target molecule (e.g. an antigen or epitope thereof). Affinity can be measured by common methods known in the art, including but not limited to surface plasmon resonance (SPR) based assay (such as the BIAcore assay); biolayer interferometry (BLI), enzyme-linked immunoabsorbent assay (ELISA); and competition assays (e.g. radio immuno assays (RIA)). Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for the purposes of the present invention. The K_{d}'s indicated for various antigen binding proteins throughout this disclosure are measured at room temperature, i.e. 20°C, by SPR.

The term "B-cell receptor" (BCR) refers to a receptor with an antigen like structure present on the surface of B cells. A B-cell is activated by its first encounter with an antigen that binds to its receptor (its "cognate antigen"), the cell proliferates and differentiates to generate a population of antibody-secreting plasma B-cells and memory B-cells. The BCR controls B-cell activation by biochemical signaling and by physical acquisition of antigens from immune synapses with antigen-presenting cells and has two crucial functions upon interaction with the antigen. One function is signal transduction, involving changes in receptor oligomerization. The second function is to mediate internalization for subsequent processing of the antigen and presentation of peptides to helper T-cells. The portion of the BCR that recognizes antigens is made up of three disparate genetic regions, termed V, D, and J, that are spliced and recombined at the genetic level in a combinatorial process unique to the immune system. The immunoglobulin molecules that form a type 1 transmembrane receptor protein are usually located on the outer surface of a B-lymphocyte. Structurally, the BCR comprises a membrane-bound immunoglobulin molecule of one isotype (IgD, IgM, IgA, IgG, or IgE) and a signal transduction moiety: A Ig-α/Ig-β (CD79) heterodimer, linked by disulfide bridges. Each member of the dimer spans the plasma membrane and has a cytoplasmic tail bearing an immunoreceptor tyrosine-based activation motif (ITAM).

The term "antibody" in the context of the present invention refers to secreted immunoglobulins which lack the transmembrane region and can thus, be released into the bloodstream and body cavities. Human antibodies are grouped into different isotypes based on the heavy chain they possess. There are five types of human Ig heavy chains denoted by the Greek letters: α, γ, δ, ε, and µ. · The type of heavy chain present defines the class of antibody, i.e. these chains are found in IgA, IgD, IgE, IgG, and IgM antibodies, respectively, each performing different roles, and directing the appropriate immune response against different types of antigens. Distinct heavy chains differ in size and composition; and may comprise approximately 450 amino acids (Janeway et al. (2001) Immunobiology, Garland Science). IgA is found in mucosal areas, such as the gut, respiratory tract and urogenital tract, as well as in saliva, tears, and breast milk and prevents colonization by pathogens (Underdown & Schiff (1986) Annu. Rev. Immunol. 4:389-417). IgD mainly functions as an antigen receptor on B-cells that have not been exposed to antigens and is involved in activating basophils and masT-cells to produce antimicrobial factors (Geisberger et al. (2006) Immunology 118:429-437; Chen et al. (2009) Nat. Immunol. 10:889-898). IgE is involved in allergic reactions via its binding to allergens triggering the release of histamine from masT-cells and basophils. IgE is also involved in protecting against parasitic worms (Pier et al. (2004) Immunology, Infection, and Immunity, ASM Press). IgG provides the majority of antibodybased immunity against invading pathogens and is the only antibody isotype capable of crossing the placenta to give passive immunity to fetus (Pier et al. (2004) Immunology, Infection, and Immunity, ASM Press). In humans there are four different IgG subclasses (IgGl, 2, 3, and 4), named in order of their abundance in serum with IgGl being the most abundant (-66%), followed by IgG2 (-23%), IgG3 (-7%) and IgG (-4%). The biological profile of the different IgG classes is determined by the structure of the respective hinge region. IgM is expressed on the surface of B-cells in a monomeric form and in a secreted pentameric form with very high avidity. IgM is involved in eliminating pathogens in the early stages of B-cell mediated (humoral) immunity before sufficient IgG is produced (Geisberger et al. (2006) Immunology 118:429-437). Antibodies are not only found as monomers but are also known to form dimers of two Ig units (e.g. IgA), tetramers of four Ig units (e.g. IgM of teleost fish), or pentamers of five Ig units (e.g. mammalian IgM). Antibodies are typically made of four polypeptide chains comprising two identical heavy chains and identical two light chains which are connected via disulfide bonds and resemble a "Y"-shaped macro-molecule. Each of the chains comprises a number of immunoglobulin domains out of which some are constant domains and others are variable domains. Immunoglobulin domains consist of a 2-layer sandwich of between 7 and 9 antiparallel ∼-strands arranged in two ∼-sheets. Typically, the heavy chain of an antibody comprises four Ig domains with three of them being constant (CH domains: CHI. CH2. CH3) domains and one of the being a variable domain (VH). The light chain typically comprises one constant Ig domain (CL) and one variable Ig domain (V L). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. Term antibody as used herein also encompasses a chimeric antibody, a humanized antibody or a human antibody.

The term "antigen-binding fragment" of an antibody, TCR, or BCR or CAR (or "binding portion" or "fragment"), as used herein, refers to one or more fragments of an antibody TCR, BCR or CAR that retain the ability to specifically bind to an antigen. It has been shown that the antigen-binding function of an antibody, of a TCR, of a BCR or CAR can be performed by fragments of a full-length antibody, TCR, BCR or CAR. Examples of binding fragments of antibodies encompassed within the term "antigen-binding portion of an antibody, BCR or CAR "include (i) Fab fragments, monovalent fragments consisting of the VL, VH, CL and CH domains; (ii) F(ab')₂ fragments, bivalent fragments comprising two Fab fragments linked by a disulfide bridge at the hinge region; (iii) Fd fragments consisting of the VH and CH domains; (iv) Fv fragments consisting of the VL and VH domains of a single arm of an antibody, (v) dAb fragments (Ward et al., (1989) Nature 341: 544-546), which consist of a VH domain; (vi) isolated complementarity determining regions (CDR), and (vii) combinations of two or more isolated CDRs which may optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242: 423-426; and Huston et al. (1988) Proc. Natl. Acad. Sci. USA 85: 5879-5883). Such single chain antibodies are also intended to be encompassed within the term "antigen-binding fragment" of an antibody, BCR or CAR. The term "antigen binding portion of a TCR" comprises at least CDR1 and CDR3 of the alpha and beta chain of a TCR, preferably CDR1, CDR2 and CDR3 of the alpha and beta chain. While these CDRs are preferably comprised in the context of their natural framework regions, they may also be comprised in another protein - a so called protein scaffold - that positions them to each other in a similar way as they are positioned in an alpha and/or beta chain. The antigen binding portion of a TCR comprises preferably the variable domain of the alpha and beta chain. The antigen binding fragments of antibodies, TCRs, BCRs or CARs can be included in a monomeric, dimeric, trimeric, tetrameric or multimeric protein complex to provide such complex with one or more different antigen binding specificities. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242: 423-426; and Huston et al. (1988) Proc. Natl. Acad. Sci. USA 85: 5879-5883). Such single chain antibodies are also intended to be encompassed within the term "antigen-binding fragment of an antibody". Further formats in which antigen binding fragments of an antibody are used to create monovalent, bivalent or multivalent binding molecules are known in the art and are termed diabody, a tetrabody, and a nanobody. Similarly, to scFV's single chain TCRs comprise the variable domains of alpha and beta chain on one protein chain linked by a linker.

As used in this specification the term "subject" relates to an "individual", "subject", or "patient" which are used interchangeably herein and refer to any mammal that may benefit from the present invention. In particular, the "individual" is a human being. The subject can be a healthy subj ect.

The term "subject in need thereof" in the context of this invention refers to a subject that suffers or is at risk of suffering a disease, for example a proliferative disease or disorder, a disease caused by a virus or a disease caused by bacteria. Such a proliferative disease or disorder, for example cancer, involve the unregulated and/or inappropriate proliferation of cells. The proliferative disorder or disease may be, for example, a tumor disease characterized by the expression of the TAA, more particular of the TAA, in a cancer or tumor cell of said tumor disease.

Accordingly, a particularly preferred cancer is a TA positive cancer, in particular a TAA positive cancer.

Abbreviations of frequently used terms throughout the claims and specification of the present invention:

**Table 5: Abbreviations of frequently used terms.**

| | |
|---|---|
| Antigen complex | AC |
| B-cell receptor | BCR |
| Chimeric antigen receptor | CAR |
| Complementary determining regions | CDR |
| Fluorescence activated cell sorting | FACS |
| Human leukocyte antigen | HLA |
| Irrelevant antigen complex | IAC |
| Irrelevant peptide | IP |
| Irrelevant protein antigen | IPA |
| Magnetic activated cell sorting | MACS |
| Major histocompatibility complex I/II | MHC I / II |
| Protein antigen of interest | PAI |
| Similar protein antigen | SPA |
| T-cell receptor | TCR |
| Target peptide | TP |
| Target similar peptide | TSP |
| Tumor-associated antigen | TAA |

### Embodiments

In the following different aspects of the invention are defined in more detail.

Immunotherapy constitutes an exciting and rapidly evolving field, and the demonstration that genetically modified T-cell receptors (TCRs) can be used to produce T-lymphocyte populations of desired specificity offers new opportunities for antigen-specific T-cell therapy. Overall, TCR-modified T-cells have the ability to target a wide variety of self and non-self-targets through the normal biology of a T-cell. However, "off-tumor/on-target" or "off-tumor/off-target" effects can lead to tremendously undesired effects of immune related toxicity. By including similar protein antigens (similar peptides) already at the stage of identification of TCRs, the inventors are able to exclude a proportion of cross-reactive T-cells before the characterization process and by that enhancing the efficiency of the whole TCR discovery procedure. For that purpose, 1D-labeled or 2D-labeled similar-peptide multimers are included to the staining panel. An in-house database allowed the inventors to identify highly relevant, target-sequence similar peptides found on normal human tissue. Such target-sequence similar peptides pose a safety risk if recognized by a TCR that is supposed to be developed towards clinical use. Therefore, the inventors have developed an in-house search algorithm that combines public and in house genomic database searches for target-similar peptides with results of actual MS-detected peptides on healthy tissue from the in-house database. The inventors use a set of target-similar peptides early during TCR identification, enabling early de-selection of cross-reactive TCRs. For this purpose, fluorochrome (streptavidin) labelled peptide major histocompatibility complex (pMHC) tetramers are generated both for the target peptide as well as for target-similar peptides, distinguishable by at least one different fluorochrome. Cells positive for both the target as well as the similar peptide are excluded from T-cell sorting for downstream TCR identification.

This surprising finding provides *inter alia* the following advantages over the art: (i) reduction of cross-reactivity of selected TCRs with similar peptides on healthy tissues (ii) increased safety profile of selected TCRs; (iii) efficient and fast identification and characterization of TCRs due to early stage selection of target specific TCRs; (v) specific TCR selection by exclusion of similar peptide binding during sorting (vi) TCRs exerting reduced off target and off-tumor cytotoxicity; and (vii) the provision of improved specific, selective and safe TCRs.

A first aspect of the invention relates to a method for selecting a eukaryotic cell expressing on its surface an antigen-binding protein specifically and/or selectively binding to a protein antigen of interest (PAI) comprising the following steps:
(i) providing a eukaryotic cell population;
(ii) contacting the eukaryotic cell population of step (i) with a first antigen complex (1^{st} AC) comprising the PAI and a detectable label A or with the PAI comprising a detectable label A;
(iii) contacting the eukaryotic cell population of step (i) with at least a second antigen complex (2^{nd} AC) comprising a similar protein antigen (SPA), wherein the amino acid sequence of the SPA differs by at least 1-20 amino acids from the amino acid sequence of the PAI and wherein the 2^{nd} AC comprises a detectable label B; or with the SPA and a detectable label B; and
(iv) selecting at least cell or a virus that specifically and/or selectively binds to the 1^{st} AC,
wherein the detectable label A and the detectable label B are detectably different from each other, and wherein the selected eukaryotic cell is a T-cell, a B-cell, or a mammalian cell expressing a heterologous antigen binding protein selected from the group consisting of a T-cell receptor (TCR) or antigen binding fragments thereof, a B-cell receptor (BCR) or antigen binding fragments thereof, and a chimeric antigen receptor (CAR) or antigen binding fragments thereof.

In one embodiment of the first aspect of the invention a cell is selected based on the principle of counterselection: MHC-presented short peptides of tumor antigens (TP) that are preferably expressed on diseased tissue are labeled with a detectable label and peptides with a similar sequence (TSP) that are expressed on healthy tissues are labeled with a detectable label. The labels used in this approach are detectably different. Upon contacting a cell, preferably a T-cell, with the TP and the TSP, the cell binds to either the TP, the TSP or to both the TP and the TSP or none and is either selected based on a positive selection criterion or on a negative selection criterion. In a conventional sorting approach, the following cells with their respective detectable cell signal can be identified: One cell can be detected by detecting the signal of the TP's label, i.e. the peptide of interest in case a cell is bound to the MHC presented peptide. Another cell can signal by the detection of the TP's label, i.e. the peptide of interest in case an immune cell is bound to the MHC presented peptide and by the detection of the TSP's label. A third cell can solely signal by the detection of the TSP's label. Positively selected are only those cells which are detectable by the TP's label because, in case the cell is a T-cell, this is the cell with a TCR of interest capable of binding to the MHC-presented peptides. The counterselection (or negative selection) can be described as follows: Cells detected by two labels, i.e. the label of TP and the TSP are negatively selected because these cells bind to the TSP beside binding to the TP. Cells only detected by the TSP's label are also negatively selected as they do only bind the TSP which is expressed on healthy tissue and generally, binding to TSP should be avoided in order to decrease off target effects. Often, the binding of a given cell to a given PAI, preferably a TP and one or more SPAs, preferably TSPs, is not all or nothing. Thus, the selection may also be based on relative differences of the binding of the PAI, preferably a TP, and a SPA, preferably a TSP. Cells are considered to specifically bind to a PAI, preferably a TP, if their binding is at least 2-fold, at least 3-fold, at least 4-fold, at least 5-fold, at least 6-fold, at least 7-fold, at least 8-fold, at least 9-fold, at least10-fold, at least 15-fold, at least 20-fold, at least 30-fold, at least 40-fold, at least 50-fold, at least 70-fold, at least 100-fold, at least 200-fold stronger at identical concentration of the PAI and the SPA. Preferably, the binding is at least 10-fold stronger to the PAI, preferably the TP, than to any of the one or more SPAs, preferably TSPs used in the method of the invention. It is even more preferably, that the binding is at least 20-fold, more preferably at least 50-fold stronger. The strength of the binding or affinity of a given cell, for example a T-cell or its TCR, or a B-cell can be determined by a variety of assays and is commonly indicated as the dissociation constant (K_{d}) of the TCR. However, for the purpose of the method of the present invention an exact determination of the K_{d} of the cell to a given PAI, preferably a TP and one or more SPAs, preferably TSPs, is not required. It is sufficient to determine relative binding strength, which can be, for example, determined by FACS analysis of cells in which the fluorescence signal of a given TP with the fluorescence signal of one or more TSPs is compared. In such a determination the relative molar amounts of the PAI, preferably the TP, and of the one or more SPA, preferably TSPs, has to be taken into consideration. If TP and TSPs are added to the cells at the same molar amounts and differences in fluorescent intensity of the respective labels used are accounted for, a cell specifically binds to a PAI, preferably TP, if an equimolar basis of the SPA, preferably TSP, shows at least 10-fold stronger fluorescence attributable to the PAI, preferably to the TP, than to the SPA, preferably TSP. The skilled person in the art understands that a change of the molar ratios of PAI, preferably TP, and the SPAs, preferably TSPs, which are contacted with the cell population in steps (ii) and (iii) can be accounted for when selecting the cells by adapting the gating accordingly.

In one embodiment of the first aspect of the present invention the selected eukaryotic cell is a mammalian cell that expresses a heterologous antigen binding protein. The mammalian cell can be any mammalian cell, such as a human cell, a mouse cell, preferably a humanized mouse cell, a rat cell, a pig cell, a monkey cell or a dog cell. Typically, the mammalian cell can be any antigen presenting cell (APC). Preferably, the mammalian cell is a human cell. In particular, the mammalian cell is engineered to express a heterologous antigen binding protein, such as a TCR or fragments thereof, or a BCR or fragments thereof or a CAR or fragments thereof or an antibody or fragments thereof.

According to the present invention the method for selecting a eukaryotic cell comprises in step (i) providing a eukaryotic cell population. Preferably, eukaryotic cells are mammalian cells expressing a library of heterologous antigen binding proteins.

Another embodiment of the first aspect the present invention relates to a method for selecting an immune cell expressing on its surface an antigen-binding protein specifically and/or selectively binding to a protein antigen of interest (PAI) comprising the following steps:
(i) providing a cell population comprising immune cells;
(ii) contacting the cell population of step (i) with a first antigen complex (1^{st} AC) comprising the PAI and a detectable label A or with the PAI comprising a detectable label A;
(iii) contacting the cell population of step (i) with at least a second antigen complex (2^{nd} AC) comprising a similar protein antigen (SPA), wherein the amino acid sequence of the SPA differs by at least 1 amino acid from the amino acid sequence of the PAI and wherein the 2^{nd} AC comprises a detectable label B; or with the SPA and a detectable label B; and
(iv) selecting at least one immune cell that specifically and/or selectively binds to the 1^{st} AC,
wherein the detectable label A and the detectable label B are detectably different from each other.

In one embodiment of the first aspect of the invention an immune cell is selected based on the principle of counterselection: MHC-presented short peptides of tumor antigens (TP) that are preferably expressed on diseased tissue are labeled with a detectable label and peptides with a similar sequence (TSP) that are expressed on healthy tissues are labeled with a detectable label. The labels used in this approach are detectably different. Upon contacting an immune cell, preferably a T-cell, with the TP and the TSP, the immune cell binds to either the TP, the TSP or to both the TP and the TSP or none and is either selected based on a positive selection criterion or on a negative selection criterion. In a conventional sorting approach, the following cells with their respective detectable cell signal can be identified: One cell can be detected by detecting the signal of the TP's label, i.e. the peptide of interest in case an immune cell is bound to the MHC presented peptide. Another cell can signal by the detection of the TP's label, i.e. the peptide of interest in case an immune cell is bound to the MHC presented peptide and by the detection of the TSP's label. A third cell can solely signal by the detection of the TSP's label. Positively selected are only those cells which are detectable by the TP's label because, in case the immune cell is a T-cell, this is the cell with a TCR of interest capable of binding to the MHC-presented peptides. The counterselection (or negative selection) can be described as follows: Cells detected by two labels, i.e. the label of TP and the TSP are negatively selected because these cells bind to the TSP beside binding to the TP. Cells only detected by the TSP's label are also negatively selected as they do only bind the TSP which is expressed on healthy tissue and generally, binding to TSP should be avoided in order to decrease off target effects. Often, the binding of a given immune cell to a given PAI, preferably a TP and one or more SPAs, preferably TSPs, is not all or nothing. Thus, the selection may also be based on relative differences of the binding of the PAI, preferably a TP, and a SPA, preferably a TSP. Immune cells are considered to specifically bind to a PAI, preferably a TP, if their binding is at least 2-fold, at least 3-fold, at least 4-fold, at least 5-fold, at least 6-fold, at least 7-fold, at least 8-fold, at least 9-fold, at least10-fold, at least 15-fold, at least 20-fold, at least 30-fold, at least 40-fold, at least 50-fold, at least 70-fold, at least 100-fold, at least 200-fold stronger at identical concentration of the PAI and the SPA. Preferably, the binding is at least 10-fold stronger to the PAI, preferably the TP, than to any of the one or more SPAs, preferably TSPs used in the method of the invention. It is even more preferably, that the binding is at least 20-fold, more preferably at least 50-fold stronger. The strength of the binding or affinity of a given immune cell, e.g. a T-cell or its TCR can be determined by a variety of assays and is commonly indicated as the dissociation constant (K_{d}) of the TCR. However, for the purpose of the method of the present invention an exact determination of the K_{d} of the immune cell to a given PAI, preferably a TP and one or more SPAs, preferably TSPs, is not required. It is sufficient to determine relative binding strength, which can be, for example, determined by FACS analysis of immune cells in which the fluorescence signal of a given TP with the fluorescence signal of one or more TSPs is compared. In such a determination the relative molar amounts of the PAI, preferably the TP, and of the one or more SPA, preferably TSPs, has to be taken into consideration. If TP and TSPs are added to the immune cells at the same molar amounts and differences in fluorescent intensity of the respective labels used are accounted for, an immune cell specifically binds to a PAI, preferably TP, if an equimolar basis of the SPA, preferably TSP, shows at least 10-fold stronger fluorescence attributable to the PAI, preferably to the TP, than to the SPA, preferably TSP. The skilled person in the art understands that a change of the molar ratios of PAI, preferably TP, and the SPAs, preferably TSPs, which are contacted with the cell population in steps (ii) and (iii) can be accounted for when selecting the cells by adapting the gating accordingly.

In one embodiment, the cell population of step (i) is contacted with at least a second antigen complex (2^{nd} AC) comprising a SPA, preferably a TSP, at least a third antigen complex (3^{rd} AC) comprising a SPA, preferably a TSP, at least a fourth antigen complex (4^{th} AC) comprising a SPA, preferably a TSP, at least a fifth antigen complex (5^{tth} AC) comprising a SPA, preferably a TSP, at least a sixth antigen complex (6^{th} AC) comprising a SPA, preferably a TSP, at least a seventh antigen complex (7^{th} AC) comprising a SPA, preferably a TSP, at least a eighth antigen complex (8^{th} AC) comprising a SPA, preferably a TSP, at least a ninth antigen complex (9^{th} AC) comprising a SPA, preferably a TSP, at least a fifth antigen complex (10^{th} AC) comprising a SPA, preferably a TSP. Accordingly, it is preferred that not more than ten SPAs, preferably TSPs, are used, not more than nine SPAs, preferably TSPs are used, not more than eight SPAs, preferably TSPs, are used, not more than seven SPAs, preferably TSPs are used, not more than six SPAs, preferably TSPs are used, not more than five SPAs, preferably TSPs are used, not more than four SPAs, preferably TSPs, are used, not more than three SPAs, preferably TSPs are used, not more than two SPAs, preferably TSPs, are used or not more than one SPA, preferably one TSP is used. Accordingly, it is preferred that in the method of the present invention between 1-10 different TSPs are used, preferably 2-8 different TSPs and more preferably 3-5 different TSPs. In an even more preferred embodiment, three TSPs are used.

In one embodiment the method of the first aspect of the invention selects an immune cell. Preferably this immune cell is a T-cell or a B-cell. More preferably, the T-cell is a CD4 T-cell. In an even more preferred embodiment the T-cell is a CD8 T-cell. The signaling domain preferably comprises CD3. In another embodiment of the first aspect of the invention the immune cell expresses on its surface an antigen binding protein. It is preferred that the antigen binding protein is a TCR or an antigen binding fragment thereof if the immune cell to be selected is a T-cell. It is preferred that the antigen binding protein is a BCR or an antigen binding fragment thereof if the immune cell to be selected is a B-cell. Such an antigen recognition or antigen binding site is preferably a single chain variable fragment (scFv) and preferably targets a PAI that is a TAA. The costimulatory domain preferably comprises CD28 or 4-1 BB. The signaling domain preferably comprises CD3. It is also preferred that the antigen binding protein is a CAR or an antigen binding fragment thereof if the immune cell to be selected is a T-cell.

In another embodiment of the first aspect of the invention the method for selecting an immune cell comprises in step (i) providing a cell population comprising immune cells. The cell population comprising immune cells is derived from peripheral blood of healthy subjects. In another embodiment, the cell population comprising immune cells is derived from peripheral blood from diseased subjects. Preferably the cell population comprising immune cells is derived from an immune cell enriched fraction of the peripheral blood of a healthy or diseased subject. Preferably, the immune cell enriched fraction is enriched in stem cells, T-cells, B-cells or plasma cells. It is even more preferred that the immune enriched fraction is enriched in CD4 T-cells and/or CD8 T-cells. In another embodiment the cell population comprising immune cells can be derived from tumor-infiltrating lymphocytes (TILs) or TCR libraries. Preferably, the TCR library contains a high number of different T cell receptor (TCR) proteins or fragments thereof, wherein each TCR protein or fragment thereof is different.

In another embodiment of the first aspect of the invention the immune cell or cells in the immune cell enriched fraction are selected by detectably labeling one or more immune cell specific surface markers. It is preferred that the immune cell surface markers are selected from the group consisting of CD3, CD8, CD4 and CD19.

In another embodiment of the first aspect of the invention, the cell population of step (i) a can be incubated in the presence of growth factors and/or cytokines in a further step. Preferably, cytokines are interleukins. More preferably, interleukins are selected from the group consisting of IL-1, IL-2, IL-7, IL-10, IL-12, Il-15, IL-17, IL-21 and IL-23. Most preferably, the cell population of step (i) is incubated with IL-2, IL-7, IL-15 and/or IL-21.

In another embodiment the protein antigen of interest (PAI) is a tumor associated antigen (TAA), a viral protein or a bacterial protein. It is preferred that if the PAI is a target peptide, i.e. a shorter fragment of the PAI, the target peptide is a viral antigenic peptide or a bacterial antigenic peptide. In another embodiment of the first aspect of the invention the diseased subject suffers from a disease selected from the group consisting of an immune disease, a neoplastic disease, a disease cause by a virus or a disease caused by bacteria. Preferably the neoplastic disease is cancer. Preferably, diseases caused by a virus is a viral infection; and a disease caused by bacteria is a bacterial infection. Preferably, the viral infection is caused by a virus selected from the group consisting of HIV, HCMV, CMV, HPV, HBV, HCV, HPV, EBV, Influenza virus. More preferably the viral infection is caused by HIV. Preferably, the bacterial infection is caused by Mycobacterium tuberculosis. Such a disease is tuberculosis.

In another preferred embodiment of the first aspect the method comprises in step (ii) contacting the cell population of step (i) with a first antigen complex (1^{st} AC) comprising the PAI and a detectable label A or with the PAI and a detectable label A. The 1^{st} AC comprising the PAI and the label A is preferably an antigen presenting cell. In another preferred embodiment the 1^{st} AC is a complex comprising a particle, the PAI and the detectable label A. More preferably, the particle is a nano- or a microbead. It is also preferred that an MHC molecule is linked to a nanoor microbead. In another preferred embodiment the 1^{st} AC consists of the PAI and the detectable label A. In another embodiment the 1^{st} AC is a complex comprising a particle, the SPA and the detectable label B. More preferably, the particle is a nano- or a microbead. It is also preferred that an MHC molecule is linked to a nano- or microbead. In another preferred embodiment the 1^{st} AC consists of the SPA and the detectable label B.

Alternatively, the PAI may comprising a detectable label. This is a preferred embodiment, if the PAI is an amino acid chain and the label is covalently linked to this amino acid chain. Examples are fluorescent labels or fluorescent proteins as GFP or EGFP. In the latter case it is preferred that the fluorescent proteins are linked to the PAI by a peptide bond.

In another preferred embodiment the method of the first aspect of the invention further comprises one or more of the steps of contacting the cell population with a further AC comprising a further PAI and a further detectable label and a further AC comprising a further SPA and a further detectable label. Using a third AC comprising a further PAI and a further detectable label C and a fourth AC comprising a further SPA and a further detectable label D mirrors the so called 2D Multimer multiplexing (2DMM) approach which allows specific rare cell detection with high sensitivity (0.0001%) in a highly cell saving manner. Two multimers labeled with different fluorochromes for each specificity (peptide MHC) enable the identification of up to 36 different specificities in one sample by using 9 different fluorochromes. In one preferred embodiment the cell population of step (i) is contacted with a third antigen complex (3^{rd} AC) comprising the PAI and a detectable label C that is detectably different from one or more or all of the other detectable labels of the other ACs contacted with the cell population. Preferably the label is detectably different from at least the detectable label A, preferably from at least the detectable label A and a detectable label D, if a detectable label D is present. A detectable label D is present if the cell population of step (i) is contacted with a fourth antigen complex (4^{th} AC) comprising the PAI and a detectable label D that is detectably different from one or more or all of the other detectable labels of the other ACs contacted with the cell population. The detectable label D is preferably detectably different from at least the detectable label A. It is also preferred that the detectable label D is detectably different from at least the detectable label A and the detectable label C. In one embodiment the cell population of step (i) is contacted with a fifth antigen complex (5^{th} AC) comprising the SPA and a detectable label E that is detectably different from one or more or all of the other detectable labels of the other ACs contacted with the cell population. It is preferred that the label E is detectably different from at least the detectable label B. It is also preferred that the detectable label E is detectably different from at least the detectable label B and a detectable label F, (6) if a detectable label F is present. A detectable label F is present if the cell population of step (i) is further contacted with a sixth antigen complex (6^{th} AC) comprising the SPA and a detectable label F that is detectably different from one or more or all of the other detectable labels of the other ACs contacted with the cell population. It is preferred that the label F is detectably different from at least the detectable label B. It is also preferred that the detectable label F is detectably different from at least the detectable label B and the detectable label E.

In another embodiment the cell population of step (i) is contacted with a first antigen complex (1^{st} AC) comprising the PAI, preferably a TP, and a detectable label A; and with at least a second antigen complex (2^{nd} AC) comprising a similar protein antigen (SPA), preferably a TSP, wherein the amino acid sequence of the SPA, preferably the TSP, differs by at least 1 amino acid from the amino acid sequence of the PAI, preferably the TP, and wherein the 2^{nd} AC comprises a detectable label B which is detectably different to label A; and with one to ten, i.e. one, two, three, four, five, six, seven, eight, nine or ten, preferably two to four, most preferably two further antigen complexes (ACs), wherein each comprises a different SPA, preferably a different TSP, that differs in at least one amino acid sequence from the amino acid sequence of the SPA of the 2^{nd} AC, and wherein each further AC comprises one or more labels, wherein the one or more label is detectably different to the one or more labels of the 2^{nd} AC. It is preferred that the 1^{st} AC comprises a further, second label which is detectably different to the one or more labels of the 2^{nd} AC and to the one or more labels of the further ACs. It is also preferred that the cell population is a T-cell population. It is further preferred that the selected immune cell that specifically and/or selectively binds to the 1^{st} AC is a T-cell. It is also preferred that the antigen-binding protein on the surface of the selected T-cell which is specifically and/or selectively binding to the PAI is a TCR.

In another embodiment the cell population of step (i) is contacted with a first antigen complex (1^{st} AC) comprising the PAI, preferably a TP, and a detectable label A; and with at least a second antigen complex (2^{nd} AC) comprising a similar protein antigen (SPA), preferably a TSP, wherein the amino acid sequence of the SPA, preferably the TSP, differs by at least 1 amino acid from the amino acid sequence of the PAI, preferably the TP, and wherein the 2^{nd} AC comprises a detectable label B; and with one to ten, i.e. one, two, three, four, five, six, seven, eight, nine or ten, preferably two to four, most preferably two further antigen complexes (AC) wherein each comprises a different SPA, preferably a different TSP, that differs in at least one amino acid sequence from the amino acid sequence of the SPA of the 2^{nd} AC, and wherein each further AC comprises one or more labels, wherein the one or more labels is the same as the one or more labels of the 2^{nd} AC. It is also preferred that the cell population is a T-cell population. It is further preferred that the selected immune cell that specifically and/or selectively binds to the 1^{st} AC is a T-cell. It is also preferred that the antigen-binding protein on the surface of the selected T-cell which is specifically and/or selectively binding to the PAI is a TCR.

In another embodiment the cell population of step (i) is contacted with a first antigen complex (1^{st} AC) comprising the PAI, preferably a TP, and a detectable label A; and with at least a second antigen complex (2^{nd} AC) comprising a similar protein antigen (SPA), preferably a TSP, wherein the amino acid sequence of the SPA, preferably the TSP, differs by at least 1 amino acid from the amino acid sequence of the PAI, preferably the TP, and wherein the 2^{nd} AC comprises a detectable label B which is detectably different to label A and the 1^{st} AC comprises at least one further detectable label and the 2^{nd} AC comprises at least one further detectable label, which are the same. It is preferred that the cell population is a T-cell population. It is further preferred that the selected immune cell that specifically and/or selectively binds to the 1^{st} AC is a T-cell. It is also preferred that the antigen-binding protein on the surface of the selected T-cell which is specifically and/or selectively binding to the PAI is a TCR.

In another embodiment the cell population of step (i) is contacted with a first antigen complex (1^{st} AC) comprising the PAI, preferably a TP, and a detectable label A; and with at least a second antigen complex (2^{nd} AC) comprising SPA, preferably a TSP, wherein the amino acid sequence of the SPA, preferably the TSP, differs by at least 1 amino acid from the amino acid sequence of the PAI, preferably the TP, and wherein the 2^{nd} AC comprises a detectable label B which is detectably different to label A and the 1^{st} AC comprises at least one further detectable label and the 2^{nd} AC comprises at least one further detectable label, which are different. It is preferred that the cell population is a T-cell population. It is further preferred that the selected immune cell that specifically and/or selectively binds to the 1^{st} AC is a T-cell. It is also preferred that the antigen-binding protein on the surface of the selected T-cell which is specifically and/or selectively binding to the PAI is a TCR.

In another embodiment the cell population of step (i) is contacted with a first antigen complex (1^{st} AC) comprising the PAI, preferably a TP, and a detectable label A; and with at least a second antigen complex (2^{nd} AC) comprising a similar protein antigen (SPA), preferably a TSP, wherein the amino acid sequence of the SPA, preferably the TSP, differs by at least 1 amino acid from the amino acid sequence of the PAI, preferably the TP, and wherein the 2^{nd} AC comprises a detectable label B which is detectably different to label A and the 1^{st} AC comprises at least one further detectable label and the 2^{nd} AC comprises at least one further detectable label, which are the same and the cell population of step (i) is contacted with one or more further antigen complexes (ACs) wherein each comprises a SPA that differs in at least one amino acid sequence from the amino acid sequence of the SPA of the 2^{nd} AC the one or more further AC comprises at least one further detectable label; wherein the at least one further label is selected in such that it allows to distinguish the 1^{st} AC from the 2^{nd} AC and the one or more further ACs. It is preferred that the cell population is a T-cell population. It is further preferred that the selected immune cell that specifically and/or selectively binds to the 1^{st} AC is a T-cell. It is also preferred that the antigen-binding protein on the surface of the selected T-cell which is specifically and/or selectively binding to the PAI is a TCR.

In another embodiment the cell population of step (i) is contacted with a first antigen complex (1^{st} AC) comprising the PAI, preferably a TP, and a detectable label A; and with at least a second antigen complex (2^{nd} AC) comprising a similar protein antigen (SPA), preferably a TSP, wherein the amino acid sequence of the SPA, preferably the TSP, differs by at least 1 amino acid from the amino acid sequence of the PAI, preferably the TP, and wherein the 2^{nd} AC comprises a detectable label B which is detectably different to label A and the 1^{st} AC comprises at least one further detectable label and the 2^{nd} AC comprises at least one further detectable label, which are different and the cell population of step (i) is contacted with one or more further antigen complexes (ACs) wherein each comprises a SPA that differs in at least one amino acid sequence from the amino acid sequence of the SPA of the 2^{nd} AC and, wherein the one or more further AC comprises at least one further detectable label; wherein the at least one further label is selected in such that it allows to distinguish the 1^{st} AC from the 2^{nd} AC and the one or more further ACs. It is preferred that the cell population is a T-cell population. It is further preferred that the selected immune cell that specifically and/or selectively binds to the 1^{st} AC is a T-cell. It is also preferred that the antigen-binding protein on the surface of the selected T-cell which is specifically and/or selectively binding to the PAI is a TCR.

In another embodiment the cell population of step (i) is contacted with a first antigen complex (1^{st} AC) comprising the PAI, preferably a TP, and a detectable label A; and with at least a second antigen complex (2^{nd} AC) comprising a similar protein antigen (SPA), preferably a TSP, wherein the amino acid sequence of the SPA, preferably the TSP, differs by at least 1 amino acid from the amino acid sequence of the PAI, preferably the TP, and wherein the 2^{nd} AC comprises a detectable label B which is detectably different to label A and the 1^{st} AC comprises at least one further detectable label and the 2^{nd} AC comprises at least one further detectable label, which are the same and the cell population of step (i) is contacted with one to ten, i.e. one, two, three, four, five, six, seven, eight, nine or ten, preferably two to four, more preferably two further antigen complexes (ACs) wherein each comprises a different SPA, preferably a different that differs in at least one amino acid sequence from the amino acid sequence of the SPA of the 2^{nd} AC and wherein the one or more further AC comprises at least one further detectable label; wherein the at least one further label is selected in such that it allows to distinguish the 1^{st} AC from the 2^{nd} AC and the one or more further ACs. It is preferred that the cell population is a T-cell population. It is further preferred that the selected immune cell that specifically and/or selectively binds to the 1^{st} AC is a T-cell. It is also preferred that the antigen-binding protein on the surface of the selected T-cell which is specifically and/or selectively binding to the PAI is a TCR.

In each of the above embodiments it is preferred that each of the SPAs, in particular each of the TSPs has a similarity to the amino acid sequence of the PAI, in particular to the amino acid sequence of the TP of at least 50%, at least 60%, at least 70%, at least 80%, at least 90%.

In another embodiment the detectable labels are provided. The skilled person in the art is well aware of how to label a protein antigen of interest, target peptide, similar protein antigen or target similar peptide of interest. Detectable labels as specified above with A-F, are independently selected from the group consisting of magnetic labels, fluorescent label, RNA-barcodes; DNA barcodes; or radioactive labels. Preferably, magnetic labels may comprise magnetic beads or magnetic nanoparticles which can be coated with e.g. antibodies against a particular surface antigen. Magnetic labels may be used in magnetic-activated cell sorting (MACS). Preferably, the detectable label is a fluorescent label selected from the group consisting of xanthens, acridines, oxazines, cyanines, styryl dyes, coumarines, porphines, metal-ligand-complexes, fluorescent proteins, nanocrystals, perylenes and phtalocyanines. Also preferred is the use of phycoerythrin (SA-PE), streptavidin-allophycocyanin (SA-APC) or streptavidin-brilliant-violet 421 (SA-BV421) as fluorescent labels for the detectable labels A-F. In another preferred embodiment the 1^{st} AC is a complex of a MHC-I or MHC-II and the PAI, and the PAI is a target peptide (TP). It is preferred that the TP is TAA. Additionally or alternatively, the 2^{nd} AC is a complex of a MHC-I or MHC-II and the SPA, and the SPA is a target similar peptide (TSP). In a further preferred embodiment the 1^{st} AC and the 2^{nd} AC is a soluble multimerized MHC-peptide complex.

### Functional differences and similarities of PAI and SPA:

As noted above the PAI is preferably expressed on diseased tissues, contrary to the SPA which is preferably expressed on healthy human tissues and thus, is selected based on the expression on healthy tissues. The inventors developed an in-house high-throughput technology platform (XPRESIDENT) including a large immunopeptidome database (comprising peptides which have been previously found to be presented on healthy tissues. SPAs are preferably from MHC, preferably HLA typed source, i.e. the SPAs are capable of binding to the respective MHC, preferably HLA molecule. This is required in the case the immune cell is a T-cell and the SPA is presented to the T-cell bound to an HLA molecule in order to allow the T-cell to recognize HLA presented SPAs. It is thus, preferred to select a SPA that is known to be presented on the same HLA allotype as the PAI. Preferably, SPAs are used in the method of the invention that are expressed on cells of healthy tissue with more than 10 copies per cell, preferably more than 20 copies per cell, preferably more than 50 copies per cell and even more preferably more than 100 copies per cell. The counterselection of T-cells that are capable of binding to such relatively abundant SPAs and at the same time to the PAI is desired to avoid off-target/off-tumor toxicity.

### Number of TSP:

TCRs of T-cells recognize a subgroup of amino acids within a given TP, i.e. the epitope of the TCR. Thus, if too many different TSP are used, it is likely that there will be no TCR that predominantly or exclusively binds to the TP but not to the TSP. Accordingly, it is preferred that not more than 10 TSPs, not more than 9 TSPs are used, not more than TSPs are used, not more than seven TSPs are used, not more than six TSPs are used, not more than five TSPs are used, not more than four TSPs are used, not more than three TSPs are used, not more than 2 TSPs are used or not more than 1 TSP is used. Accordingly, it is preferred that in the method of the present invention between 1-10 different TSPs, between 2-8 different TSPs, between 3-5 different TSPs or between one to three different TSPs are used. In a preferred embodiment three TSPs are used. The TSPs are fragments of SPAs and are selected on the basis of the same criteria as outlined for the SPA above. Similarly, TSPs are selected that are strongly expressed in healthy tissue. Accordingly, the TSPs to be included in the method of the invention are those, with high sequence similarity as defined above, i.e. it is preferred that each of the SPAs, in particular each of the TSPs has a similarity to the amino acid sequence of the PAI, in particular to the amino acid sequence of the TP of at least 50%, at least 60%, at least 70%, at least 80%, or at least 90%, and that show the highest expression on healthy tissue.

### Length of TP and TSP:

In one embodiment, the TP comprises 8-11 amino acids in length. The TP may also comprise 12 amino acids. In one embodiment, the TP comprises 13-25 amino acids in length. In another embodiment, the TP comprises 13-18 amino acids in length. Typically, the TSPs are chosen to have the same length as the given TP. However, alternatively, the length of the TSP may be longer or shorter by one to three amino acids as the TP. In the embodiments in which the TP is MHC presented, the length of the one or more TSPs are chosen in such that they can also be MHC presented. For example, if the TP comprises 8 amino acids in length, it is preferred that the TSP either has a length of 7 or less amino acids or a length of 8 or more amino acids. More preferably, a mixture of TSPs comprising sequences of different amino acids in length are used. In an embodiment wherein the TP is bound to MHC-I, the TP comprises 8-12 amino acids in length. In another embodiment, wherein the TP is bound to MHC-I, the TP comprises 8-11 amino acids in length. In an embodiment wherein the TP is bound to MHC-I, the TP comprises 8-10 amino acids in length. In an embodiment wherein the TP is bound to MHC-II, the TP comprises 13-23 amino acids in length. In a preferred embodiment wherein the TP is bound to MHC-II, the TP comprises 13-18 amino acids in length.

### Structural difference/similarity of TP and TSP:

In another embodiment the TSP is selected from the XPRESIDENT database of healthy tissue-presented HLA bound peptides based on high sequence similarity (similarity BLAST search) to the TP. The XPRESIDENT database comprises peptides presented by different HLA allotypes on healthy or diseased tissues. It is preferred that the TSP and the TP are presented by the same HLA allotype. HLA allotypes presenting TSP and TPs can be selected from the group consisting of HLA-A, HLA-B, HLA-C, HLA-E, HLA-F, HLA-G, HLA-H, HLA-J, HLA-K, HLA-L. Preferably the HLA-A protein is selected from the group consisting of HLA-A1, HLA-A2, HLA-A3, and HLA-A11. Preferred HLA-A alleles are HLA-A*02:01; HLA-A*01:01, HLA-A*03:01 or HLA-A*24:02. Preferred HLA-B alleles are HLA-B*07:02; HLA-B*08:01, HLA-B*15:01, HLA-B*35:01 or HLA-B*44:05.

Generally, for most of the HLA allotypes listed above, the second amino acid (when counting from the N-terminus) and the C-terminal amino acid of a given MHC presented peptide are not comprised in the epitope of that peptide recognized by a TCR that specifically binds to that peptide.

In another embodiment the amino acid sequence of the TSP has a length of 8 to 16 amino acids and the TP has a length of 8 amino acids and wherein the amino acid sequence of the TSP differs from the amino acid sequence of the TP as follows:
X₁-X₂-X₃-X₄-X₅-X₆-X₇-X₈ (SEQ ID NO: 1)
(i) at position X₁, X₂ and X₃, and wherein position X₄ to X₈ are identical or similar, preferably identical to the TP; or
(ii) at position X₄, X₅ and X₆, and wherein positions X₁ to X₃ and X₇ and X₈ are identical or similar, preferably identical to the TP; or
(iii) at position X₇ and X₈, and wherein position X₁ to X₆ are identical or similar, preferably identical to the TP.

In a preferred embodiment the positions X₁, X₂ and X₃ are mutated in the TSP wherein the position X₄ to X₈ are identical compared to the TP. In another preferred embodiment positions X₄, X₅ and X₆ are mutated in the TSP and positions X₁ to X₃ and X₇ and X₈ are identical to the TP. In another preferred embodiment position X₇ and X₈ in the TSP are mutated and position X₁ to X₆ are identical to the TP. In another preferred embodiment position X₇ and X₈ in the TSP are mutated and position X₁ to X₆ are identical to the TP.

In another preferred embodiment a mixture of the TSP described above in (i) to (iii), i.e. TSP with different mutation patterns are used in the method of the first aspect of the invention. In another preferred embodiments a mixture of the TSP described above in (i) to (iii), i.e. TSP with different mutation patterns and also different amino acid sequences in length are used. The use of such a mixture of TSP allows the fast and efficient positive selection of immune cells binding to the TP and negative selection of immune cells binding to one or more TSPs.

In another preferred embodiment the amino acid sequence of the TSP has a length of 8 to 16 amino acids and the TP has a length of 9 amino acids and the amino acid sequence of the TSP differs from the amino acid sequence of the TP
X₁-X₂-X₃-X₄-X₅-X₆-X₇-X₈-X₉ (SEQ ID NO: 2)
(i) at position X₁, X₂ and X₃, and wherein position X₄ to X₉ are identical or similar, preferably identical to the TP;
(ii) at position X₄, X₅ and X₆, and wherein position X₁ to X₃ and positions X₇ to X₉ are identical or similar, preferably identical to the TP; or
(iii) at position X₄, X₅, X₆ and X₇, and wherein position X₁ to X₃ and positions X₈ to X₉ are identical to the TP; or
(iv) at position X₇, X₈ and X₉, and wherein position X₁ to X₆ are identical or similar, preferably identical to the TP.

In a preferred embodiment the positions X₁, X₂ and X₃ are mutated in the TSP wherein the position X₄ to X₉ are identical compared to the TP. In another preferred embodiment positions X₄, X₅ and X₆ and X₇ are mutated in the TSP and positions X₁ to X₃ and positions X₈ and X₉ are identical to the TP. In another preferred embodiment positions X₇ to X₉ are mutated in the TSP and positions X₁ to X₆ are identical to the TP. In another preferred embodiment position X₇ to X₉ in the TSP are mutated and position X₁ to X₆ are identical to the TP. In another preferred embodiment a mixture of the TSP described above in (i) to (iv), i.e. TSP with different mutation patterns are used in the method of the first aspect of the invention. In another preferred embodiments a mixture of the TSP described above in (i) to (iv), i.e. TSP with different mutation patterns and also different amino acid sequences in length are used.

In another preferred embodiment the amino acid sequence of the TSP has a length of 8 to 16 amino acids, the TP has a length of 10 amino acids and the amino acid sequence of the TSP differs from the amino acid sequence of the TP
X₁-X₂-X₃-X₄-X₅-X₆-X₇-X₈-X₉-X₁₀ (SEQ ID NO: 3)
(i) at position X₁, X₂ and X₃, wherein position X₄ to X₁₀ are identical or similar, preferably identical to the TP;
(ii) at position X₄, X₅, X₆ and X₇, wherein position X₁ to X₃ and positions X₈ to X₁₀ are identical or similar, preferably identical to the TP; or
(iii) at position X₄, X₅ and X₆, and wherein position X₁ to X₃ and positions X₇ to X₁₀ are identical or similar, preferably identical to the TP; or
(iv) at position X₈, X₉ and X₁₀, wherein position X₁ to X₇ are identical or similar, preferably identical to the TP.

In a preferred embodiment the positions X₁, X₂ and X₃ are mutated in the TSP wherein the position X₄ to X₁₀ are identical compared to the TP. In another preferred embodiment positions X₄ to X₇ are mutated in the TSP and positions X₁ to X₃ and positions X₈ and X₁₀ are identical to the TP. In another preferred embodiment position X₄ to X₆ are mutated in the TSP and position X₁ to X₃ and positions X₇ to X₁₀ are identical to the TP. In another preferred embodiment positions X₈ to X₁₀ are mutated in the TSP and positions X₁ to X₇ are identical to the TP. In another preferred embodiment a mixture of the TSP described above in (i) to (iv), i.e. TSP with different mutation patterns are used in the method of the first aspect of the invention. In another preferred embodiments a mixture of the TSP described above in (i) to (iv), i.e. TSP with different mutation patterns and also different amino acid sequences in length are used.

In another preferred embodiment the amino acid sequence of the TSP has a length of 8 to 16 amino acids, the TP has a length of 11 amino acids and the amino acid sequence of the TSP differs from the amino acid sequence of the TP
X₁-X₂-X₃-X₄-X₅-X₆-X₇-X₈-X₉-X₁₀X₁₁ (SEQ ID NO: 4)
(i) at position X₁, X₂ and X₃, wherein position X₄ to X₁₁ are identical or similar, preferably identical to the TP;
(ii) at position X₄, X₅, X₆ and X₇, wherein position X₁ to X₃ and positions X₈ to X₁₁ are identical or similar, preferably identical to the TP; or
(iii) at position X₄, X₅ and X₆, and wherein position X₁ to X₃ and positions X₇ to X₁₁ are identical or similar, preferably identical to the TP; or
(iv) at position X₈, X₉, X₁₀ and X₁₁, wherein position X₁ to X₇ are identical or similar, preferably identical to the TP; or
(v) at position X₉, X₁₀ and X₁₁, wherein position X₁ to X₈ are identical or similar, preferably identical to the TP.

In a preferred embodiment the positions X₁, X₂ and X₃ are mutated in the TSP wherein the position X₄ to X₁₁ are identical compared to the TP. In another preferred embodiment positions X₄ to X₇ are mutated in the TSP and positions X₁ to X₃ and positions X₈ to X₁₁ are identical to the TP. In another preferred embodiment position X₄ to X₆ are mutated in the TSP and position X₁ to X₃ and positions X₇ to X₁₁ are identical to the TP. In another preferred embodiment positions X₈ to X₁₁ are mutated in the TSP and positions X₁ to X₇ are identical to the TP. In another preferred embodiment positions X₉ to X₁₁ are mutated in the TSP and positions X₁ to X₈ are identical to the TP.

In another preferred embodiment a mixture of the TSP described above in (i) to (iv), i.e. TSP with different mutation patterns are used in the method of the first aspect of the invention. In another preferred embodiments a mixture of the TSP described above in (i) to (iv), i.e. TSP with different mutation patterns and also different amino acid sequences in length are used.

In another preferred embodiment the amino acid sequence of the TSP has a length of 8-16 amino acids, the TP has a length of 12 amino acids and the amino acid sequence of the TSP differs from the amino acid sequence of the TP
X₁-X₂-X₃-X₄-X₅-X₆-X₇-X₈-X₉-X₁₀X₁₁ X₁₂ (SEQ ID NO: 5)
(i) at position X₁, X₂ and X₃, wherein position X₄ to X₁₂ are identical or similar to the TP;
(ii) at position X₄, X₅, X₆ and X₇, wherein position X₁ to X₃ and positions X₈ to X₁₂ are identical or similar to the TP; or
(iii) at position X₄, X₅ and X₆, and wherein position X₁ to X₃ and positions X₇ to X₁₂ are identical or similar to the TP; or
(iv) at position X₈, X₉, X₁₀, X₁₁ and X₁₂, wherein position X₁ to X₇ are identical or similar to the TP; or
(v) at position X₉, X₁₀, X₁₁ and X₁₂, wherein position X₁ to X₈ are identical or similar to the TP.

In a preferred embodiment the positions X₁, X₂ and X₃ are mutated in the TSP wherein the position X₄ to X₁₂ are identical compared to the TP. In another preferred embodiment positions X₄ to X₇ are mutated in the TSP and positions X₁ to X₃ and positions X₈ to X₁₂ are identical to the TP. In another preferred embodiment position X₄ to X₆ are mutated in the TSP and position X₁ to X₃ and positions X₇ to X₁₂ are identical to the TP. In another preferred embodiment positions X₈ to X₁₂ are mutated in the TSP and positions X₁ to X₇ are identical to the TP. In another preferred embodiment positions X₉ to X₁₂ are mutated in the TSP and positions X₁ to X₈ are identical to the TP.

In another preferred embodiment a mixture of the TSP described above in (i) to (iv), i.e. TSP with different mutation patterns are used in the method of the first aspect of the invention. In another preferred embodiments a mixture of the TSP described above in (i) to (iv), i.e. TSP with different mutation patterns and also different amino acid sequences in length are used.

In another embodiment of the first aspect of the invention the amino acid sequence of the SPA or of at least one protein or peptide comprised in the SPA has a similarity to the amino acid sequence of the PAI of at least 50%, at least 60%, at least 70%, at least 80%, or of at least 90% or of at least 95%. In another embodiment the amino acid sequence of the SPA or of at least one protein or peptide comprised in the SPA has less than or 90%, less than or 89%, less than or 88%, less than or 87% or less than or 86% amino acid identity to the PAI. In another embodiment the amino acid sequence of the SPA or of at least one protein or peptide comprised in the SPA has less than or 85%, less than or 84%, less than or 83%, less than or 82%, less than or 81% or less than or 80% amino acid identity to the PAI.

In another embodiment of the first aspect of the invention the amino acid sequence of the TSP has less than or 96%, less than or 95%, less than or 94%, less than or 93%, less than or 92% or less than or 91% amino acid identity to the TP. In another embodiment the amino acid sequence of the TSP has less than 90%, less than or 89%, less than or 88%, less than or 87% or less than or 86% amino acid identity to the TP. In another embodiment the amino acid sequence of the TSP has less than or 85%, less than or 84%, less than or 83%, less than or 82%, less than or 81% or less than or 80% amino acid identity to the TP.

In another embodiment the absolute expression of a TSP on healthy tissue is correlated with the lowest sequence identity of TSPs included in the method of the invention. If a TSP is highly expressed on healthy tissue, TCRs which only bind with low affinity to the TSP may nevertheless bind to the TSP expressed in healthy tissue due to avidity effects. Thus, if a given TSP has a low copy number on healthy tissue, it is included in the method of the present invention only, if it shows a high similarity to the TP. Correspondingly, if a given TSP has a high copy number on healthy tissue, it is included in the method of the present invention although it may have a low similarity to the TP. For example, if TSPs have a copy number below 10 per healthy cell than it is included, if it has at least a 90% sequence similarity to the TP. If the copy number of TSPs are between 1 to 25 such TSPs are included, if they have at least 85% sequence similarity with the TP. If the copy number of TSPs are between 25 to 100/cell such TSPs are included, if they have at least 80% sequence similarity with the TP. If the copy number of TSPs are between 100 to 250/cell such TSPs are included, if they have at least 75% sequence similarity with the TP. If the copy number of TSPs are above 250/cell such TSPs are included, if they have at least 50% sequence similarity with the TP.

In another embodiment of the method of the first aspect of the present invention the steps (ii) and (iii) of the method are carried out consecutively or concomitantly. In another embodiment of the first aspect of the invention steps (a), (b), (c) and (d) as outline above are carried out consecutively or concomitantly. Whether steps (a), (b), (c) and (d) are combined depends on the use of the number of ACs labeled with a detectable label.

In another embodiment of the method of the first aspect of the present invention step (iv) comprises positively selecting (selecting) cells bound to the 1^{st} AC, 1^{st} and 3^{rd} or 1^{st}, 3^{rd} and 4^{th} AC. In another embodiment step (iv) comprises negatively selecting (excluding) cells bound to the 2^{nd} AC, the 2^{nd} and 5^{th} or the 2^{nd}, 5^{th} and 6^{th} AC. In another preferred embodiment the step (iv) comprises selecting cells bound to the 1^{st} AC, 1^{st} and 3^{rd} or 1^{st}, 3^{rd} and 4^{th} AC and excluding cells bound to the 2^{nd} AC, the 2^{nd} and 5^{th} or the 2^{nd}, 5^{th} and 6^{th} AC.

In another embodiment of the method of the first aspect of the present invention the detectable label is detected by flow cytometry analysis. In a preferred embodiment the detectable label is detected by FACS analysis. In another preferred embodiment the detectable label is detected by preparative sorting analysis.

In another embodiment of the method of the first aspect of the present invention the cells comprised in the population of step i) of the method of the first aspect of the invention are T-cells and are phenotyped. In another embodiment the cells comprised in the population of step i) are B-cells and are phenotyped.

In another embodiment of the method of the first aspect of the present invention the phenotyping of T-cells comprises the determination of one or more T-cell marker. T-cell marker are preferably selected from the group consisting of CD3, CD4, CD8, CD11a, CD14, CD19, CD25, CD27, CD28, CD44, CD45RA, CD45RO, CD57, CD62L, CD69, CD122, CD127, CD137 CD197 (CCR7), IFNy, IL-2, TNFα, IL7R and telomer length. In another preferred embodiment T-cell markers are CD45RA, CD45RO, CD197, CD25, CD27, CD57, CD95, CD127 and CD62L It is particularly preferred that CD69 and CD137 are used for the phenotyping of T-cells. In another preferred embodiment the phenotyping of B-cells comprises the determination of one or more B-cell marker. B-cell marker are preferably selected from the group consisting of CD19, CD27, CD45R, CD21, CD40, CD20, CD38, and CD83.

In another embodiment of the method of the first aspect of the present invention the method further comprises the step of contacting the cell population of step (i) with an irrelevant antigen complex (IAC) comprising an irrelevant protein antigen (IPA), wherein the amino acid sequence of the IPA when aligned with the amino acid sequence of the PAI is identical to the PAI at two amino acid positions or less and wherein the IAC comprises a detectable label G that is detectably different from the detectable label A. Preferably, an irrelevant protein antigen is the gene product of a housekeeping gene. The housekeeping gene product is expressed in all cells of an organism under normal and pathophysiological conditions which make it suitable to function as a reference gene because it is usually not up or down regulated under different or varying cell conditions. Generally, a natural immune cell population should not comprise any immune cells binding to housekeeping genes or peptides derived therefrom. Thus, the inclusion of an IPC in the method of the invention allows the identification of T-cells that nonspecifically bind to AC, which are also undesirable.

In another embodiment the amino acid sequence of at least one IP is selected by one or more of the following criteria: presentation of the IP on healthy tissue; the IP is derived from a HLA typed source; or the binding to the respective HLA. It is preferred that the amino acid sequence of the IP or of at least one protein or peptide comprised in the IPA has less than 50%, less than 40%, less than 30%, less than 29%, less than 28%, less than 27%, less than 26%, less than 25%, less than 24%, less than 23%, less than 22%, less than 21%, less than 20%, less than 19%, less than 18%, less than 17%, less than 16%, less than 15%, less than 10%, less than 5% amino acid identity to the PAI. In another embodiment the IAC is a complex of a MHC-I or MHC-II and an IP. It is preferred that the amino acid sequence of the IP when aligned with the amino acid sequence of the TP is identical to the TP at one or none amino acid positions. Preferably, the IP is encoded by a housekeeping gene.

A second aspect of the invention further relates to a method for determining the sequence of a nucleic acid encoding an antigen-binding protein or an antigen-binding part thereof comprising the steps of:
(i) isolating the nucleic acid encoding the antigen-binding protein or the antigen-binding part thereof from the cell selected in the method of the first aspect of the invention; and
(ii) determining the sequence of the nucleic acid.
In a preferred embodiment the nucleic acid is isolated from the selected immune cell by methods well known in the art, e.g. organic extraction, solid phase extraction, e.g. using a resin comprising a styrene-divinylbenzene co-polymer containing iminodiacetic acid groups. In another embodiment the isolated nucleic acid is either DNA or RNA. In another embodiment it is preferred to amplify the nucleic acid after isolation. Preferably, amplification is conducted by polymerase chain reaction (PCR). More preferably the nucleic acid is amplified in a rapid amplification of cDNA-ends with PCR (RACE PCR). In another embodiment the synthesis of DNA from an RNA template, via reverse transcription, produces complementary DNA (cDNA). Reverse transcriptases (RTs) use an RNA template and a short primer complementary to the 3' end of the RNA to direct the synthesis of the first strand cDNA, which can be used directly as a template for the PCR. In another embodiment the sequence of the isolated nucleic acid can be determined by known methods in the art, for example next generation sequencing, e.g. Illumina (Solexa) sequencing by simultaneously identifying DNA bases, as each base emits a unique fluorescent signal, and adding them to a nucleic acid chain, Roche 454 sequencing based on pyrosequencing, a technique which detects pyrophosphate release, again using fluorescence, after nucleotides are incorporated by polymerase to a new strand of DNA, or ion torrent: Proton / PGM sequencing measuring the direct release of protons from the incorporation of individual bases by DNA polymerases.

A third aspect of the invention relates to a method for producing a eukaryotic cell expressing a nucleic acid encoding an antigen-binding protein or an antigen-binding part thereof comprising the steps of:
(i) providing the nucleic acid sequence encoding the antigen-binding protein or an antigen-binding part thereof from the cell selected in the method of the first aspect of the invention;
(ii) producing a nucleic acid vector comprising the nucleic acid sequence provided in step (i) optionally under the control of an expression control element; and
(iii) introducing the nucleic acid vector of step (ii) into a host cell.
In one embodiment the antigen-binding protein or an antigen binding part thereof is cloned into a vector.

In one embodiment the antigen-binding protein is a TCR or an antigen binding fragment thereof; a BCR or an antigen binding fragment thereof or an antibody or an antigen binding fragment thereof. In another embodiment, the antigen binding protein is a TCR or the part thereof comprise at least the variable domains of the alpha and beta chain. Preferably, the sequence of the TCR or antigen binding part thereof is inserted into a suitable vector. In another embodiment the amino acid sequence of the TCR, BCR or antibody comprises six CDRs. In another embodiment two or three CDRs of the variable alpha and/or beta domain of an identified TCR are inserted into the framework or another TCR or antibody. Preferably, the gene sequence of one, two or three CDRs of the variable alpha domain of a TCR are cloned into a suitable vector comprising framework regions. The expression vector may either comprise nucleic acids encoding both the light or heavy chain or alpha and beta chain (or the variable domains thereof) - soc-called "tandem type" - or they may be encoded by nucleic acids comprised in separate vectors. It is preferred that humanized antibody expression vectors of the tandem type are used (Shitara K et al. J Immunol Methods. 1994 Jan. 3; 167(1-2):271-8). Examples of tandem type humanized antibody expression vector include e.g. pKANTEX93 (WO 97/10354), and pEE18.

In another embodiment the vector of step (ii) is introduced into a host cell. In one embodiment such recombinant host cells can be used for the production of at least one antigen binding protein of the invention or part thereof. Preferably, the host cell is transformed, transduced or transfected with a nucleic acid and/or a vector encoding the antigen binding protein or antigen binding part thereof. Transduction or transfection of host cells with nucleic acid encoding the antigen binding protein or part of the antigen binding protein is conducted using methods well known in the art, for example methods described in US20190216852. In another embodiment the host cells comprising the antigen binding protein or antigen binding part thereof can be a eukaryotic cell, e.g., plant, animal, fungi, or algae, or can be a prokaryotic cell, e.g., bacteria or protozoa. The host cell can be a cultured cell or a primary cell, i.e., isolated directly from an organism, e.g., a human. The host cell can be an adherent T-cell or a suspended cell, i.e., a cell that grows in suspension. For purposes of producing an antigen binding protein or part of the antigen binding protein, such as a recombinant TCR or fragment thereof, the host cell is preferably a mammalian cell. Most preferably, the host cell is a human cell. While the host cell can be of any cell type, can originate from any type of tissue, and can be of any developmental stage, the host cell preferably is a peripheral blood leukocyte (PBL) or a peripheral blood mononuclear cell (PBMC) or a B-cell. More preferably, the host cell is a T-cell. The T-cell can be any T-cell, such as a cultured T-cell, e.g., a primary T-cell, or a T-cell from a cultured T-cell line, e.g., Jurkat, SupT1, etc., or a T-cell obtained from a mammal, preferably a T-cell or T-cell precursor from a human patient. If obtained from a mammal, the T-cell can be obtained from numerous sources, including but not limited to blood, bone marrow, lymph node, the thymus, or other tissues or fluids. Preferably, the T-cell is a human T-cell. More preferably, the T-cell is a T-cell isolated from a human. The T-cell can be any type of T-cell and can be of any developmental stage, including but not limited to, CD4-positive and/or CD8-positive, CD4-positive helper T-cells, e.g., Th1 and Th2 cells, CD8-positive T-cells (e.g., cytotoxic T-cells), tumor infiltrating cells (TILs), memory T-cells, naive T-cells. Preferably, the T-cell is a CD8-positive T-cell or a CD4-positive T-cell. In another embodiment the host cell may be any cell for recombinant expression. Preferably, the host cell is a Chinese hamster ovary (CHO) cell.

### Examples

**Example 1: Direct sorting of target-peptide specific T cells with and without prior target-specific expansion.** Fig. 2 and 3 show a comparison of two different approaches which lead to sorting of target-specific T cells while sparing cross-reactive T cells which recognize target and similar peptides. Method 1 (Fig. 2) does not require prior amplification of target-specific T cells. PBMCs are isolated and enriched for T cell populations by magnetic bead separation. The T cell population is stained with fluorochrome-conjugated target-peptide and similar-peptide tetramers. Subsequently, those cells can be further enriched for target-specific T cells by using magnetic bead separation targeting one of the fluorochrome-conjugates of the target-tetramers. The target peptide-specific T cell population is then stained for surface markers such as CD4 and CD8 as well as viability markers to exclude dead cells. By using flow cytometric sorting approaches the target-specific T cells can be sorted for desired surface marker expression while sparing target+similar peptide-specific T cells as shown in fig. 2. Method 2 (Fig. 3) utilizes stimulation with target-peptide HLA-coated artificial antigen-presenting cells to amplify low frequency target-specific T cells. Here, enriched CD8 T cells are cultivated in individual vessels to allow for the growth of mono- or oligoclonal target-peptide specific T cell populations. After repeated stimulation with artificial antigen-presenting cells the individual mono- or oligoclonal populations are stained with surface markers as well as target- and similar-peptide tetramers (target and similar peptide tetramers are labelled with 2 distinct fluorochromes each in a 2D staining approach) which allows for distinction of target-specific and cross-reactive mono- or oligoclonal T cell populations as shown in Fig 3.

**Example 2: Functional assessment of T cell receptors derived from T cells sorted with target-peptide multimers only.** To assess functionality and specificity of TCRs identified by sorting with target multimers, T cell receptor mRNA is generated by *in vitro* transcription and subsequently used to transfect CD8 positive T cells of healthy donors by electroporation. Eighteen hours after electroporation 20,000 transfected T cells are then co-incubated with T2 cells loaded either with target peptide, different target-sequence similar peptides, an irrelevant peptide or unloaded T2 cells at a 1:1 ratio. Supernatants are harvested 24h after start of co-culture and analyzed for secreted IFN-y by ELISA-technique. Cytokine secretion demonstrates antigen recognition and activity of the respective T cells as illustrated in Figure 4. Whereas all TCRs in Fig. 4A, B and C recognize the target (positive control), TCRs in Fig. 4A and Fig.4B are also cross-reactive towards target-sequence similar peptides expressed on normal tissue and are thus excluded from further analysis, only "clean" TCRs (Fig. 4C) are worth to be selected for further characterization. ("Target" = target peptide; TP; "SIM 1-10" = target similar peptides; TSPs 1-10).

### Peptides used in this example:

TP and SIM 1- SIM 10 are all 9mers.
- TP and **SIM 1** differ in amino acid position 2, 5, 8 and 9 wherein SIM 1 has an isoleucine residue at position 2, a threonine residue at position 5, a leucine residue at position 8 and a valine residue at position 9.
- TP and **SIM 2** differ in position 3, 4 and 7, wherein SIM 2 has an isoleucine residue at position 3, a glutamic acid residue at position 4 and a glutamine residue at position 7.
- TP and **SIM 3** differ in position 2, 7, 8 and 9, wherein SIM 3 has an isoleucine residue at position 3, a glutamic acid residue at position 7 and 8 and an isoleucine residue at position 9.
- TP and **SIM 4** differ in position 4, 5 and 8, wherein SIM 4 has a lysine residue at position 4, an asparagine residue at position 5 and a tyrosine residue at position 8.
- TP and **SIM 5** differ in position 4, 7 and 8, wherein SIM 5 has an asparagine residue at position 4, a proline residue at position 7 and a tyrosine residue at position 8.
- TP and **SIM 6** differ in position 6, 7 and 8, wherein SIM 6 has valine residue at position 6 and a leucine residue at position 7 and 8.
- TP and **SIM 7** differ in position 5, 6 and 8, wherein SIM 7 has lysine residue at position 5, a glutamine residue at position 6 and a methionine residue at position 8.
- TP and **SIM 8** differ in position 3, 5, 7 and 9, wherein SIM 8 has serine residue at position 3, a glutamic acid residue at position 5 and a valine residue at position 7 and 9.
- TP and **SIM 9** differ in position 2, 4, 5 and 9, wherein SIM 9 has valine residue at position 2, a glycine residue at position 4, an alanine residue at position 5 and a valine residue at position 9.
- TP and **SIM 10** differ in position 1, 4 and 6, wherein SIM 10 has valine residue at position 1, a histidine residue at position 4 and a glutamine residue at position 9.
- TP and **control peptide** differ in positions 4-9.

**Example 3: Functional assessment of T cell receptors derived from target-peptide as well as target- and similar-peptide specific T cells.** To this end, T cell receptor mRNA is generated using in vitro transcription and subsequently used to transfect NFAT-luciferase Jurkat cells by electroporation. The transfected Jurkat cells start to express the newly introduced TCRs transiently on their surface. Eighteen hours after electroporation 50,000 Jurkat cells are then co-incubated with T2 cells at a 1:1 ratio loaded either with a target peptide or the similar peptides which are used for sorting, as well as a control peptide or no peptide. Upon specific binding of the TCR to its cognate peptide-HLA, signaling leads to NFAT activation which in turn leads to expression of luciferase. After overnight incubation luciferase substrate is added and a luminescence signal can be detected when the T cell is activated. Figure 5 shows that TCRs derived from target-tetramer binding T cells lead to functional activation when stimulated with target-peptide loaded T2 cells. ("Target" = target peptide; TP; "SIM 1-3" = target similar peptides; TSPs 1-3).

### Peptides used in this example:

TP and SIM 1- SIM 3 are all 9mers.
- TP and SIM 1 differ in amino acid position 4, 6 and 7 wherein SIM 1 has a glutamic acid residue at position 4, a leucine residue at position 6 and an isoleucine residue at position 7.
- TP and SIM 2 differ in position 2, 7 and 8, wherein SIM 2 has a methionine residue at position 2, a glutamic acid residue at position 7 and lysine residue at position 8.
- TP and SIM 3 differ in position 1, 5 and 6, wherein SIM 3 has a phenylalanine residue at position 1, a glycine residue at position 5 and a serine residue at position 6.
- TP and **control peptide** differ in positions 1 and 4-9.

Overall SIM 1 has a similarity to TP of 77%, SIM 2 has a similarity to TP of 77% and SIM 3 has a similarity to TP of 75% using BLASTP, BLOSUM62 scoring matrix, a word length of 3, and expectation (E) of 10.

### Example 4: Relevant and irrelevant target similar peptides for a given target peptide.

The relevance of a peptide as TSP to a given TP is determined mainly by its similarity to the TP, and can additionally be guided by its frequency of presentation as well as and quantitative presentation level (copy numbers per cell (CpC)) on primary normal tissues. The higher the similarity to the TP and the higher the presentation frequency and CpC on normal tissues, the higher the relevance of a TSP. Table 6 shows example sequences of a TP, two corresponding TSPs as well as an IP. Per peptide, the number of identical amino acids (aa) to the TP, the similarity based on the pmbec positional scoring matrix in comparison to the TP sequence and the CpC range on normal tissues is depicted. Figs. 6, 7 and 8 additionally shows the peptide presentation profiles of the two TSPs as well as the IP. TSP (TSP1) has 4 identical amino acids to the TP but a higher overall similarity to the target as compared to TSP2 which has 5 identical amino acids in comparison to the target peptide. Both TSPs are considered relevant based on their similarity to the TP and their presentation on normal tissues (Figs. 6 and 7). The depicted IP shows an even higher presentation frequency on normal tissues (Fig.8) and is in general also presented at a higher copy number per cell. The sequence similarity as well as the number of identical amino acids is however rather low (17% similarity and 0 identical amino acids).

**Table 6:**

| | Amino acid equence | Number of identical aa to TP | Similarity to TP (PMBEC Score) | CpC range normal tissue |
|---|---|---|---|---|
| TP | VLLHHQIGL (SEQ ID NO: 164 ) | 9 | 100% | n.a. |
| TSP1 | ALMYHTITL (SEQ ID NO: 165) | 4 | 63% | 5-60 |
| TSP2 | LLLAHIIAL (SEQ ID NO: 166) | 5 | 55 % | 15-35 |
| IP | AIVDKVPSV (SEQ ID NO: 167) | 0 | 17% | 55-600 |

## Claims

1. A method for selecting a eukaryotic cell expressing on its surface an antigen-binding protein specifically and/or selectively binding to a protein antigen of interest (PAI) comprising the following steps:
(i) providing a eukaryotic cell population;
(ii) contacting the eukaryotic cell population of step (i) with a first antigen complex (1^{st} AC) comprising the PAI and a detectable label A or with the PAI comprising a detectable label A;
(iii) contacting the eukaryotic cell population of step (i) with at least a second antigen complex (2^{nd} AC) comprising a similar protein antigen (SPA), wherein the amino acid sequence of the SPA differs by 1-20 amino acids from the amino acid sequence of the PAI, preferably by 2-10 amino acids from the amino acid sequence of the PAI, and wherein the 2^{nd}AC comprises a detectable label B; or with the SPA and a detectable label B; and
(iv) selecting at least one eukaryotic cell that specifically and/or selectively binds to the 1^{st} AC,
wherein the detectable label A and the detectable label B are detectably different from each other, and wherein the selected eukaryotic cell is a T-cell, a B-cell, or a mammalian cell expressing a heterologous antigen binding protein selected from the group consisting of a T-cell receptor (TCR) or antigen binding fragments thereof, a B-cell receptor (BCR) or antigen binding fragments thereof, and a chimeric antigen receptor (CAR) or antigen binding fragments thereof.

2. The method according to claim 1, wherein the
amino acid sequence of the SPA differs by 2-10 amino acids from the amino acid sequence of the PAI.

3. The method according to claim 1 or 2, wherein
(a) the eukaryotic cell population comprises:
(i) immune cells, preferably tumor-infiltrating lymphocytes (TILs), T cell receptor libraries, peripheral blood of healthy subjects, peripheral blood of diseased subjects or an immune cell enriched fraction thereof, more preferably wherein
(1) the immune cell enriched fraction is enriched in stem cells; T-cells, more preferably CD8 T-cells or CD4 T-cells; B-cells; plasma cell;
(2) the diseased subject suffers from a disease selected from the group consisting of immune diseases, neoplastic diseases, a disease caused by a virus or a disease caused by bacteria; or
(ii) mammalian cells expressing a library of heterologous antigen binding proteins; and/or
(b) the protein antigen of interest (PAI) is a tumor associated antigen (TAA), a viral protein or a bacterial protein.

4. The method according to claim 3, wherein
(i) the immune cell enriched fraction is selected by detectably labeling one or more immune cell specific surface marker; and/or
(ii) the method comprises the further step of incubating the eukaryotic cell population in the presence of growth and/or differentiation factors, preferably cytokines; and/or
(iii) the AC is an antigen-presenting cell, or a complex comprising a particle, the PAI and the detectable label A or the SPA and the detectable label B.

5. The method according to any of claims 1 to 4, comprising one or more of the following further steps:
(a) contacting the eukaryotic cell population of step (i) with a third antigen complex (3^{rd} AC) comprising the PAI and a detectable label C that is detectably different from one or more or all of the other detectable labels of the other ACs contacted with the eukaryotic cell population, preferably detectably different from at least the detectable label A, preferably from at least the detectable label A and a detectable label D, if a detectable label D is present; and/or
(b) contacting the eukaryotic cell population of step (i) with a fourth antigen complex (4^{th} AC) comprising the PAI and a detectable label D that is detectably different from one or more or all of the other detectable labels of the other ACs contacted with the eukaryotic cell population, preferably detectably different from at least the detectable label A and, preferably from at least the detectable label A and the detectable label C; and/or
(c) contacting the eukaryotic cell population of step (i) with a fifth antigen complex (5^{th} AC) comprising the SPA and a detectable label E that is detectably different from one or more or all of the other detectable labels of the other ACs contacted with the eukaryotic cell population, preferably detectably different from at least the detectable label B and, preferably from at least the detectable label B and a detectable label F, if a detectable label F is present; and/or
(d) contacting the eukaryotic cell population of step (i) with a sixth antigen complex (6^{th} AC) comprising the SPA and a detectable label F that is detectably different from one or more or all of the other detectable labels of the other ACs contacted with the eukaryotic cell population, preferably detectably different from at least the detectable label B and, preferably from at least the detectable label B and the detectable label E; and/or
(e) contacting the eukaryotic cell population of step (i) with one or more further antigen complexes (AC) wherein each comprises a SPA that differs in at least one amino acid sequence from the amino acid sequence of the SPA of the 2^{nd} AC, and wherein each further AC comprises one or more labels, wherein the one or more label is the same to or detectably different from the one or more labels of the 2^{nd} AC.

6. The method according to any one of claims 1 to 5, wherein
(i) the 1^{st} AC comprises at least one further detectable label and the 2^{nd} AC comprises at least one further detectable label, which are either the same or different; and/or
(ii) the one or more further AC comprises at least one further detectable label; wherein the at least one further label is selected in such that it allows to distinguish the 1^{st} AC from the 2^{nd} AC and the one or more further ACs; and/or
(iii) the detectable labels are independently selected from a fluorescent label, preferably selected from the group consisting of xanthens, acridines, oxazines, cyanines, styryl dyes, coumarines, porphines, metal-ligand-complexes, fluorescent proteins, nanocrystals, perylenes and phtalocyanines.

7. The method according to any of claims 1 to 5, wherein the 1^{st} AC is a complex of a MHC-I or MHC-II and the PAI, and wherein the PAI is a target peptide (TP), preferably a tumor-specific target peptide and/or the 2^{nd} AC is a complex of a MHC-I or MHC-II and the SPA, and wherein the SPA is a target similar peptide (TSP) and wherein the TSP differs by at least 1 amino acid from the amino acid sequence of the TP,
preferably wherein the amino acid sequence of the at least one TSP is selected by one or more of the following criteria:
(a) presentation of the TSP on healthy tissue;
(b) derived from HLA typed source; and
(c) binding to the respective HLA.

8. The method according to claim 7, wherein the amino acid sequence of the TSP has a length of 8 to 16 amino acids and wherein:
(1) the amino acid sequence of the TSP differs from the amino acid sequence of the TP
X₁-X₂-X₃-X₄-X₅-X₆-X₇-X₈
(i) at position X₁, X₂ and X₃, and wherein position X₄ to X₈ are identical or similar to the TP;
(ii) at position X₄, X₅ and X₆, and wherein positions X₁ to X₃ and X₇ and X₉ are identical or similar to the TP; or
(iii) at position X₇ and X₈, and wherein position X₁ to X₆ are identical or similar to the TP;
if the TP has a length of 8 amino acids; or
(2) the amino acid sequence of the TSP differs from the amino acid sequence of the TP
X₁-X₂-X₃-X₄-X₅-X₆-X₇-X₈-X₉
(i) at position X₁, X₂ and X₃, and wherein position X₄ to X₉ are identical or similar to the TP;
(ii) at position X₄, X₅ and X₆, and wherein position X₁ to X₃ and positions X₇ to X₉ are identical or similar to the TP; or
(iii) at position X₄, X₅, X₆ and X₇, and wherein position X₁ to X₃ and positions X₈ to X₉ are identical or similar to the TP; or
(iv) at position X₇ X₈ and X₉, and wherein position X₁ to X₆ are identical or similar to the TP;
if the TP has a length of 8-9 amino acids; or
(3) the amino acid sequence of the TSP differs from the amino acid sequence of the TP
X₁-X₂-X₃-X₄-X₅-X₆-X₇-X₈-X₉-X₁₀
(i) at position X₁, X₂ and X₃, wherein position X₄ to X₁₀ are identical or similar to the TP;
(ii) at position X₄, X₅, X₆ and X₇, wherein position X₁ to X₃ and positions X₈ to X₁₀ are identical or similar to the TP; or
(iii) at position X₄, X₅ and X₆, and wherein position X₁ to X₃ and positions X₇ to X₁₀ are identical or similar to the TP; or
(iv) at position X₈, X₉ and X₁₀, wherein position X₁ to X₇ are identical or similar to the TP;
if the TP has a length of 8-10 amino acids; or
(4) the amino acid sequence of the TSP differs from the amino acid sequence of the TP
X₁-X₂-X₃-X₄-X₅-X₆-X₇-X₈-X₉-X₁₀X₁₁
(i) at position X₁, X₂ and X₃, wherein position X₄ to X₁₁ are identical or similar to the TP;
(ii) at position X₄, X₅, X₆ and X₇, wherein position X₁ to X₃ and positions X₈ to X₁₁ are identical or similar to the TP; or
(iii) at position X₄, X₅ and X₆, and wherein position X₁ to X₃ and positions X₇ to X₁₁ are identical or similar to the TP; or
(iv) at position X₈, X₉, X₁₀ and X₁₁, wherein position X₁ to X₇ are identical or similar to the TP; or
(v) at position X₉, X₁₀ and X₁₁, wherein position X₁ to X₈ are identical or similar to the TP;
if the TP has a length of 8-11 amino acids; or
(5) the amino acid sequence of the TSP differs from the amino acid sequence of the TP
X₁-X₂-X₃-X₄-X₅-X₆-X₇-X₈-X₉-X₁₀X₁₁ X₁₂
(i) at position X₁, X₂ and X₃, wherein position X₄ to X₁₂ are identical or similar to the TP;
(ii) at position X₄, X₅, X₆ and X₇, wherein position X₁ to X₃ and positions X₈ to X₁₂ are identical or similar to the TP; or
(iii) at position X₄, X₅ and X₆, and wherein position X₁ to X₃ and positions X₇ to X₁₂ are identical or similar to the TP; or
(iv) at position X₈, X₉, X₁₀, X₁₁ and X₁₂, wherein position X₁ to X₇ are identical or similar to the TP; or
(v) at position X₉, X₁₀, X₁₁ and X₁₂, wherein position X₁ to X₈ are identical or similar to the TP;
if the TP has a length of 8-12 amino acids;
preferably wherein the amino acid sequence of the TSP has a different length than the TP.

9. The method according to any one of claims 1 to 8, wherein
(i) the amino acid sequence of the SPA or the TSP has less than 95% amino acid identity to the PAI or TP, less than 90% amino acid identity or less than 85% amino acid identity; and/or
(ii) the eukaryotic cell population of step (i) of claim 1 is contacted with not more than 10 antigen complexes (AC) each comprising a different similar protein antigen (SPA), not more than nine different SPAs, not more than eight different SPAs, not more than seven different SPAs, not more than six different SPAs, not more than five different SPAs, not more than four different SPAs, not more than three different SPAs, not more than two different SPAs, or not more than one SPA, is used, preferably wherein the SPA is a TSP, even more preferably wherein the number of different TSPs is between 1-10; between 2-8; between 3-5 or between 1-3, preferably three TSPs are used, preferably wherein the SPA is a TSP, more preferably wherein the number of different TSPs is between 1-10; between 2-8; between 3-5 or between 1-3, preferably three TSPs are used; and/or
(iii) steps (ii) and (iii) of claim 1 and/or steps (a), (b), (c) and/or (d) of claim 10 are carried out subsequently in any order or combination or concomitantly.

10. The method according to any of claims 1 to 9, wherein step (iv) of claim 1 and/or steps (a), (b), (c) or (d) of claim 5 comprises:
a) positively selecting (selecting) eukaryotic cells bound to the 1^{st} AC, 1^{st} and 3^{rd} or 1^{st}, 3^{rd} and 4^{th} AC; and/or
b) negatively selecting (excluding) eukaryotic cells bound to the 2^{nd} AC, the 2^{nd} and 5^{th} or the 2^{nd}, 5^{th} and 6^{th} AC.

11. The method according to any one of claims 1 to 9, wherein
(i) the label is detected by flow cytometry analysis, preferably MACS analysis, FACS analysis or preparative sorting analysis; and/or
(ii) the eukaryotic cells comprised in the population of step i) of claim 1 are T-cells and/or B-cells and are phenotyped, preferably wherein the phenotyping of T-cells comprises the determination of one or more T-cell marker; and/or
(iii) phenotyping of B-cells comprises the determination of one or more B-cell marker.

12. The method according to any one of claims 1 to 11, further comprising the step of:
contacting the eukaryotic cell population of step (i) of claim 1 with an irrelevant antigen complex (IAC) comprising an irrelevant protein antigen (IPA), wherein the amino acid sequence of the IPA when aligned with the amino acid sequence of the PAI is identical to the PAI at two amino acid positions or less and wherein the IAC comprises a detectable label G that is detectably different from the detectable label A, preferably wherein
(i) the amino acid sequence of at least one IPA is selected by one or more of the following criteria:
(a) presentation of the IPA on healthy tissue;
(b) derived from HLA typed source;
(c) binding to the respective HLA; and/or
(ii) the amino acid sequence of the IPA or of at least one protein or peptide comprised in the IPA has less than 30% amino acid identity to the PAI, less than 20% amino acid identity or less than 10% amino acid identity; and/or
(iii) the IAC is a complex of an MHC-I or MHC-II and an irrelevant peptide (IP), and wherein the amino acid sequence of the IP when aligned with the amino acid sequence of the TP is identical to the TP at one or none amino acid positions.

13. A method for determining the sequence of a nucleic acid encoding an antigen-binding protein or an antigen-binding part thereof comprising the steps of:
(i) isolating the nucleic acid encoding the antigen-binding protein or the antigen-binding part thereof from the eukaryotic cell selected according to any one of claims 1 to 11; and
(ii) determining the sequence of the nucleic acid,
preferably wherein the antigen-binding protein is a TCR or a fragment thereof; a BCR or a fragment thereof or an antibody or a fragment thereof.

14. A method for producing a eukaryotic cell expressing a nucleic acid encoding an antigen-binding protein or an antigen-binding part thereof comprising the steps of
(i) providing the nucleic acid sequence encoding the antigen-binding protein or an antigen-binding part thereof from the eukaryotic cell selected in the method according to any one of claims 1 to 11;
(ii) producing a nucleic acid vector comprising the nucleic acid sequence provided in step (i) optionally under the control of an expression control element; and
(iii) introducing the nucleic acid vector of step (ii) into a host cell,
preferably wherein the antigen-binding protein is a TCR or a fragment thereof; a BCR or a fragment thereof or an antibody or a fragment thereof, more preferably wherein the amino acid sequences of the TCR, the BCR or the antibody comprises six CDR sequences.

## Patentansprüche

1. Verfahren zum Auswählen einer eukaryotischen Zelle, die auf ihrer Oberfläche ein Antigenbindungsprotein exprimiert, das spezifisch und/oder selektiv an ein Proteinantigen von Interesse (PAI) bindet, umfassend die folgenden Schritte:
(i) Bereitstellen einer eukaryotischen Zellpopulation;
(ii) Inkontaktbringen der eukaryotischen Zellpopulation aus Schritt (i) mit einem ersten Antigenkomplex (1. AC), der das PAI und eine nachweisbare Markierung A umfasst, oder mit dem PAI, das eine nachweisbare Markierung A umfasst;
(iii) Inkontaktbringen der eukaryotischen Zellpopulation aus Schritt (i) mit mindestens einem zweiten Antigenkomplex (2. AC), der ein ähnliches Proteinantigen (SPA) umfasst, wobei sich die Aminosäuresequenz des SPA um 1-20 Aminosäuren von der Aminosäuresequenz des PAI, vorzugsweise um 2-10 Aminosäuren von der Aminosäuresequenz des PAI, unterscheidet und wobei der 2. AC eine nachweisbare Markierung B umfasst; oder mit dem SPA und einer nachweisbaren Markierung B; und
(iv) Auswählen von mindestens einer eukaryotischen Zelle, die spezifisch und/oder selektiv an den 1. AC bindet,
wobei sich die nachweisbare Markierung A und die nachweisbare Markierung B nachweisbar voneinander unterscheiden und wobei die ausgewählte eukaryotische Zelle eine T-Zelle, eine B-Zelle oder eine Säugetierzelle ist, die ein heterologes Antigenbindungsprotein exprimiert, das aus der Gruppe bestehend aus einem T-Zell-Rezeptor (TCR) oder antigenbindenden Fragmenten davon, einem B-Zell-Rezeptor (BCR) oder antigenbindenden Fragmenten davon und einem chimären Antigenrezeptor (CAR) oder antigenbindenden Fragmenten davon ausgewählt ist.

2. Verfahren nach Anspruch 1, wobei sich die Aminosäuresequenz des SPA um 2-10 Aminosäuren von der Aminosäuresequenz des PAI unterscheidet.

3. Verfahren nach Anspruch 1 oder 2, wobei
(a) die eukaryotische Zellpopulation Folgendes umfasst:
(i) Immunzellen, vorzugsweise tumorinfiltrierende Lymphozyten (TILs), T-Zellrezeptorbibliotheken, peripheres Blut gesunder Subjekte, peripheres Blut erkrankter Subjekte oder eine hinsichtlich Immunzellen angereicherte Fraktion davon, wobei stärker bevorzugt
(1) die hinsichtlich Immunzellen angereicherte Fraktion bezüglich Stammzellen; T-Zellen, stärker bevorzugt CD8-T-Zellen oder CD4-T-Zellen; B-Zellen; Plasmazellen angereichert ist;
(2) das erkrankte Subjekt an einer Krankheit leidet, die ausgewählt ist aus der Gruppe bestehend aus Immunkrankheiten, neoplastischen Krankheiten, einer durch ein Virus verursachten Krankheit oder einer durch Bakterien verursachten Krankheit; oder
(ii) Säugetierzellen, die eine Bibliothek heterologer Antigenbindungsproteine exprimieren;
und/oder
(b) das Proteinantigen von Interesse (PAI) ein tumorassoziiertes Antigen (TAA), ein virales Protein oder ein bakterielles Protein ist.

4. Verfahren nach Anspruch 3, wobei
(i) die hinsichtlich Immunzellen angereicherte Fraktion durch nachweisbares Markieren eines oder mehrerer immunzellspezifischer Oberflächenmarker ausgewählt wird; und/oder
(ii) das Verfahren den weiteren Schritt des Inkubierens der eukaryotischen Zellpopulation in Gegenwart von Wachstums- und/oder Differenzierungsfaktoren, vorzugsweise Zytokinen, umfasst; und/oder
(iii) es sich bei dem AC um eine antigenpräsentierende Zelle oder einen Komplex, umfassend ein Partikel, das PAI und die nachweisbare Markierung A oder das SPA und die nachweisbare Markierung B, handelt.

5. Verfahren nach einem der Ansprüche 1 bis 4, umfassend einen oder mehrere der folgenden weiteren Schritte:
(a) Inkontaktbringen der eukaryotischen Zellpopulation aus Schritt (i) mit einem dritten Antigenkomplex (3. AC), der das PAI und eine nachweisbare Markierung C umfasst, die nachweisbar verschieden ist von einer oder mehreren oder allen der anderen nachweisbaren Markierungen der anderen ACs, die mit der eukaryotischen Zellpopulation in Kontakt gebracht werden, vorzugsweise nachweisbar von mindestens der nachweisbaren Markierung A, vorzugsweise von mindestens der nachweisbaren Markierung A und einer nachweisbaren Markierung D, wenn eine nachweisbare Markierung D vorliegt, verschieden ist; und/oder
(b) Inkontaktbringen der eukaryotischen Zellpopulation aus Schritt (i) mit einem vierten Antigenkomplex (4. AC), der das PAI und eine nachweisbare Markierung D umfasst, die nachweisbar verschieden ist von einer oder mehreren oder allen der anderen nachweisbaren Markierungen der anderen ACs, die mit der eukaryotischen Zellpopulation in Kontakt gebracht werden, vorzugsweise nachweisbar von mindestens der nachweisbaren Markierung A und, vorzugsweise, von mindestens der nachweisbaren Markierung A und der nachweisbaren Markierung C, verschieden ist; und/oder
(c) Inkontaktbringen der eukaryotischen Zellpopulation aus Schritt (i) mit einem fünften Antigenkomplex (5. AC), der das SPA und eine nachweisbare Markierung E umfasst, die nachweisbar verschieden ist von einer oder mehreren oder allen der anderen nachweisbaren Markierungen der anderen ACs, die mit der eukaryotischen Zellpopulation in Kontakt gebracht werden, vorzugsweise nachweisbar von mindestens der nachweisbaren Markierung B und, vorzugsweise, von mindestens der nachweisbaren Markierung B und einer nachweisbaren Markierung F, wenn eine nachweisbare Markierung F vorliegt, verschieden ist; und/oder
(d) Inkontaktbringen der eukaryotischen Zellpopulation aus Schritt (i) mit einem sechsten Antigenkomplex (6. AC), der das SPA und eine nachweisbare Markierung F umfasst, die nachweisbar verschieden ist von einer oder mehreren oder allen der anderen nachweisbaren Markierungen der anderen ACs, die mit der eukaryotischen Zellpopulation in Kontakt gebracht werden, vorzugsweise nachweisbar von mindestens der nachweisbaren Markierung B und vorzugsweise von mindestens der nachweisbaren Markierung B und der nachweisbaren Markierung E verschieden ist; und/oder
(e) Inkontaktbringen der eukaryotischen Zellpopulation aus Schritt (i) mit einem oder mehreren weiteren Antigenkomplexen (AC), wobei jeder ein SPA umfasst, das sich in mindestens einer Aminosäuresequenz von der Aminosäuresequenz des SPA des 2. AC unterscheidet, und wobei jeder weitere AC eine oder mehrere Markierungen umfasst, wobei die eine oder mehreren Markierungen zu den ein oder mehreren Markierungen des 2. AC gleich oder nachweisbar verschieden davon sind.

6. Verfahren nach einem der Ansprüche 1 bis 5, wobei
(i) der 1. AC mindestens eine weitere nachweisbare Markierung und der 2. AC mindestens eine weitere nachweisbare Markierung umfasst, die entweder gleich oder verschieden sind; und/oder
(ii) der eine oder die mehreren weiteren AC mindestens eine weitere nachweisbare Markierung umfassen; wobei die mindestens eine weitere Markierung so ausgewählt ist, dass sie die Unterscheidung des 1. AC vom 2. AC und dem einen oder den mehreren weiteren ACs ermöglicht; und/oder
(iii) die nachweisbaren Markierungen unabhängig ausgewählt sind aus einer fluoreszierenden Markierung, vorzugsweise ausgewählt aus der Gruppe bestehend aus Xanthenen, Acridinen, Oxazinen, Cyaninen, Styrylfarbstoffen, Cumarinen, Porphinen, Metall-Ligand-Komplexen, fluoreszierenden Proteinen, Nanokristallen, Perylenen und Phtalocyaninen.

7. Verfahren nach einem der Ansprüche 1 bis 5, wobei der 1. AC ein Komplex aus einem MHC-I oder MHC-II und dem PAI ist, und wobei das PAI ein Zielpeptid (TP), vorzugsweise ein tumorspezifisches Zielpeptid, ist, und/oder der 2. AC ein Komplex aus einem MHC-I oder MHC-II und dem SPA ist, und wobei das SPA ein zielähnliches Peptid (TSP) ist, und wobei sich das TSP um mindestens 1 Aminosäure von der Aminosäuresequenz des TP unterscheidet,
wobei vorzugsweise die Aminosäuresequenz des mindestens einen TSP nach einem oder mehreren der folgenden Kriterien ausgewählt wird:
(a) Präsentation des TSP auf gesundem Gewebe;
(b) abgeleitet von einer HLA-typisierten Quelle; und
(c) Bindung an das jeweilige HLA.

8. Verfahren nach Anspruch 7, wobei die Aminosäuresequenz des TSP eine Länge von 8 bis 16 Aminosäuren aufweist und wobei:
(1) die Aminosäuresequenz des TSP sich unterscheidet von der Aminosäuresequenz des TP
X₁-X₂-X₃-X₄-X₅-X₆-X₇-X₈
(i) an Position X₁, X₂ und X₃, und wobei Position X₄ bis X₈ identisch oder ähnlich zu dem TP sind;
(ii) an Position X₄, X₅ und X₆, und wobei die Positionen X₁ bis X₃ und X₇ und X₈ identisch oder ähnlich zu dem TP sind; oder
(iii) an Position X₇ und X₈, und wobei Position X₁ bis X₆ identisch oder ähnlich zu dem TP sind;
wenn das TP eine Länge von 8 Aminosäuren aufweist; oder
(2) die Aminosäuresequenz des TSP sich unterscheidet von der Aminosäuresequenz des TP
X₁-X₂-X₃-X₄-X₅-X₆-X₇-X₈-X₉
(i) an Position X₁, X₂ und X₃, und wobei Position X₄ bis X₈ identisch oder ähnlich zu dem TP sind;
(ii) an Position X₄, X₅ und X₆, und wobei die Position X₁ bis X₃ und Positionen X₇ bis X₈ identisch oder ähnlich zu dem TP sind; oder
(iii) an Position X₄, X₅, X₆ und X₇, und wobei Position X₁ bis X₃ und Positionen X₈ bis X₉ identisch oder ähnlich zu dem TP sind; oder
(iv) an Position X₇, X₈ und X₉, und wobei Position X₁ bis X₆ identisch oder ähnlich zu dem TP sind;
wenn das TP eine Länge von 8-9 Aminosäuren aufweist; oder
(3) die Aminosäuresequenz des TSP sich unterscheidet von der Aminosäuresequenz des TP
X₁-X₂-X₃-X₄-X₅-X₆-X₇-X₈-X₉-X₁₀
(i) an Position X₁, X₂ und X₃, wobei Position X₄ bis X₁₀ identisch oder ähnlich zu dem TP sind;
(ii) an Position X₄, X₅, X₆ und X₇, wobei Position X₁ bis X₃ und Positionen X₈ bis X₁₀ identisch oder ähnlich zu dem TP sind; oder
(iii) an Position X₄, X₅ und X₆, und wobei Position X₁ bis X₃ und Positionen X₇ bis X₁₀ identisch oder ähnlich zu dem TP sind; oder
(iv) an Position X₈, X₉ und X₁₀, wobei Position X₁ bis X₇ identisch oder ähnlich zu dem TP sind;
wenn das TP eine Länge von 8-10 Aminosäuren aufweist; oder
(4) die Aminosäuresequenz des TSP sich unterscheidet von der Aminosäuresequenz des TP
X₁-X₂-X₃-X₄-X₅-X₆-X₇-X₈-X₉-X₁₀-X₁₁
(i) an Position X₁, X₂ und X₃, wobei Position X₄ bis X₁₁ identisch oder ähnlich zu dem TP sind;
(ii) an Position X₄, X₅, X₆ und X₇, wobei Position X₁ bis X₃ und Positionen X₈ bis X₁₁ identisch oder ähnlich zu dem TP sind; oder
(iii) an Position X₄, X₅ und X₆, und wobei Position X₁ bis X₃ und Positionen X₇ bis X₁₁ identisch oder ähnlich zu dem TP sind; oder
(iv) an Position X₈, X₉, X₁₀ und X₁₁, wobei Position X₁ bis X₇ identisch oder ähnlich zu dem TP sind; oder
(v) an Position X₉, X₁₀ und X₁₁, wobei Position X₁ bis X₈ identisch oder ähnlich zu dem TP sind;
wenn das TP eine Länge von 8-11 Aminosäuren aufweist; oder
(5) die Aminosäuresequenz des TSP sich unterscheidet von der Aminosäuresequenz des TP
X₁-X₂-X₃-X₄-X₅-X₆-X₇-X₈-X₉-X₁₀-X₁₁-X₁₂
(i) an Position X₁, X₂ und X₃, wobei Position X₄ bis X₁₂ identisch oder ähnlich zu dem TP sind;
(ii) an Position X₄, X₅, X₆ und X₇, wobei Position X₁ bis X₃ und Positionen X₈ bis X₁₂ identisch oder ähnlich zu dem TP sind; oder
(iii) an Position X₄, X₅ und X₆, und wobei Position X₁ bis X₃ und Positionen X₇ bis X₁₂ identisch oder ähnlich zu dem TP sind; oder
(iv) an Position X₈, X₉, X₁₀, X₁₁ und X₁₂, wobei Position X₁ bis X₇ identisch oder ähnlich zu dem TP sind; oder
(v) an Position X₉, X₁₀, X₁₁ und X₁₂, wobei Position X₁ bis X₈ identisch oder ähnlich zu dem TP sind;
wenn das TP eine Länge von 8-12 Aminosäuren aufweist;
wobei vorzugsweise die Aminosäuresequenz des TSP eine unterschiedliche Länge aufweist als die des TP.

9. Verfahren nach einem der Ansprüche 1 bis 8, wobei
(i) die Aminosäuresequenz des SPA oder des TSP weniger als 95 % Aminosäureidentität zu dem PAI oder TP, weniger als 90 % Aminosäureidentität oder weniger als 85 % Aminosäureidentität, aufweist; und/oder
(ii) die eukaryotische Zellpopulation aus Schritt (i) von Anspruch 1 mit nicht mehr als 10 Antigenkomplexen (AC) in Kontakt gebracht wird, die jeweils ein verschiedenes ähnliches Proteinantigen (SPA) umfassen, wobei nicht mehr als neun verschiedene SPAs, nicht mehr als acht verschiedene SPAs, nicht mehr als sieben verschiedene SPAs, nicht mehr als sechs verschiedene SPAs, nicht mehr als fünf verschiedene SPAs, nicht mehr als vier verschiedene SPAs, nicht mehr als drei verschiedene SPAs, nicht mehr als zwei verschiedene SPAs oder nicht mehr als ein SPA verwendet werden, wobei vorzugsweise das SPA ein TSP ist, wobei noch bevorzugter die Anzahl an verschiedenen TSPs zwischen 1-10; zwischen 2-8; zwischen 3-5 oder zwischen 1-3 liegt, wobei vorzugsweise drei TSPs verwendet werden, wobei vorzugsweise das SPA ein TSP ist, wobei stärker bevorzugt die Anzahl an verschiedenen TSPs zwischen 1-10; zwischen 2-8; zwischen 3-5 oder zwischen 1-3 liegt, wobei vorzugsweise drei TSPs verwendet werden; und/oder
(iii) die Schritte (ii) und (iii) von Anspruch 1 und/oder die Schritte (a), (b), (c) und/oder (d) von Anspruch 10 nacheinander in beliebiger Reihenfolge oder Kombination oder gleichzeitig ausgeführt werden.

10. Verfahren nach einem der Ansprüche 1 bis 9, wobei Schritt (iv) von Anspruch 1 und/oder die Schritte (a), (b), (c) oder (d) von Anspruch 5 Folgendes umfassen:
a) positives Selektieren (Auswählen) von eukaryotischen Zellen, die an den 1. AC, den 1. und 3. oder den 1., 3. und 4. AC gebunden sind; und/oder
b) negatives Selektieren (Ausschließen) von eukaryotischen Zellen, die an den 2. AC, den 2. und 5. oder den 2., 5. und 6. AC gebunden sind.

11. Verfahren nach einem der Ansprüche 1 bis 9, wobei
(i) die Markierung mittels Durchflusszytometrieanalyse, vorzugsweise MACS-Analyse, FACS-Analyse oder präparative Sortieranalyse, nachgewiesen wird; und/oder
(ii) die in der Population von Schritt i) von Anspruch 1 enthaltenen eukaryotischen Zellen T-Zellen und/oder B-Zellen sind und phänotypisiert werden, wobei vorzugsweise die Phänotypisierung von T-Zellen die Bestimmung eines oder mehrerer T-Zellmarker umfasst; und/oder
(iii) Phänotypisierung von B-Zellen die Bestimmung eines oder mehrerer B-Zellmarker umfasst.

12. Verfahren nach einem der Ansprüche 1 bis 11, das ferner den folgenden Schritt umfasst:
Inkontaktbringen der eukaryotischen Zellpopulation aus Schritt (i) von Anspruch 1 mit einem irrelevanten Antigenkomplex (IAC), der ein irrelevantes Proteinantigen (IPA) umfasst, wobei die Aminosäuresequenz des IPA, wenn sie mit der Aminosäuresequenz des PAI aligniert wird, an zwei oder weniger Aminosäurepositionen mit dem PAI identisch ist und wobei der IAC eine nachweisbare Markierung G umfasst, die von der nachweisbaren Markierung A nachweisbar verschieden ist, wobei vorzugsweise
(i) die Aminosäuresequenz von mindestens einem IPA nach einem oder mehreren der folgenden Kriterien ausgewählt wird:
(a) Präsentation des IPA auf gesundem Gewebe;
(b) abgeleitet von einer HLA-typisierten Quelle;
(c) Bindung an das jeweilige HLA; und/oder
(ii) die Aminosäuresequenz des IPA oder von mindestens einem Protein oder Peptid, das in dem IPA enthalten ist, weniger als 30 % Aminosäureidentität, weniger als 20 % Aminosäureidentität oder weniger als 10 % Aminosäureidentität zu dem PAI aufweist; und/oder
(iii) der IAC ein Komplex aus einem MHC-I oder MHC-II und einem irrelevanten Peptid (IP) ist, und wobei die Aminosäuresequenz des IP, wenn sie mit der Aminosäuresequenz des TP aligniert wird, an einer oder keiner Aminosäureposition mit dem TP identisch ist.

13. Verfahren zum Bestimmen der Sequenz einer Nukleinsäure, die ein Antigenbindungsprotein oder einen antigenbindenden Teil davon codiert, umfassend die Schritte:
(i) Isolieren der Nukleinsäure, die das Antigenbindungsprotein oder den antigenbindenden Teil davon codiert, aus der eukaryotischen Zelle, die gemäß einem der Ansprüche 1 bis 11 ausgewählt wurde; und
(ii) Bestimmen der Sequenz der Nukleinsäure,
wobei vorzugsweise das Antigenbindungsprotein ein TCR oder ein Fragment davon; ein BCR oder ein Fragment davon oder ein Antikörper oder ein Fragment davon ist.

14. Verfahren zur Herstellung einer eukaryotischen Zelle, die eine Nukleinsäure exprimiert, die ein Antigenbindungsprotein oder einen antigenbindenden Teil davon codiert, umfassend die Schritte:
(i) Bereitstellen der Nukleinsäuresequenz, die das Antigenbindungsprotein oder einen antigenbindenden Teil davon codiert, aus der eukaryotischen Zelle, die in dem Verfahren gemäß einem der Ansprüche 1 bis 11 ausgewählt wurde;
(ii) Herstellen eines Nukleinsäurevektors, der die in Schritt (i) bereitgestellte Nukleinsäuresequenz, optional unter der Kontrolle eines Expressionskontrollelements, umfasst; und
(iii) Einbringen des Nukleinsäurevektors aus Schritt (ii) in eine Wirtszelle,
wobei vorzugsweise das Antigenbindungsprotein ein TCR oder ein Fragment davon; ein BCR oder ein Fragment davon oder ein Antikörper oder ein Fragment davon ist, wobei stärker bevorzugt die Aminosäuresequenzen des TCR, des BCR oder des Antikörpers sechs CDR-Sequenzen umfassen.

## Revendications

1. Procédé pour la sélection d'une cellule eucaryote exprimant sur sa surface une protéine de liaison à un antigène se liant spécifiquement et/ou sélectivement à un antigène protéique d'intérêt (PAI) comprenant les étapes suivantes :
(i) fourniture d'une population de cellules eucaryotes ;
(ii) mise en contact de la population de cellules eucaryotes de l'étape (i) avec un premier complexe d'antigène (1^{er} AC) comprenant le PAI et un marqueur détectable A ou avec le PAI comprenant un marqueur détectable A ;
(iii) mise en contact de la population de cellules eucaryotes dans l'étape (i) avec au moins un deuxième complexe d'antigène (2^{e} AC) comprenant un antigène protéique similaire (SPA), la séquence d'acides aminés du SPA différant de 1 à 20 acides aminés par rapport à la séquence d'acides aminés du PAI, préférablement 2 à 10 acides aminés par rapport à la séquence d'acides aminés du PAI, et le 2^{e} AC comprenant un marqueur détectable B ; ou avec le SPA et un marqueur détectable B ; et
(iv) sélection d'au moins une cellule eucaryote qui se lie de manière spécifique et/ou de manière sélective au 1^{er} AC,
le marqueur détectable A et le marqueur détectable B étant différents l'un de l'autre en ce qui concerne la détection, et la cellule eucaryote choisie étant une cellule T, une cellule B ou une cellule de mammifère exprimant une protéine de liaison à un antigène hétérologue choisie dans le groupe constitué par un récepteur de cellules T (TCR) ou des fragments de liaison à un antigène correspondant, un récepteur de cellules B (BCR) ou des fragments de liaison à un antigène correspondant, et un récepteur antigénique chimérique (CAR) ou des fragments de liaison à un antigène correspondant.

2. Procédé selon la revendication 1, la séquence d'acides aminés du SPA différant de 2 à 10 acides aminés par rapport à la séquence d'acides aminés du PAI.

3. Procédé selon la revendication 1 ou 2,
(a) la population de cellules eucaryotes comprenant :
(i) des cellules immunitaires, préférablement des lymphocytes infiltrant une tumeur (TIL), des bibliothèques de récepteurs de cellules T, du sang périphérique de sujets sains, du sang périphérique de sujets malades ou une fraction enrichie en cellules immunitaires correspondante, plus préférablement,
(1) la fraction enrichie en cellules immunitaires étant enrichi en cellules souches ; en cellules T, plus préférablement en cellules T CD8 ou en cellules T CD4 ; en cellules B ; en cellule plasmatique ;
(2) le sujet malade souffrant d'une maladie choisie dans le groupe constitué par des maladies immunitaires, des maladies néoplasiques, une maladie causée par un virus ou une maladie causée par des bactéries ; ou
(ii) des cellules de mammifère exprimant une bibliothèque de protéines de liaison à un antigène hétérologue ;
et/ou
(b) l'antigène protéique d'intérêt (PAI) étant un antigène associé à une tumeur (TAA), une protéine virale ou une protéine bactérienne.

4. Procédé selon la revendication 3,
(i) la fraction enrichie en cellules immunitaires étant choisie par marquage en ce qui concerne la détection d'un ou plusieurs marqueurs de surface spécifiques à des cellules immunitaires ; et/ou
(ii) le procédé comprenant l'étape supplémentaire d'incubation de la population de cellules eucaryotes en la présence de facteurs de croissance et/ou de différenciation, préférablement des cytokines ; et/ou
(iii) l'AC étant une cellule présentant un antigène, ou un complexe comprenant une particule, le PAI et le marqueur détectable A ou le SPA et le marqueur détectable B.

5. Procédé selon l'une quelconque des revendications 1 à 4, comprenant une ou plusieurs des étapes supplémentaires suivantes :
(a) mise en contact de la population de cellules eucaryotes de l'étape (i) avec un troisième complexe d'antigène (3^{e} AC) comprenant le PAI et un marqueur détectable C qui est différent en ce qui concerne la détection d'un ou plusieurs ou de la totalité de tous les autres marqueurs détectables des autres AC mis en contact avec la population de cellules eucaryotes, préférablement différent en ce qui concerne la détection d'au moins le marqueur détectable A, préférablement d'au moins le marqueur détectable A et un marqueur détectable D, si un marqueur détectable D est présent ; et/ou
(b) mise en contact de la population de cellules eucaryotes de l'étape (i) avec un quatrième complexe d'antigène (4^{e} AC) comprenant le PAI et un marqueur détectable D qui est différent en ce qui concerne la détection d'un ou plusieurs ou de la totalité des autres marqueurs détectables des autres AC mis en contact avec la population de cellules eucaryotes, préférablement différent en ce qui concerne la détection d'au moins le marqueur détectable A et, préférablement d'au moins le marqueur détectable A et le marqueur détectable C ; et/ou
(c) mise en contact de la population de cellules eucaryotes de l'étape (i) avec un cinquième complexe d'antigène (5^{e} AC) comprenant le SPA et un marqueur détectable E qui est différent en ce qui concerne la détection d'un ou plusieurs ou de la totalité des autres marqueurs détectables des autres AC mis en contact avec la population de cellules eucaryotes, préférablement différent en ce qui concerne la détection d'au moins le marqueur détectable B et, préférablement d'au moins le marqueur détectable B et un marqueur détectable F, si un marqueur détectable F est présent ; et/ou
(d) mise en contact de la population de cellules eucaryotes de l'étape (i) avec un sixième complexe d'antigène (6^{e} AC) comprenant le SPA et un marqueur détectable F qui est différent en ce qui concerne la détection d'un ou plusieurs ou de la totalité des autres marqueurs détectables des autres AC mis en contact avec la population de cellules eucaryotes, préférablement différent en ce qui concerne la détection d'au moins le marqueur détectable B et, préférablement d'au moins le marqueur détectable B et le marqueur détectable E ; et/ou
(e) mise en contact de la population de cellules eucaryotes de l'étape (i) avec un ou plusieurs autres complexes d'antigène (AC), chacun comprenant un SPA qui diffère dans au moins une séquence d'acides aminés par rapport à la séquence d'acides aminés du SPA du 2^{e} AC, et chaque AC supplémentaire comprenant un ou plusieurs marqueurs, le ou les marqueurs étant les mêmes ou différents en ce qui concerne la détection du ou des marqueurs du 2^{e} AC.

6. Procédé selon l'une quelconque des revendications 1 à 5,
(i) le 1^{er} AC comprenant au moins un marqueur détectable supplémentaire et le 2^{e} AC comprenant au moins un marqueur détectable supplémentaire, qui sont soit les mêmes, soit différents ; et/ou
(ii) le ou les AC supplémentaires comprenant au moins un marqueur détectable supplémentaire ; l'au moins un marqueur supplémentaire étant choisi de telle manière qu'il permette de distinguer le 1^{er} AC du 2^{e} AC et du ou des AC supplémentaires ; et/ou
(iii) les marqueurs détectables étant indépendamment choisis parmi un marqueur fluorescent, préférablement choisi dans le groupe constitué par des xanthènes, des acridines, des oxazines, des cyanines, des colorants styryliques, des coumarines, des porphines, des complexes métal-ligand, des protéines fluorescentes, des nanocristaux, des pérylènes et des phtalocyanines.

7. Procédé selon l'une quelconque des revendications 1 à 5, le 1^{er} AC étant un complexe d'un MHC-I ou MHC-II et du PAI, et le PAI étant un peptide cible (TP), préférablement un peptide cible spécifique à une tumeur et/ou le 2^{e} AC étant un complexe d'un MHC-I ou MHC-II et du SPA, et le SPA étant un peptide similaire à une cible (TSP) et le TSP différant d'au moins 1 acide aminé par rapport à la séquence d'acides aminés du TP,
préférablement la séquence d'acides aminés de l'au moins un TSP étant choisie par un ou plusieurs des critères suivants :
(a) présentation du TSP sur un tissu sain ;
(b) issue d'une source typée HLA ; et
(c) se liant au HLA respectif.

8. Procédé selon la revendication 7, la séquence d'acides aminés du TSP ayant une longueur de 8 à 16 acides aminés et :
(1) la séquence d'acides aminés du TSP différant de la séquence d'acides aminés du TP
X₁-X₂-X₃-X₄-X₅-X₆-X₇-X₈
(i) au niveau des positions X₁, X₂ et X₃, et les positions X₄ à X₈ étant identiques ou similaires au TP ;
(ii) au niveau des positions X₄, X₅ et X₆, et les positions X₁ à X₃ et X₇ et X₈ étant identiques ou similaires au TP ; ou
(iii) au niveau des positions X₇ et X₈, et les positions X₁ à X₆ étant identiques ou similaires au TP ;
si le TP a une longueur de 8 acides aminés ; ou
(2) la séquence d'acides aminés du TSP différant de la séquence d'acides aminés du TP
X₁-X₂-X₃-X₄-X₅-X₆-X₇-X₈-X₉
(i) au niveau des positions X₁, X₂ et X₃, et les positions X₄ à X₈ étant identiques ou similaires au TP ;
(ii) au niveau des positions X₄, X₅ et X₆, et les positions X₁ à X₃ et les positions X₇ à X₉ étant identiques ou similaires au TP ; ou
(iii) au niveau des positions X₄, X₅, X₆ et X₇, et les positions X₁ à X₃ et les positions X₈ à X₉ étant identiques ou similaires au TP ; ou
(iv) au niveau des positions X₇, X₈ et X₉, et les positions X₁ à X₆ étant identiques ou similaires au TP ; ou
si le TP a une longueur de 8-9 acides aminés ; ou
(3) la séquence d'acides aminés du TSP différant de la séquence d'acides aminés du TP
X₁-X₂-X₃-X₄-X₅-X₆-X₇-X₈-X₉-X₁₀
(i) au niveau des positions X₁, X₂ et X₃, les positions X₄ à X₂₀ étant identiques ou similaires au TP ;
(ii) au niveau des positions X₄, X₅, X₆ et X₇, les positions X₁ à X₃ et les positions X₈ à X₁₀ étant identiques ou similaires au TP ; ou
(iii) au niveau des positions X₄, X₅ et X₆, et les positions X₁ à X₃ et les positions X₇ à X₁₀ étant identiques ou similaires au TP ; ou
(iv) au niveau des positions X₈, X₉ et X₁₀, les positions X₁ à X₇ étant identiques ou similaires au TP ;
si le TP a une longueur de 8-10 acides aminés ; ou
(4) la séquence d'acides aminés du TSP différant de la séquence d'acides aminés du TP
X₁-X₂-X₃-X₄-X₅-X₆-X₇-X₈-X₉-X₁₀X₁₁
(i) au niveau des positions X₁, X₂ et X₃, les positions X₄ à X₁₁ étant identiques ou similaires au TP ;
(ii) au niveau des positions X₄, X₅, X₆ et X₇, les positions X₁ à X₃ et les positions X₈ à X₁₁ étant identiques ou similaires au TP ; ou
(iii) au niveau des positions X₄, X₅ et X₆, et les positions X₁ à X₃ et les positions X₇ à X₁₁ étant identiques ou similaires au TP ; ou
(iv) au niveau des positions X₈, X₉, X₁₀ et X₁₁, les positions X₁ à X₇ étant identiques ou similaires au TP ; ou
(v) au niveau des positions X₉, X₁₀ et X₁₁, les positions X₁ à X₈ étant identiques ou similaires au TP ;
si le TP a une longueur de 8-11 acides aminés ; ou
(5) la séquence d'acides aminés du TSP différant de la séquence d'acides aminés du TP
X₁-X₂-X-₃-X₄-X₅-X₆-X₇-X₈-X₉-X₁₀X₁₁ X₁₂
(i) au niveau des positions X₁, X₂ et X₃, les positions X₄ à X₁₂ étant identiques ou similaires au TP ;
(ii) au niveau des positions X₄, X₅, X₆ et X₇, les positions X₁ à X₃ et les positions X₈ à X₁₂ étant identiques ou similaires au TP ; ou
(iii) au niveau des positions X₄, X₅ et X₆, et les positions X₁ à X₃ et les positions X₇ à X₁₂ étant identiques ou similaires au TP ; ou
(iv) au niveau des positions X₈, X₉, X₁₀, X₁₁ et X₂₂, les positions X₁ à X₇ étant identiques ou similaires au TP ; ou
(v) au niveau des positions X₉, X₁₀, X₁₁ et X₂₂, les positions X₁ à X₈ étant identiques ou similaires au TP ;
si le TP a une longueur de 8-12 acides aminés ;
préférablement la séquence d'acides aminés du TSP ayant une longueur différente du TP.

9. Procédé selon l'une quelconque des revendications 1 à 8,
(i) la séquence d'acides aminés du SPA ou du TSP ayant moins de 95 % d'identité d'acides aminés avec le PAI ou le TP, moins de 90 % d'identité d'acides aminés ou moins de 85 % d'identité d'acides aminés ; et/ou
(ii) la population de cellules eucaryotes de l'étape (i) selon la revendication 1 étant mise en contact avec pas plus de 10 complexes d'antigène (AC) comprenant chacun un antigène protéique similaire (SPA) différent, pas plus de neuf SPA différents, pas plus de huit SPA différents, pas plus de sept SPA différents, pas plus de six SPA différents, pas plus de cinq SPA différents, pas plus de quatre SPA différents, pas plus de trois SPA différents, pas plus de deux SPA différents, ou pas plus d'un SPA, étant utilisé(s), préférablement le SPA étant un TSP, encore plus préférablement le nombre de TSP différents étant compris entre 1 et 10 ; entre 2 et 8 ; entre 3 et 5 ou entre 1 et 3, préférablement trois TSP étant utilisés, préférablement le SPA étant un TSP, plus préférablement le nombre de TSP différents étant compris entre 1 et 10 ; entre 2 et 8 ; entre 3 et 5 ou entre 1 et 3, préférablement trois TSP étant utilisés ; et/ou
(iii) les étapes (ii) et (iii) selon la revendication 1 et/ou les étapes (a), (b), (c) et/ou (d) selon la revendication 10 étant mises en oeuvre subséquemment selon un ordre ou une combinaison quelconque ou de manière concomitante.

10. Procédé selon l'une quelconque des revendications 1 à 9, l'étape (iv) selon la revendication 1 et/ou les étapes (a), (b), (c) ou (d) selon la revendication 5 comprenant :
a) une sélection de manière positive (sélection) de cellules eucaryotes liées au 1^{er} AC, 1^{er} et 3^{e} ou 1^{er}, 3^{e} et 4^{e} AC ; et/ou
b) une sélection de manière négative (exclusion) de cellules eucaryotes liées au 2^{e} AC, 2^{e} et 5^{e} ou 2^{e}, 5^{e} et 6^{e} AC.

11. Procédé selon l'une quelconque des revendications 1 à 9,
(i) le marqueur étant détecté par analyse de cytométrie de flux, préférablement analyse MACS, analyse FACS ou analyse de classement préparative ; et/ou
(ii) les cellules eucaryotes comprises dans la population de l'étape i) selon la revendication 1 étant des cellules T et/ou des cellules B et étant phénotypées, préférablement le phénotypage de cellules T comprenant la détermination d'un ou plusieurs marqueurs de cellules T ; et/ou
(iii) le phénotypage de cellules B comprenant la détermination d'un ou plusieurs marqueurs de cellules B.

12. Procédé selon l'une quelconque des revendications 1 à 11, comprenant en outre l'étape de :
mise en contact de la population de cellules eucaryotes de l'étape (i) selon la revendication 1 avec un complexe d'antigène non pertinent (IAC) comprenant un antigène protéique non pertinent (IPA), la séquence d'acides aminés de l'IPA lorsqu'elle est alignée avec la séquence d'acides aminés du PAI étant identique au PAI au niveau de deux positions d'acides aminés ou moins et, l'IAC comprenant un marqueur détectable G qui est différent en ce qui concerne la détection du marqueur détectable A, préférablement,
(i) la séquence d'acides aminés d'au moins un IPA étant choisie par un ou plusieurs des critères suivants :
(a) présentation de l'IPA sur un tissu sain ;
(b) issue d'une source typée HLA ;
(c) se liant au HLA respectif ; et/ou
(ii) la séquence d'acides aminés de l'IPA ou d'au moins une protéine ou d'au moins un peptide compris(e) dans l'IPA ayant moins de 30 % d'identité d'acides aminés avec le PAI, moins de 20 % d'identité d'acides aminés ou moins de 10 % d'identité d'acides aminés ; et/ou
(iii) l'IAC étant un complexe d'un MHC-I ou MHC-II et d'un peptide non pertinent (IP), et la séquence d'acides aminés de l'IP lorsqu'elle est alignée avec la séquence d'acides aminés du TP étant identique au TP au niveau d'une ou d'aucune position d'acides aminés.

13. Procédé pour la détermination de la séquence d'un acide nucléique codant pour une protéine de liaison à un antigène ou une partie de liaison à un antigène de celle-ci comprenant les étapes de :
(i) isolement de l'acide nucléique codant pour la protéine de liaison à un antigène ou la partie de liaison à un antigène de celle-ci à partir de la cellule eucaryote choisie selon l'une quelconque des revendications 1 à 11 ; et
(ii) détermination de la séquence de l'acide nucléique,
préférablement, la protéine de liaison à un antigène étant un TCR ou un fragment correspondant ; un BCR ou un fragment correspondant ou un anticorps ou un fragment correspondant.

14. Procédé pour la production d'une cellule eucaryote exprimant un acide nucléique codant pour une protéine de liaison à un antigène ou une partie de liaison à un antigène de celle-ci comprenant les étapes de :
(i) fourniture de la séquence d'acides nucléiques codant pour la protéine de liaison à un antigène ou une partie de liaison à un antigène de celle-ci à partir de la cellule eucaryote choisie dans le procédé selon l'une quelconque des revendications 1 à 11 ;
(ii) production d'un vecteur d'acide nucléique comprenant la séquence d'acides nucléiques fournie dans l'étape (i) éventuellement sous le contrôle d'un élément de contrôle d'expression ; et
(iii) introduction du vecteur d'acide nucléique de l'étape (ii) dans une cellule hôte,
préférablement, la protéine de liaison à un antigène étant un TCR ou un fragment correspondant ; un BCR ou un fragment correspondant ou un anticorps ou un fragment correspondant, plus préférablement les séquences d'acides aminés du TCR, du BCR ou de l'anticorps comprenant six séquences CDR.
